# EUROPÄISCHE PATENTSCHRIFT

(11) **EP 1 399 576 B1**
(45) Veröffentlichungstag und Bekanntmachung des Hinweises auf die Patenterteilung: **22.08.2007**
(21) Anmeldenummer: 02748324.7
(22) Anmeldetag: 28.02.2002
(51) Int. Cl.: C12N 15/90

(54) **TESTSYSTEM ZUR BESTIMMUNG VON GENTOXIZITÄTEN**
TEST SYSTEM FOR DETERMINING GENE TOXICITIES
SYSTEME DE TEST DESTINE A DETERMINER DES TOXICITES GENETIQUES

(30) Priorität: 05.03.2001 DE 10110449
(43) Veröffentlichungstag der Anmeldung: 24.03.2004
(73) Patentinhaber: Wiesmüller, Lisa, 86163 Augsburg (DE)
(72) Erfinder: Wiesmüller, Lisa, 86163 Augsburg (DE)
(74) Vertreter: UEXKÜLL & STOLBERG
(86) Internationale Anmeldenummer: PCT/EP2002/002194
(87) Internationale Veröffentlichungsnummer: WO 2002/070740

(56) Entgegenhaltungen:
- SLEBOS ROBBERT J C ET AL: "A novel host cell reactivation assay to assess homologous recombination capacity in human cancer cell lines." BIOCHEMICAL AND BIOPHYSICAL RESEARCH COMMUNICATIONS, Bd. 281, Nr. 1, 16. Februar 2001 (2001-02-16), Seiten 212-219, XP002227693 ISSN: 0006-291X in der Anmeldung erwähnt
- PIERCE A J ET AL: "XRCC3 PROMOTES HOMOLOGY-DIRECTED REPAIR OF DNA DAMAGE IN MAMMALIAN CELLS" GENES AND DEVELOPMENT, COLD SPRING HARBOR LABORATORY PRESS, NEW YORK, US, Bd. 13, Nr. 20, 15. Oktober 1999 (1999-10-15), Seiten 2633-2638, XP001000711 ISSN: 0890-9369 in der Anmeldung erwähnt
- KANAAR ROLAND ET AL: "Molecular mechanisms of DNA double-strand break repair." TRENDS IN CELL BIOLOGY, Bd. 8, Nr. 12, Dezember 1998 (1998-12), Seiten 483-489, XP001122136 ISSN: 0962-8924 in der Anmeldung erwähnt
- KAWAMOTO S ET AL: "A novel reporter mouse strain that expresses enhanced green fluorescent protein upon Cre-mediated recombination" FEBS LETTERS, ELSEVIER SCIENCE PUBLISHERS, AMSTERDAM, NL, Bd. 470, Nr. 3, 31. März 2000 (2000-03-31), Seiten 263-268, XP004336951 ISSN: 0014-5793
- SUTER KELLY J ET AL: "Genetic targeting of green fluorescent protein to gonadotropin-releasing hormone neurons: Characterization of whole-cell electrophysiological properties and morphology." ENDOCRINOLOGY, Bd. 141, Nr. 1, Januar 2000 (2000-01), Seiten 412-419, XP002227727 ISSN: 0013-7227
- THORPE P ET AL: "Optimising gene repair strategies in cell culture." GENE THERAPY, Bd. 9, Nr. 11, Juni 2002 (2002-06), Seiten 700-702, XP009004392 June, 2002 ISSN: 0969-7128

### SEQUENZPROTOKOLL

<110> Prof. Dr. Wiesmueller, Lisa
<120> Testsystem zur Bestimmung von Gentoxizitaeten
<130> P59532
<140>
   <141>
<160> 20
<170> PatentIn Ver. 2.1
<210> 1
   <211> 18
   <212> DNA
   <213> Kuenstliche Sequenz
<220>
   <223> Beschreibung der kuenstlichen Sequenz: Primer EGFPwt-1
<400> 1
   gtgaccaccc tgacctac 18
<210> 2
   <211> 20
   <212> DNA
   <213> Kuenstliche Sequenz
<220>
   <223> Beschreibung der kuenstlichen Sequenz: Primer EGFPSeq1
<400> 2
   gcagcacgac ttcttcaagt 20
<210> 3
   <211> 21
   <212> DNA
   <213> Kuenstliche Sequenz
<220>
   <223> Beschreibung der kuenstlichen Sequenz: Primer EGFPSeq3
<400> 3
   gttgtggctg ttgtagttgt a 21
<210> 4
   <211> 73
   <212> DNA
   <213> Kuenstliche Sequenz
<220>
   <223> Beschreibung der kuenstlichen Sequenz: Primer EGFP-11
<400> 4
   gttcatctgc accaccggca agctgcccgg aattctaggg ataacagggt aatgcttcag 60
   ccgctacccc gac 73
<210> 5
   <211> 41
   <212> DNA
   <213> Kuenstliche Sequenz
<220>
   <223> Beschreibung der kuenstlichen Sequenz: Primer EGFP-13
<400> 5
   aggagggaat tcggatccgc ggccgcttta cttgtacagc t 41
<210> 6
   <211> 33
   <212> DNA
   <213> Kuenstliche Sequenz
<220>
   <223> Beschreibung der kuenstlichen Sequenz: Primer EGFP-14
<400> 6
   tccctagaat tccgggcagc ttgccggtgg tgc 33
<210> 7
   <211> 58
   <212> DNA
   <213> Kuenstliche Sequenz
<220>
   <223> Beschreibung der kuenstlichen Sequenz: Primer EGFP-15
<400> 7
   cgcgcgacct ggtgcatgac ccgcaagccc ggtgccatgg tgagcaaggg cgaggagc 58
<210> 8
   <211> 37
   <212> DNA
   <213> Kuenstliche Sequenz
<220>
   <223> Beschreibung der kuenstlichen Sequenz: Primer EGFP-EJ-1
<400> 8
   tgacctacta gggataacag ggtaatccta cggcgtg 37
<210> 9
   <211> 49
   <212> DNA
   <213> Kuenstliche Sequenz
<220>
   <223> Beschreibung der kuenstlichen Sequenz: Primer EGFP-EJ-2B
<400> 9
   cacgccgtag gattaccctg ttatccctag taggtcaggg tggtcacga 49
<210> 10
   <211> 20
   <212> DNA
   <213> Kuenstliche Sequenz
<220>
   <223> Beschreibung der kuenstlichen Sequenz: Primer Purol
<400> 10
   cccgcaacct ccccttctac 20
<210> 11
   <211> 21
   <212> DNA
   <213> Kuenstliche Sequenz
<220>
   <223> Beschreibung der kuenstlichen Sequenz: Primer EGFP20-2
<400> 11
   gtgaacagct cctcgccctt g 21
<210> 12
   <211> 22
   <212> DNA
   <213> Kuenstliche Sequenz
<220>
   <223> Beschreibung der kuenstlichen Sequenz: Primer Sce1-1
<400> 12
   ggtccgaact ctaaactgct ga 22
<210> 13
   <211> 20
   <212> DNA
   <213> Kuenstliche Sequenz
<220>
   <223> Beschreibung der kuenstlichen Sequenz: Primer Scel-2b
<400> 13
   tcggggtcag gtagttttca 20
<210> 14
   <211> 20
   <212> DNA
   <213> Kuenstliche Sequenz
<220>
   <223> Beschreibung der kuenstlichen Sequenz: Primer EGFP-PCR1
<400> 14
   gtgagcaagg gcgaggagct 20
<210> 15
   <211> 23
   <212> DNA
   <213> Kuenstliche Sequenz
<220>
   <223> Beschreibung der kuenstlichen Sequenz: Primer EGFP-PCR2
<400> 15
   ctttacttgt acagctcgtc cat 23
<210> 16
   <211> 24
   <212> DNA
   <213> Kuenstliche Sequenz
<220>
   <223> Beschreibung der kuenstlichen Sequenz: Primer EGFP-PCR3
<400> 16
   gacgttgtgg ctgttgtagt tgta 24
<210> 17
   <211> 22
   <212> DNA
   <213> Kuenstliche Sequenz
<220>
   <223> Beschreibung der kuenstlichen Sequenz: Primer Hygl-1
<400> 17
   ggaaagcctg aactcaccgc ga 22
<210> 18
   <211> 22
   <212> DNA
   <213> Kuenstliche Sequenz
<220>
   <223> Beschreibung der kuenstlichen Sequenz: Primer Hyg2-2
<400> 18
   gcttctgcgg gcgatttgtg ta 22
<210> 19
   <211> 22
   <212> DNA
   <213> Kuenstliche Sequenz
<220>
   <223> Beschreibung der kuenstlichen Sequenz: Primer Hyg4-1
<400> 19
   tacacaaatc gcccgcagaa gc 22
<210> 20
   <211> 9320
   <212> DNA
   <213> Kuenstliche Sequenz
<220>
   <223> Beschreibung der kuenstlichen Sequenz: Plasmid p5-Puro-CMV-(N'-EGFP)-CMV-Red-(EGFP-EJ)
<220>
   <221> misc_feature
   <222> (1)..(1592)
   <223> Retroviraler Vektor p5NM
<220>
   <221> misc feature
   <222> (3374)..(3392)
   <223> Retroviraler Vektor p5NM
<220>
   <221> misc feature
   <222> (5527)..(9320)
   <223> Retroviraler Vektor p5NM
<220>
   <221> gene
   <222> (1617)...(2216)
   <223> Puromycin-Resistenzgen aus pRetroOn (Clontech, Palo Alto, CA, USA)
<220>
   <221> promoter
   <222> (2267)..(2848)
   <223> CMV-Promotor aus pEGFP-N1 (Clontech, Palo Alto, CA, USA)
<220>
   <221> gene
   <222> (2906)..(3348)
   <223> N'-EGFP, abgeleitet von EGFP aus pEGFP-N1 (Clontech, Palo Alto, CA, USA)
<220>
   <221> promoter
   <222> (3411)..(3992)
   <223> CMV-Promotor aus pDsRed1-N1 (Clontech, Palo Alto, CA, USA)
<220>
   <221> gene
   <222> (4038)..(4718)
   <223> Red aus pDsRed1-N1 (Clontech, Palo Alto, CA, USA)
<220>
   <221> gene
   <222> (4766)..(5508)
   <223> EGFP-EJ, abgeleitet von EGFP aus pEGFP-N1 (Clontech, Palo Alto, CA, USA)
<400> 20
   cgattagtcc aatttgttaa agacaggata tcaggtggtc caggctctag ttttgactca 60
   acaatatcac cagctgaagc ctatagagta cgagccatag atagaataaa agattttatt 120
   tagtctccag aaaaaggggg gaatgaaaga ccccacctgt aggtttggca agctagctta 180
   agtaacgcca ttttgcaagg catggaaaat acataactga gaatagagaa gttcagatca 240
   aggttaggaa cagagagaca gcagaatatg ggccaaacag gatatctgtg gtaagcagtt 300
   cctgccccgc tcagggccaa gaacagatgg tccccagatg cggtcccgcc ctcagcagtt 360
   tctagagaac catcagatgt ttccagggtg ccccaaggac ctgaaaatga ccctgtgcct 420
   tatttgaact aaccaatcag ttcgcttctc gcttctgttc gcgcgcttct gctccccgag 480
   ctcaataaaa gagcccacaa cccctcactc ggcgcgccag tcctccgatt gactgcgtcg 540
   cccgggtacc cgtattccca ataaagcctc ttgctgtttg catccgaatc gtggactcgc 600
   tgatccttgg gagggtctcc tcagattgat tgactgccca cctcgggggt ctttcatttg 660
   gaggttccac cgagatttgg agaccccagc ccagggacca ccgacccccc cgccgggagg 720
   caagctggcc agcggtcgtt tcgtgtctgt ctctgtcttt gtgcgtgttt gtgccggcat 780
   ctaatgtttg cgcctgcgtc tgtactagtt ggctaactag atctgtatct ggcggtcccg 840
   cggaagaact gacgagttcg tattcccggc cgcagcccct aggagacgtc ccagcggcct 900
   cgggggcccg ttttgtggcc cgttctgtgt cgttaaccac ccgagtcgga ctttttggag 960
   ctccgccact gtccgagggg tacgtggctt tgttggggga cgagagacag agacacttcc 1020
   cgcccccgtc tgaatttttg ctttcggttt tacgccgaaa ccgcgccgcg cgtcttgtct 1080
   gctgcagcat cgttctgtgt tgtctctgtc tgactgtgtt tctgtatttg tctgaaaatt 1140
   agggccagac tgttaccact cccttaagtt tgaccttaga tcactggaaa gatgtcgagc 1200
   ggatcgctca caaccagtcg gtagatgtca agaagagacg atgggttacc ttctgctctg 1260
   cagaatggcc aacctttaac gtcggatggc cgcgagacgg cacctttaac cgagacctca 1320
   tcacccaggt taagatcaag gtcttttcac ctggcccgca tggacaccca gaccaggtcc 1380
   cctacatcgt gacctgggaa gccttggctt ttgacccccc tccctgggtc aagccctttg 1440
   tacaccctaa gcctccgcct cctcttcctc catccgcccc gtctctcccc cttgaacctc 1500
   ctctttcgac cccgcctcga tcctcccttt atccagccct cactccttct ctaggcggct 1560
   ccaccgcggt ggcggccgct ctagaactag tggatccggg gccggatcag cttaccatga 1620
   ccgagtacaa gcccacggtg cgcctcgcca cccgcgacga cgtccccagg gccgtacgca 1680
   ccctcgccgc cgcgttcgcc gactaccccg ccacgcgcca caccgtcgat ccggaccgcc 1740
   acatcgagcg ggtcaccgag ctgcaagaac tcttcctcac gcgcgtcggg ctcgacatcg 1800
   gcaaggtgtg ggtcgcggac gacggcgccg cggtggcggt ctggaccacg ccggagagcg 1860
   tcgaagcggg ggcggtgttc gccgagatcg gcccgcgcat ggccgagttg agcggttccc 1920
   ggctggccgc gcagcaacag atggaaggcc tcctggcgcc gcaccggccc aaggagcccg 1980
   cgtggttcct ggccaccgtc ggcgtctcgc ccgaccacca gggcaagggt ctgggcagcg 2040
   ccgtcgtgct ccccggagtg gaggcggccg agcgcgccgg ggtgcccgcc ttcctggaga 2100
   cctccgcgcc ccgcaacctc cccttctacg agcggctcgg cttcaccgtc accgccgacg 2160
   tcgagtgccc gaaggaccgc gcgacctggt gcatgacccg caagcccggt gcctgacgcc 2220
   cgccccacga cccgcagcgc ccgaccgaaa ggagcgcacg accccataat agtaatcaat 2280
   tacggggtca ttagttcata gcccatatat ggagttccgc gttacataac ttacggtaaa 2340
   tggcccgcct ggctgaccgc ccaacgaccc ccgcccattg acgtcaataa tgacgtatgt 2400
   tcccatagta acgccaatag ggactttcca ttgacgtcaa tgggtggagt atttacggta 2460
   aactgcccac ttggcagtac atcaagtgta tcatatgcca agtacgcccc ctattgacgt 2520
   caatgacggt aaatggcccg cctggcatta tgcccagtac atgaccttat gggactttcc 2580
   tacttggcag tacatctacg tattagtcat cgctattacc atggtgatgc ggttttggca 2640
   gtacatcaat gggcgtggat agcggtttga ctcacgggga tttccaagtc tccaccccat 2700
   tgacgtcaat gggagtttgt tttggcacca aaatcaacgg gactttccaa aatgtcgtaa 2760
   caactccgcc ccattgacgc aaatgggcgg taggcgtgta cggtgggagg tctatataag 2820
   cagagctggt ttagtgaacc gtcagatccg ctagcgctac cggactcaga tctcgagctc 2880
   aagctcgata tcaagcttcg ccaccatggt gagcaagggc gaggagctgt tcaccggggt 2940
   ggtgcccatc ctggtcgagc tggacggcga cgtaaacggc cacaagttca gcgtgtccgg 3000
   cgagggcgag ggcgatgcca cctacggcaa gctgaccctg aagttcatct gcaccaccgg 3060
   caagctgccc gtgccctggc ccaccctcgt gaccaccctg acctacggcg tgcagtgctt 3120
   cagccgctac cccgaccaca tgaagcagca cgacttcttc aagtccgcca tgcccgaagg 3180
   ctacgtccag gagcgcacca tcttcttcaa ggacgacggc aactacaaga cccgcgccga 3240
   ggtgaagttc gagggcgaca ccctggtgaa ccgcatcgag ctgaagggca tcgacttcaa 3300
   ggaggacggc aacatcctgg ggcacaagct ggagtacaac tacaacagag cttatcgata 3360
   ccgtcgaggc cgcggatccc ccgggctgca ggaattcgag ctcggtaccc taatagtaat 3420
   caattacggg gtcattagtt catagcccat atatggagtt ccgcgttaca taacttacgg 3480
   taaatggccc gcctggctga ccgcccaacg acccccgccc attgacgtca ataatgacgt 3540
   atgttcccat agtaacgcca atagggactt tccattgacg tcaatgggtg gagtatttac 3600
   ggtaaactgc ccacttggca gtacatcaag tgtatcatat gccaagtacg ccccctattg 3660
   acgtcaatga cggtaaatgg cccgcctggc attatgccca gtacatgacc ttatgggact 3720
   ttcctacttg gcagtacatc tacgtattag tcatcgctat taccatggtg atgcggtttt 3780
   ggcagtacat caatgggcgt ggatagcggt ttgactcacg gggatttcca agtctccacc 3840
   ccattgacgt caatgggagt ttgttttggc accaaaatca acgggacttt ccaaaatgtc 3900
   gtaacaactc cgccccattg acgcaaatgg gcggtaggcg tgtacggtgg gaggtctata 3960
   taagcagagc tggtttagtg aaccgtcaga tccgctagcg ctaccggact cagatctcga 4020
   gctcaagctt cgccaccatg gtgcgctcct ccaagaacgt catcaaggag ttcatgcgct 4080
   tcaaggtgcg catggagggc accgtgaacg gccacgagtt cgagatcgag ggcgagggcg 4140
   agggccgccc ctacgagggc cacaacaccg tgaagctgaa ggtgaccaag ggcggccccc 4200
   tgcccttcgc ctgggacatc ctgtcccccc agttccagta cggctccaag gtgtacgtga 4260
   agcaccccgc cgacatcccc gactacaaga agctgtcctt ccccgagggc ttcaagtggg 4320
   agcgcgtgat gaacttcgag gacggcggcg tggtgaccgt gacccaggac tcctccctgc 4380
   aggacggctg cttcatctac aaggtgaagt tcatcggcgt gaacttcccc tccgacggcc 4440
   ccgtaatgca gaagaagacc atgggctggg aggcctccac cgagcgcctg tacccccgcg 4500
   acggcgtgct gaagggcgag atccacaagg ccctgaagct gaaggacggc ggccactacc 4560
   tggtggagtt caagtccatc tacatggcca agaagcccgt gcagctgccc ggctactact 4620
   acgtggactc caagctggac atcacctccc acaacgagga ctacaccatc gtggagcagt 4680
   acgagcgcac cgagggccgc caccacctgt tcctgtagcg gcctcgagct caagctcctg 4740
   gtgcatgacc cgcaagcccg gtgccatggt gagcaagggc gaggagctgt tcaccggggt 4800
   ggtgcccatc ctggtcgagc tggacggcga cgtaaacggc cacaagttca gcgtgtccgg 4860
   cgagggcgag ggcgatgcca cctacggcaa gctgaccctg aagttcatct gcaccaccgg 4920
   caagctgccc gtgccctggc ccaccctcgt gaccaccctg acctactagg gataacaggg 4980
   taatcctacg gcgtgcagtg cttcagccgc taccccgacc acatgaagca gcacgacttc 5040
   ttcaagtccg ccatgcccga aggctacgtc caggagcgca ccatcttctt caaggacgac 5100
   ggcaactaca agacccgcgc cgaggtgaag ttcgagggcg acaccctggt gaaccgcatc 5160
   gagctgaagg gcatcgactt caaggaggac ggcaacatcc tggggcacaa gctggagtac 5220
   aactacaaca gccacaacgt ctatatcatg gccgacaagc agaagaacgg catcaaggtg 5280
   aacttcaaga tccgccacaa catcgaggac ggcagcgtgc agctcgccga ccactaccag 5340
   cagaacaccc ccatcggcga cggccccgtg ctgctgcccg acaaccacta cctgagcacc 5400
   cagtccgccc tgagcaaaga ccccaacgag aagcgcgatc acatggtcct gctggagttc 5460
   gtgaccgccg ccgggatcac tctcggcatg gacgagctgt acaagtaaag cggccgcgac 5520
   tctagagtcg acctcgaggg ggggcccgcg attagtccaa tttgttaaag acaggatatc 5580
   agtggtccag gctctagttt tgactcaaca atatcaccag ctgaagccta tagagtacga 5640
   gccatagata gaataaaaga ttttatttag tctccagaaa aaggggggaa tgaaagaccc 5700
   cacctgtagg tttggcaagc tagcttaagt aagccatttt gcaaggcatg gaaaaataca 5760
   taactgagaa tagagaagtt cagatcaagg ttaggaacag agagacagga gaatatgggc 5820
   caaacaggat atctgtggta agcagttcct gccccggctc agggccaaga acagttggaa 5880
   cagcagaata tgggccaaac aggatatctg tggtaagcag ttcctgcccc ggctcagggc 5940
   caagaacaga tggtccccag atgcggtccc gccctcagca gtttctagag aaccatcaga 6000
   tgtttccagg gtgccccaag gacctgaaat gaccctgtgc cttatttgaa ctaaccaatc 6060
   agttcgcttc tcgcttctgt tcgcgcgctt ctgctccccg agctcaataa aagagcccac 6120
   aacccctcac tcggcgcgcc agtcctccga tagactgcgt cgcccgggta cccgtgttct 6180
   caataaaccc tcttgcagtt gcatccgact cgtggtctcg ctgttccttg ggagggtctc 6240
   ctctgagtga ttgactaccc gtcagcgggg gtctttcagt ttctcccacc tacacaggtc 6300
   tcactaacat tcctgatgtg ccgcagggac tccgtcagcc cggtttgtgt ttataataaa 6360
   atgcaagaac agtgttccct tcaagccaga ctacatcctg actctcggct ttataaaaga 6420
   atgttgaagg gctctgtgga ctatctgcca cacgactttt aagattttta tgcctcctgg 6480
   atgagggatt tagtcaatct atcctcgtct attttgctgg cttctccgta ttttaaattt 6540
   ctagtttgca ctcccttcct gagagcacgg cgattgcaga gtagttaata ctctgagggc 6600
   aggcttctgt gaaaaggttg cctgggctca gtgtgagatt ttgccataaa aaggggtcct 6660
   gcccctgtgt acagacagat cggaatctag agtgcatact cagagtcccc gcggttccgg 6720
   ggctctgatc tcagggcatc tttgcctaga gatcctctac gccggacgca tcgtggccgg 6780
   catcaccggc gccacaggtg cggttgctgg cgcctatatc gccgacatca ccgatgggga 6840
   agatcgggct cgccacttcg ggctcatgag cgcttgtttc ggcgtgggta tggtggcagg 6900
   ccccgtggcc gggggactgt tgggcgccat ctccttgcat gcaccattcc ttgcggcggc 6960
   ggtgctcaac ggcctcaacc tactactggg ctgcttccta atgcaggagt cgcataaggg 7020
   agagcgtcct gcattaatga atcggccaac gcgcggggag aggcggtttg cgtattgggc 7080
   gctcttccgc ttcctcgctc actgactcgc tgcgctcggt cgttcggctg cggcgagcgg 7140
   tatcagctca ctcaaaggcg gtaatacggt tatccacaga atcaggggat aacgcaggaa 7200
   agaacatgtg agcaaaaggc cagcaaaagg ccaggaaccg taaaaaggcc gcgttgctgg 7260
   cgtttttcca taggctccgc ccccctgacg agcatcacaa aaatcgacgc tcaagtcaga 7320
   ggtggcgaaa cccgacagga ctataaagat accaggcgtt tccccctgga agctccctcg 7380
   tgcgctctcc tgttccgacc ctgccgctta ccggatacct gtccgccttt ctcccttcgg 7440
   gaagcgtggc gctttctcaa tgctcacgct gtaggtatct cagttcggtg taggtcgttc 7500
   gctccaagct gggctgtgtg cacgaacccc ccgttcagcc cgaccgctgc gccttatccg 7560
   gtaactatcg tcttgagtcc aacccggtaa gacacgactt atcgccactg gcagcagcca 7620
   ctggtaacag gattagcaga gcgaggtatg taggcggtgc tacagagttc ttgaagtggt 7680
   ggcctaacta cggctacact agaaggacag tatttggtat ctgcgctctg ctgaagccag 7740
   ttaccttcgg aaaaagagtt ggtagctctt gatccggcaa acaaaccacc gctggtagcg 7800
   gtggtttttt tgtttgcaag cagcagatta cgcgcagaaa aaaaggatct caagaagatc 7860
   ctttgatctt ttctacgggg tctgacgctc agtggaacga aaactcacgt taagggattt 7920
   tggtcatgag attatcaaaa aggatcttca cctagatcct tttaaattaa aaatgaagtt 7980
   ttaaatcaat ctaaagtata tatgagtaaa cttggtctga cagttaccaa tgcttaatca 8040
   gtgaggcacc tatctcagcg atctgtctat ttcgttcatc catagttgcc tgactccccg 8100
   tcgtgtagat aactacgata cgggagggct taccatctgg ccccagtgct gcaatgatac 8160
   cgcgagaccc acgctcaccg gctccagatt tatcagcaat aaaccagcca gccggaaggg 8220
   ccgagcgcag aagtggtcct gcaactttat ccgcctccat ccagtctatt aattgttgcc 8280
   gggaagctag agtaagtagt tcgccagtta atagtttgcg caacgttgtt gccattgcta 8340
   caggcatcgt ggtgtcacgc tcgtcgtttg gtatggcttc attcagctcc ggttcccaac 8400
   gatcaaggcg agttacatga tcccccatgt tgtgcaaaaa agcggttagc tccttcggtc 8460
   ctccgatcgt tgtcagaagt aagttggccg cagtgttatc actcatggtt atggcagcac 8520
   tgcataattc tcttactgtc atgccatccg taagatgctt ttctgtgact ggtgagtact 8580
   caaccaagtc attctgagaa tagtgtatgc ggcgaccgag ttgctcttgc ccggcgtcaa 8640
   tacgggataa taccgcgcca catagcagaa ctttaaaagt gctcatcatt ggaaaacgtt 8700
   cttcggggcg aaaactctca aggatcttac cgctgttgag atccagttcg atgtaaccca 8760
   ctcgtgcacc caactgatct tcagcatctt ttactttcac cagcgtttct gggtgagcaa 8820
   aaacaggaag gcaaaatgcc gcaaaaaagg gaataagggc gacacggaaa tgttgaatac 8880
   tcatactctt cctttttcaa tattattgaa gcatttatca gggttattgt ctcatgagcg 8940
   gatacatatt tgaatgtatt tagaaaaata aacaaatagg ggttccgcgc acatttcccc 9000
   gaaaagtgcc acctgacgtc taagaaacca ttattatcat gacattaacc tataaaaata 9060
   ggcgtatcac gaggcccttt cgtctcgcgc gtttcggtga tgacggtgaa aacctctgac 9120
   acatgcagct cccggagacg gtcacagctt gtctgtaagc ggatgccggg agcagacaag 9180
   cccgtcaggg cgcgtcagcg ggtgttggcg ggtgtcgggg ctggcttaac tatgcggcat 9240
   cagagcagat tgtactgaga gtgcaccata tgcggtgtga aataccgcac agatgcgtaa 9300
   ggagaaaata ccgcatcagg                                            9320

## Beschreibung

### 1. Gegenstand der Erfindung:

Gegenstand der Erfindung ist ein Verfahren, welches den Nachweis von gentoxischem Stress in lebenden Säugerzellen durch Detektion aller bisher beschriebenen Typen der DNA-Rekombination und weiterhin von Mutationen als Antwort auf Noxen ermöglicht. Der Nachweis basiert auf der Detektion der Fluoreszenz intakter autofluoreszierender Proteine oder der Lumineszenz mittels intakter biolumineszierender oder Chemilumineszenz-Substrate umsetzender Enzyme, zeichnet sich durch eine kurze. Reaktionszeit im Stundenbereich aus und erlaubt insbesondere durch die Verwendung eines retroviralen Vektorgerüstes die Übertragung des Testsystems auf unterschiedliche Säugerzellen und auf lebende Versuchstiere.

### 2. Stand der Technik:

Der Ausschluß möglicher Gentoxizitäten stellt eine der Grundvoraussetzungen für die Zulassung von neuen NahrungsBestandteilen, Kosmetika und Medikamenten dar. Die existierenden Verfahren zum Nachweis von Gentoxizitäten lassen sich aufgrund der Bestimmung unterschiedlicher biologischer Antworten auf die Behandlung mit einer gentoxischen Substanz unterteilen: In Tierversuchen werden neben der Entstehung von Tumoren physiologische Parameter, wie Änderungen des Körpergewichts, des Wasserverbrauchs, des Blutprofils, der Blutchemie oder histopathologische Veränderungen festgestellt (Burdock, G.A. et al. (2000) Food Chem. Toxicol. 38: 429-442). Auf molekularer Ebene haben sich überwiegend Verfahren zum Nachweis genetischer Veränderungen durchgesetzt. So werden größere Chromosomen-Rearrangements zytogenetisch als Anstieg von Schwesterchromatid-Austäuschen (SCE) in menschlichen oder murinen Knochenmarkszellen abgeschätzt (Hadagny, W. et al. (1989) Mutat. Res. 225: 27-32; Burdock, G.A. et al. (2000) Food Chem. Toxicol. 38: 429-442), die Entstehung von Mikronuklei in Lymphozyten exponierter Personen oder in den Zellen von Indikatororganismen, z.B. der Pflanze Tradescantia, beobachtet (Kazimierz, G. und Brokos, B. (2000) Mutagenesis 15: 137-141; Monarca, S. et al. (1999) Mutat. Res. 426: 189-192), Mutationen im HPRT-Gen über das Auftauchen von gegenüber 6-Thioguanin resistenten Zell-Klonen im Verlauf der Zucht von Säugerzellen nach Schadstoff-Exposition gemessen (O' Donovan, M.R. et al. (1990) Mutagenesis 5: 275-277) oder genetische Veränderungen in der DNA von Shuttle-Vektoren analysiert, indem die Mutationen phänotypisch nach Transfer der Vektor-DNA vom Testorganismus in Bakterien nachgewiesen werden (de Groene, E.M. et al. (1995) Eur. J. Pharmacol. 293: 47-53). Desweiteren stehen der Nachweis unreparierter DNA-Strangbrüche in Lymphozyten-DNAs durch den sogenannten *Comet Assay* (Somorovska, M. et al. (1999) Mutat. Res. 445: 181-192) und die Detektion erhöhter DNA-Reparatur-Synthese-Aktivitäten in primären Rattenleberzellen (Williams, G.M. (1977) Cancer. Res. 37: 1845-1851) als Indikatoren für vorangegangene DNA-Schädigung zur Verfügung.

Die Aussagekraft der erwähnten physiologischen Untersuchungen am lebenden Tier im Hinblick auf die Kanzerogenität bestimmter Substanzen ist eingeschränkt, der Nachweis von tatsächlich entstandenen Tumoren im Hinblick auf die primären Schäden sehr unsensitiv, da der Entstehung eines Tumors die Veränderung tausender von Genen vorausgeht (Loeb, L. und Loeb, L.A. (2000) Carcinogenesis 21: 379-385). Jede der oben beschriebenen molekularen Methoden erfaßt Genom-Schäden, welche in Zellen des eukaryontischen Zielorganismus enstanden sind, und berücksichtigt damit bei der Bewertung von potentiell gentoxischen Substanzen die mögliche metabolische Aktivierung von mutagenen Effekten. Trotzdem haben diese Methoden gewisse Limitationen: Die Feststellung von SCEs oder von Micronuklei erfordert die Präparation, Fixierung und Färbung des Zellmaterials sowie eine anschließende mikroskopische Untersuchung. Darüberhinaus bedarf es für den SCE-Nachweis zusätzlich einer Colchizin- oder Bromdesoxyuridin-Behandlung vor der Präparation, für den Mikronukleus *Assay* mit Cytochalasin B, deren Einflüsse auf die Behandlungen mit den eigentlich zu analysierenden Substanzen ungeklärt sind. Der Nachweis von 6-Thioguanin-Resistenzen in eukaryontischen Zellen erfordert entweder die langwierige Zucht, während derer die ursprüngliche Wirkung des toxischen Agens modifiziert werden kann, oder mikroskopische Analysen nach Bromdesoxyuridin-Behandlung. Das Arbeiten mit *Shuttle*-Vektoren macht die Rückgewinnung der Vektor-DNA aus den Eukaryonten-Zellen und deren Wiedereinbringung in Bakterien nötig. Während des letzteren, zumeist chemischen Vorgangs ist die Vektor-DNA verstärkt den Angriffen durch die bakteriellen, die DNA modifizierenden Enzymen ausgesetzt, was zu einer scheinbaren Erhöhung der Mutagenizität des ursprünglich an Säugerzellen analysierten Agens führen kann. Den gleichen Effekt können geringfügige physiologische Störungen bei den verwendeten Bakterien hervorrufen, da deren Mutationsraten bereits ohne äußere Einflüsse um 1-2 Größenordnungen über denen von Eukaryontenzellen liegen (Friedberg, E.C. (1984) in DNA Repair, W.H. Freeman and Company, New York). Die wohl größten Schwierigkeiten beim *Comet Assay* wie auch bei der Quantifizierung von DNA-Reparatur-Synthese-Aktivitäten liegen in der starken Schwankungsbreite der Ergebnisse, so daß zur Erlangung statistisch relevanter Daten zahlreiche Mehrfachbestimmungen möglichst unter automatisierter Datenerfassung durchgeführt werden müssen. Darüberhinaus geht der Bestimmung von DNA-Reparatur-Synthese-Aktivitäten zur Detektion meist eine Behandlung mit radioaktiv markierten Nukleotidvorstufen mit wiederum unvollständig geklärter Wirkung auf die Effekte der zu untersuchenden Noxen voraus.

In einigen Fällen wurden bei Eukaryonten auch Änderungen im Genexpressionmuster für die Darstellung der zellulären Antwort auf DNA-Schädigung benützt (Billinton, N. et al. (1998) Biosensors & Bioelectronics 13: 831-838; Todd, M.D. et al. (1999) J. Biomol. Screen 4: 259-268). Beim Genexpressions-Nachweis handelt es sich generell um eine Technik, welche im Zuge der Chip Array-Verfahren zunehmend an Bedeutung gewinnen könnte. Als Nachteil dieser Methode muß jedoch die meist damit verbundene Notwendigkeit der Nukleinsäure- bzw. Proteinextraktion erwähnt werden. Diese erhöht nicht nur den Arbeits- und Zeitaufwand des Nachweises, sondern birgt auch die Gefahr mangelnder Reproduzierbarkeit in sich. In einem Verfahren, welches auf dem Nachweis der Anschaltung des durch DNA-Schäden induzierbaren Genes *Rad54* beruht, wurde der Nukleinsäure-/Proteinextraktions-Schritt durch Kopplung des induzierbaren *Rad54*-Promotors an eine nachgeschaltete *EGFP*-cDNA unnötig gemacht (Billinton, N. et al. (1998) Biosensors & Bioelectronics 13: 831-838). Hier zeigte das durch das Genprodukt EGFP (Enhanced Green *Fluorescence* Protein) verursachte grüne Leuchten DNA-Schädigung an. Nach Anregung der EGFP-Autofluoreszenz durch Licht der Wellenlänge von 488 nm konnte die Lichtemission bei einer Wellenlänge von 511 nm fluorometrisch bestimmt werden. Als entscheidende Nachteile dieser Methode müssen gewertet werden, daß diese erstens für *Saccharomyces cerevisiae* und nicht für Säugerzellen etabliert wurde und zweitens, daß die Fluoreszenzen erst nach einer Reihe von Extraktionsschritten, also nicht an intakten Zellen, gemessen werden konnten.

Der mit Abstand am häufigsten verwendete Gentoxizitäts-Assay ist der Ames *Assay* (Ames, B.N. et al. (1973) Proc. Natl. Acad. Sci. USA 70: 2281-2285; Maron, D.M. und Ames, B.N. (1983) Mutat. Res. 113: 173-215). Der *Assay* wird in der pharmazeutischen Industrie verwendet, um während der Entwicklung neuer Drogen Vorauswahlen treffen zu können und um diese im Zulassungsverfahren zu bewerten. Dieser *Assay* basiert auf dem Nachweis von reversen, d.h. rekonstituierenden Punkmutationen im his-Gen einer Defekt-Mutante von *Salmonella typhimurium,* indem Wachstum in Ansätzen ohne Histidin quantifiziert wird. Die Mutationen können je nach verwendetem *Ames-Salmonella-*Stamm und je nach Schadstoff vom Typ der Basensubstitutionen, Leserasterverschiebungen oder komplexen Leserasterverschiebungen mit benachbarten Basensubstitutionen sein, und durch das Agens selbst oder durch nachgeschaltete fehlerhafte Reparatur-Vorgänge verursacht werden.

Der Ames *Assay* hat den entscheidenden Nachteil, daß *Salmonella* die zelluläre Maschinerie der Eukaryonten nicht besitzt, welche für die Umwandlung von Prokarzinogenen in die reaktiven Metaboliten nötig ist. Aus diesem Grunde werden zahlreiche, z.B. beim Menschen, gentoxisch wirkende Substanzen mit diesem Test nicht erfaßt. Dies trifft auch auf neuere bakterielle *Assays,* wie auf den *SOS* Chromotest zu (Quillardet, P. et al. (1982) Proc. Natl. Acad. Sci. USA 79: 5971-5975). Zwar wurde versucht, den *Ames Assay* durch Zugabe von Rattenleber-Extrakten zum Testansatz zu optimieren, um den menschlichen Stoffwechsel zu simulieren, doch diese Extrakte enthalten möglicherweise nicht genügend der Enzym-Aktivitäten, welche für die Metabolisierung erforderlich sind (Ames, B.N. (1974) Genetics 78: 91-95). Weiterhin erschwert die Methodik des Ames *Assay* die Automatisierung als Grundlage für standardisierte Überwachungsverfahren. Dazu kommt, daß die Mehrheit aller DNA-Schäden fehlerfrei repariert werden. Im *Ames Assay* wird jedoch nur fehlerhafte DNA-Reparatur gemessen, was zu einer Unterschätzung der DNA-Schädigung führt. Darüberhinaus bleiben größere Chromosomen-Rearrangements, wie Insertionen, Deletionen oder Translokationen, mit diesem Assay unentdeckt. So waren beispielsweise o-Toluidin und o-Anisidin im *Ames Assay* negativ, obwohl diese Substanzen in Mäusen und Ratten Tumore verursachen (Gold, L.S. et al. (1991) Environ. Health Perspect. 100: 65-168). Demgegenüber konnte für o-Toluidin die Entstehung von Strangbrüchen und gesteigerte DNA-Reparatur-Synthese-Aktivitäten nachgewiesen werden (Danford N. (1991) Mutat. Res. 258: 207-236). Für o-Anisidin konnten bis zum letzten Jahr nur aufgrund der geringfügigen Zunahme von Punkmutationen in der Blase der sogenannten *Big Blue* Indikator-Mäuse (siehe unten) Anzeichen für Gentoxizitäten gewonnen werden (Ashby J. et al. (1991) Carcinogenesis 15: 2291-2296). Zusammenfassend sind viele beim Menschen mutagen oder karzinogen wirkenden Substanzen derzeit als solche mit den bisherigen Gentoxizitäts-Nachweisverfahren noch nicht eindeutig nachweisbar. Mit dem Ames *Assay* werden nur 50 % aller Karzinogene erfaßt (Ashby, J. und Tennant, R.W. (1991) Mutat Res. 257: 229-306).

Um der Tatsache Rechnung zu tragen, daß größere Genom-Rearrangements als die im Ames *Assay* geprüften Punktmutationen krebsverursachend sind (Tlsty, T.D. et al. (1995) Mutat Res. 337: 1-7; Sandberg, A.A. (1991) Mutat. Res. 247: 231-240; Bishop, J.M. (1987) Science 235: 305-311; Loeb, L. und Loeb, L.A. (2000) Carcinogenesis 21: 379-385), wurden von Schiestl und Kollegen (Brennan, R.J. und Schiestl, R.H. (1999) Mutat. Res. 430: 37-45) Nachweissysteme entwickelt (*DEL Assay*), welche Deletions-Ereignisse auf genomischer Ebene detektieren können. Deletierende Rekombinations-Ereignisse werden in Hefen und Säugerzellen von einer Vielzahl von Karzinogenen, wie z.B. Anilinen und o-Toluidin ausgelöst, und sind mit Hilfe der meisten existierenden Gentoxizitäts-Nachweisverfahren nicht darstellbar (Schiestl, R.H. (1989) Nature 337: 285-288; Brennan, R.J. et al. (1994) Mutat. Res. 308: 159-167; Carls, N. und Schiestl, R.H. (1994) Mutat. Res. 320: 293-303; Aubrecht, J. et al. (1995) Carcinogenesis 16: 2841-2846; Schiestl, R.H: et al. (1997) Proc. Natl. Acad. Sci. USA 94: 4576-4581). Der für den HIS3-Lokus der Hefe *Saccharomyces cerevisiae* etablierte *DEL Assay* leidet unter den bereits für andere Verfahren dargestellten Limitationen, nämlich daß die für Säuger spezifische Metabolisierung von Prokarzinogenen unberücksichtigt bleibt, und daß Überlebensraten entweder durch Zucht unter bestimmten Selektionsbedingungen oder durch Bestimmung der Aufnahme einer fluoreszierenden Substanz an Zellextrakten gemessen werden (Brennan, R.J. und Schiestl, R.H. (1999) Mutat. Res. 430: 37-45).

Der Nachweis von durch Schadstoffe stimulierten Rekombinations-Ereignissen in situ, also am lebenden Tier, birgt im Vergleich zu den bisher aufgeführten Methoden weit interessantere Möglichkeiten und muß deswegen für die Routine-Bestimmungen in der pharmazeutischen Industrie vorbereitet werden. Erst im Tiermodell ist die Analyse der Genomstabilität als Antwort auf die Einwirkung von Strahlung oder gentoxischen Agentien in Abhängigkeit von physiologischen Prozessen und in Abhängigkeit vom Genotyp des Tieres möglich. Eine gezielte Änderungen von Genotypen wird heute durch Herstellen von sogenannten *Knockout* oder transgenen Mäusen, also von Mäusen mit bestimmten Gendefekten oder zusätzlichen Genkopien, bzw. durch das Einkreuzen solcher genetisch veränderter Mäuse bewerkstelligt. Durch die Einbringung eines Gentoxizitäts-Nachweissystems in genetisch veränderte Mäuse könnten neue Fragen gestellt werden, wie nach der Bedeutung einzelner Gene für die Suszeptibilität des Tieres oder eines bestimmten Gewebes für die gentoxische Wirkung von Mutagenen. Dies repräsentiert einen äußerst zukunftsträchtigen Ansatz, da in den nächsten Jahren durch den Einzug der Chip *Array*-Technologie in moderne Kliniken und Praxen individuelle Genotypen für einzelne Patienten erstellt werden können. Die gleichzeitige Kenntnis des individuellen Genotyps eines Krebspatienten (gesundes und Tumor-Gewebe) und der Bedeutung der bei diesem Patienten veränderten Gene für die Antwort auf bestimmte Noxen aufgrund von Gentoxizitäts-Studien in den entsprechenden *Knockout* oder transgenen Mäusen würde die Entwicklung eines individuell optimierten Chemotherapieprotokolls ermöglichen. So ist insbesondere für einige Tumorsuppressorproteine bekannt, daß deren funktioneller Ausfall genomische Instabilitäten fördert bzw. sogar Resistenzen gegenüber Mutagenen bewirkt, so daß Tumorzellen gegenüber dem gesunden Gewebe unter diesen genetischen Voraussetzungen sogar Wachstumsvorteile besäßen. Dies trifft insbesondere für den Tumorsuppressor p53 zu, der Wachstum und DNA-Reparatur reguliert, und der sogar je nach Mutation im p53-Gen Sensitivität bzw. Resistenz gegenüber chemotherapeutischen Behandlungen auslöst (McGilland, G. und Fisher, D.E. (1999) J. Clin Invest. 104: 223-225; Albrechtsen, N. et a1. (1999) Oncogene 18: 7706-7717; Wiesmüller, L. (2000) J. Biomed. Biotech. 1: 7-10).

Bisher wurden zur Gentoxizitäts-Bestimmung Testsysteme in lebenden Tieren generiert, welche auf der Basis der Rekonstitution der ß-Galaktosidase-Aktivität entweder für Messungen der Punktmutations-Häufigkeiten im *lacI-*Gen in der *Big Blue Mouse* (Stratagene) oder für Messungen rekonstituierender Rekombinations-Häufigkeiten im *lacZ*-Gen in männlichen Keimzellen der transgenen Mäuse (Akgün, E. et al. (1997) Mol. Cell. Biol. 517: 5559-5570) zur Verfügung stehen. Ein schwerwiegender Nachteil der sogenannten *Big Blue Mouse* (Stratagene) und davon abgeleiteten transgenen Mäusen ist, daß das Mutations-Indikator-Gen, hier das *lacI-*Gen*,* welches als Repressor der ß-Galaktosidase-Expression fungiert, nach gentoxischer Behandlung der Maus aus dem Genom über λ-Phagen in Bakterien transduziert werden muß. Erst in den Bakterien wird die Aktivität des Genproduktes über das chromogene β-Galaktosidase-Substrat X-Gal (Boehringer Mannheim) durch Blaufärbung nachgewiesen. Da jedoch, wie erwähnt, Mutationsraten in Bakterien und noch viel mehr in λ-Phagen-Genomen um Größenordnungen über denen in Säugerzellen liegen, muß mit einer für Säuger unphysiologisch hohen Basalrate gerechnet werden (Friedberg, E.C. (1984) in DNA Repair, W.H. Freeman and Company, New York). Dies könnte die mit der *Big Blue Mouse* gemessenen Raten von 10⁻⁵ bis 10⁻³ pro Lokus erklären. Das zweite Testsystem von Akgün und Kollegen (Moynahan, M. E. et a1. (1996). Hum. Mol. Genetics. 5: 875-886; Akgün, E. et a1. (1997) Mol. Cell. Biol. 517: 5559-5570) wurde zur Beantwortung von Fragen zum Einfluß der Keimzellenentwicklung nicht aber der Schadstoff-Einwirkung entwickelt. Hier wurde eine von der β-Galaktosidase in eine fluoreszierende Form konvertierte Chemikalie (5-Chloromethylfluorescein-di-β-D-Galaktopyranosid) zum Nachweis des rekonstituierten *lacZ-*Gens verwendet. Bei diesem *Assay* können jedoch aufgrund des Arrangements der Sequenz-Elemente genomische Veränderungen ausschließlich eines einzigen homologen Rekombinations-Subtyps, nämlich der Genkonversion, und dies ausschließlich in männlichen Keimbahnzellen, aufgezeigt werden.

Schließlich wurde kürzlich das sogenannte *Pink Eyed Unstable* (*p*^{un}) Mausmodell der Firma Jackson Laboratory (Bar Harbor, ME) zur Messung von größeren DNA-Rearrangements nach Röntgenbestrahlung und nach Benzo[a]pyren-Behandlung der Tiere verwendet (Aubrecht, J. et al. (1999) Carcinogenesis 20: 2229-2236). *p*^{un} Mäuse tragen in ihrem Genom Tandemduplikationen des 70 kiloBasenpaar langen *p*-Gens, wodurch das Gen instabil, d.h. für größere Rearrangements vom Typ der homologen Rekombination prädestiniert ist. Das Wildtyp-Gen wird in Melanozyten exprimiert und kodiert für ein integrales Membranprotein, welches für die Assemblierung eines hochmolekularen Komplexes nötig ist, welcher schwarze Pigmentierung im Fell der Mäuse verursacht (Rosemblat, S. et al. (1994) Proc. Natl. Acad. Sci. USA 91: m12071-12075). So konnte die Deletion einer Kopie der duplizierten Sequenzen und die damit verbundene Rekonstitution des Wildtyp-Gens durch das Auftauchen schwarzer Flecken im grauen Fell beobachtet werden. Der Nachteil dieser Methode ist, daß die rekombinativen Ereignisse ausschließlich dann nachweisbar sind, wenn diese in embryonalen Prämelanozyten abgelaufen sind, so daß statistisch signifikante Toxizitäts-Bestimmungen die Analyse einer Vielzahl von im Embryonalstadium behandelter Mäuse erfordern würden.

Aufgabe der vorliegenden Erfindung war es daher, eine Alternative zum AMES-Test zur Verfügung zu stellen und ein einfaches, verläßliches Testsystem oder Testverfahren (Assay) zu entwickeln, mit dem sich Substanzen darauf untersuchen lassen, ob sie Genrekombinationen auslösen und somit gentoxisch wirken. Vorzugsweise soll dieses Testsystem geeignet sein, die Art der Rekombinationsereignisse zu ermitteln.

Die Aufgabe wird erfindungsgemäß durch die Entwicklung eines Verfahrens gelöst, welches nach Insertion besonderer Vektoren in das Genom von Zellen Gen-Rekombinations-Prozesse als Antwort auf gentoxische Behandlung durch Expression autofluores-zierender Proteine anzeigt.

### 3. Beschreibung der Erfindung:

Gegenstand der vorliegenden Erfindung ist insbesondere ein in vitro-Verfahren zur Bestimmung von Gentoxizitäten, bei dem man eukaryontische Zellen, die mit einem Vektor transfiziert sind, der den allgemeinen Aufbau
5'-[*Ins1*]-[*Prom1*]-[*Marker1*]-[*Prom2*]-[*Ins2*]-[*Marker2*]-3'
aufweist, wobei
- [*Ins1*]: ein Sequenzabschnitt ist, der für einen Selektions-marker (insbesondere ein Puromycin-oder Hygromycin-Resistenzgen), für ein autofluoreszierendes Protein (AFP), für ein biolumineszierendes oder ein Chemilumineszenz-Substrate umsetzendes Enzym (LE) oder eine Mutante derselben kodiert, oder völlig fehlen kann,
- [*Prom1*]: ein Promotor ist oder völlig fehlen kann,
- [*Marker1*]: ein Sequenzabschnitt ist, der für ein Derivat oder eine Mutante eines biolumineszierenden oder autofluoreszierenden Proteins (AFP) oder eines Chemolumineszenz-Substrate umsetzenden Enzyms (LE) kodiert,
- [*Prom2*]: ein Promotor ist,
- *[Ins2]*: ein Sequenzabschnitt ist, der für für ein AFP, LE oder eine Mutante derselben oder für I-*Sce*I, kodiert oder ein Abstandhalter, vorzugsweise mit einer Länge von 1 kB, ist,
- [*Marker2*]: ein Sequenzabschnitt ist, der für ein Derivat oder eine Mutante eines autofluoreszierenden Proteins oder eines biolumineszierenden oder eines Chemolumineszenz-Substrate umsetzendes Enzym (AFP oder LE) kodiert,
wobei [*Marker1*] und [*Marker2*] zwei über mindestens 200 Basenpaare homologe DNA-Sequenz-Abschnitte sind, die mutierte Varianten eines Gens sind, aus denen sich durch homologen DNA-Austausch das Wildtyp-Gen (AFP oder LE) rekonstituiert, wobei es bei nicht-konservativer homologer Rekombination oder Replikationssprüngen zwischen [*Marker1*] und *[Marker2]* zum Verlust von [*Ins2*] kommt, wobei es bei inaktivierenden Mutationen in [*Ins1*] oder [*Ins2*] zur Inaktivierung des jeweiligen Genproduktes kommt, wobei es bei revertierenden Mutationen in [*Ins1*] oder *[Ins2]* zur Aktivierung des jeweiligen Genproduktes kommt;
oder eukaryontische Zellen, die mit einem Retrovirus infiziert sind, der einen solchen Vektor enthält,
oder einen transgenen nicht-menschlichen Säuger, dessen Keimzellen oder somatische Zellen die Sequenz eines solchen Vektors umfassen,
mit einer Testsubstanz in Kontakt bringt, und man mittels FACS-Analyse, Fluoreszenz-Messung, Fluoreszenz-Mikroskopie, Laser Scanning Cytometrie oder mittels einer Lumineszenz-Nachweis-Reaktion ein Auftauchen, eine Zunahme oder Abnahme von fluoreszierenden oder lumineszierenden Zellen entsprechend der Aktivität des Wildtyp-Genproduktes von [*Marker1*]*,* [*Marker2*]*,* [*Ins1*] oder [*Ins2*] in den genannten Zellen oder in Zellen des genannten Säugers nachweist,
wobei die Zellen keine menschlichen kümzellen sind.

Unter "Homologie" wird im Zusammenhang mit der Erfindung eine perfekte Homologie verstanden, d.h. idealerweise eine Identität zwischen den entsprechenden Sequenzabschnitten oder mit Sequenzunterschieden, welche in Abständen von nicht weniger als etwa 150 Bp innerhalb der ansonsten identischen Sequenzen vorliegen.

Bei den erfindungsgemäß verwendeten Vektoren, die vorliegend beschrieben sind, handelt es sich vorzugsweise um retrovirale Vektoren.

Unter "Wildtyp-Gen" wird in diesem Zusammenhang das Wildtyp-Gen im eigentlichen (engeren) Sinne verstanden, wobei im Rahmen der vorliegenden Erfindung auch Varianten bzw. Mutanten dieses Gens eingeschlossen sind, welche Autofluoreszenz-Aktivität bzw. Biolumineszenz oder Chemilumineszenz Substrate umsetzende Aktivität auf weisen. Das bedeutet, daß als [*Marker1*] und [*Marker2*] zwei homologe DNA-Sequenz-Abschnitte verwendet werden, aus denen sich in einer Ausführungsform bei homologer Rekombination durch homologen DNA-Austausch das Wildtyp-Gen rekonstituiert, so daß die Autofluoreszenz- oder EnzymAktivität des Genprodukts nachweisbar wird. Der Vektor zeichnet sich weiter dadurch aus, daß es bei nicht-konservativer homologer Rekombination oder Replikationssprüngen zwischen [*Marker1*] und [*Marker2*] zum Verlust (Zerstörung von *[Ins2])* oder Änderung von *[Ins2]* kommt. Der Vektor zeichnet sich weiter dadurch aus, daß es bei inaktivierenden Mutationen in [*Ins1*] oder *[Ins2]* zur Inaktivierung des jeweiligen Genproduktes (Verlust der Funktion der Wildtyp-Gene), es bei revertierenden Mutationen in [*Ins1*] oder *[Ins2]* zur Aktivierung des jeweiligen Genproduktes (Rekonstitution der Wildtyp-Funktion) kommt.

Für [*Ins1*] kommen im Gegensatz zu [*Ins2*] z.B. solche Gene nicht in Betracht, welche wie I-SceI streng induzierbar exprimiert werden sollen, da für [*Ins1*] der 5'LTR-Promotor aufgrund des retroviralen Vektordesigns nicht austauschbar ist.

Bei dem erfindungsgemäß verwendeten Vektor-Konstrukt ist [*Ins1*] vorzugsweise ein Sequenzabschnitt, der eine für einen Selektionsmarker kodierende Nukleinsäuresequenz, wie z.B. ein Antibiotika-Resistenzgen (beispielsweise das Puromycin- oder Hygromycin-Resistenzgen oder ein ähnlich kleines Gen kodierend für einen alternativen Selektionsmarker), eine kodierende Nukleinsäuresefür ein autofluoreszierendes Protein (AFP), für ein biolumineszierendes oder ein Chemilumineszenz-Substrate umsetzendes Enzym (LE) oder für eine Mutante derselben kodierende Nukleinsäuresequenz enthält.

Soweit im Rahmen der vorliegenden Erfindung Tetrazyklin oder Östradiol responsive Promotoren oder Transkriptionsfaktoren, wie z.B. rtTA oder GalER-VP, verwendet werden, existieren zahlreiche weitere Varianten derselben, die erfindungsgemäß ebenfalls eingeschlossen sind.

Der Vektor, bei dem [*Ins1*] ein Sequenzabschnitt ist, der eine für ein autofluoreszierendes Protein, für ein biolumineszierendes oder ein Chemilumineszenz-Substrate umsetzendes Enzym oder für eine Mutante derselben kodierende Nukleinsäuresequenz enthält, zeichnet sich dadurch aus, daß bei Mutationen in *[Ins1]* das kodierte AFP oder LE inaktiviert werden kann. Soweit [*Ins1*] ein durch Mutation inaktiviertes AFP- oder LE-Gen darstellt, werden revertierende Mutationen in [*Ins1*] durch das Auftauchen eines Fluoreszenz- oder Chemilumineszenz-Signals des Produktes des jeweiligen Wildtyp-Gens nachweisbar.

[*Ins2*] ist vorzugsweise ein Sequenzabschnitt, der ein AFP-Gen (beispielsweise das DsRed-Gen), ein LE-Gen (beispielweise kodierend für die alkalische Phosphatase) oder ein abgeleitetes Mutanten-Gen derselben, oder einen Abstandhalter mit bevorzugter Länge von 1 kB enthält.

Soweit die Sequenzabschnitte [*Ins1*] und [*Ins2*] für AFP- oder LE-Gene kodierende Nukleinsäuresequenzen enthalten, muß sichergestellt sein, daß das jeweilige AFP- oder LE-Gen keine signifikanten Homologien zu eventuellen AFP- oder LE-Genen im *[Marker1]* und im [*Marker2*] aufweist und daß die Genprodukte im Falle von AFP-Genen ein Fluoreszenz-Emissionsspektrum aufweisen, das deutlich von dem der von den Marker-Genen kodierten AFPs verschieden ist. Dies bedeutet, daß ein Verlust von *[Ins2]* nach erfolgter nicht-konservativer homologer Rekombination zwischen den DNA-Abschnitten [*Marker1*] und *[Marker2]* wie auch inaktivierende Mutationen in *[Ins2]* bzw. in [*Ins1*] durch den Verlust des Fluoreszenz- oder Lumineszenz-Signals des Produktes des jeweiligen Wildtyp-Gens separat nachweisbar sind. Soweit [*Ins1*] und *[Ins2]* durch Mutation inaktivierte AFP- oder LE-Gene darstellen, sind revertierende Mutationen in *[Ins2]* bzw. in *[Ins1]* durch das Auftauchen des spezifischen Fluoreszenz- oder Chemilumineszenz-Signals des Produktes des jeweiligen Wildtyp-Gens nachweisbar.

Soweit es sich bei *[Ins2]* um einen Sequenzabschnitt handelt, der eine für ein autofluoreszierendes Protein oder eine für ein biolumineszierendes oder ein Chemolumineszenz-Substrate umsetzendes Enzym oder für eine Mutante derselben kodierende Nukleinsäuresequenz enthält, zeichnet sich dieser Vektor dadurch aus, daß bei nicht-konservativer homologer Rekombination oder Replikationsprüngen zwischen den Sequenzabschnitten *[Marker1]* und *[Marker2]* oder bei inaktivierenden Mutationen in *[Ins2]* dieses *[Ins2]* verloren geht bzw. das kodierte AFP oder LE inaktiviert werden kann. Soweit *[Ins2]* ein durch Mutation inaktiviertes AFP- oder LE-Gen darstellt, sind auch revertierende Mutationen in *[Ins2]* durch das Auftauchen eines Lumineszenz- oder Chemilumineszenz-Signals des Produktes des jeweiligen Wildtyp-Gens nachweisbar.

Gemäß einer Ausführungsform der Erfindung können die Sequenz-abschnitte *[ [Prom1] -* [*Marker1*]] und *[Marker2]* an ihren Positionen ausgetauscht werden, wenn gleichzeitig die Orientierung beider Sequenzabschnitte umgedreht wird (d.h. von 5'->3' nach 3'->5'). Bei Vektoren, die für den Nachweis von nichtkonservativen homologen Rekombinations-Ereignissen (nkHR) nicht geeignet sind, darf ohne Orientierungsänderung getauscht und die jeweilige Orientierung von [[*Prom1*] *-* [*Marker1*]] und/oder [*Marker2*] ohne Austausch geändert werden. Die resultierende Sequenz entspricht demnach 5'- [*Ins1*] - (3'-[*Marker1*] - [*Prom1*] -5') - [*Prom2*] - [*Ins2*] *-* [*Marker2*] *-* 3', 5' - [*Ins1*] - [*Prom1*] *-* [*Marker1*] *-* [*Prom2*]*-*[*Ins2*]*-*(3'-[*Marker2*]-5')-3', 5'*-*[*Ins1*]*-*(3'*-*[*Marker1*]-[*Prom1*] *-* 5') *-* [*Prom2*] *-* [*Ins2*] *-* (3' - [*Marker2*] - 5') - 3' oder 5' - [*Ins1*] - *[Marker2] -* [*Prom2*] *-* [*Ins2*] *-* ([*Prom1*] *-* [*Marker1*]) *-* 3'

Im Rahmen der Erfindung wird ferner ein Vektor verwendet, der von den genannten. Vektoren abgeleitet ist, indem die Orientierung des Sequenzabschnitts [[*Prom2*] *-* [*Ins2*]]*,* umgedreht ist.

Selbstverständlich darf die Änderung der orientierung von Sequenzabschnitten, die Verwendung von alternativen Selektions-Markern, alternativen eukaryontischen Promotoren, alternativen AFP-Genen und Abstandhalter-Genen, im Vergleich zu denen in den bevorzugten Ausführungsformen, nicht zu Interferenzen mit dem 5'LTR- oder den Promotoren oder zur Einführung von tatsächlichen oder kryptischen Spleiß-Signalen führen.

Der Sequenzabschnitt [*Prom1*] ist vorzugsweise ein CMV-Promotor oder ein alternativer eukaryontischer Promotor (wie zum Beispiel lentivirale Promotoren) durch den mindestens eine ebenso hohe AFP-Expression in den verschiedensten Zelltypen und Geweben gewährleistet ist, wie bei Verwendung des CMV-Promotors.

Der Sequenzabschnitt [*Prom2*] ist vorzugsweise ein Promotor aus der Gruppe bestehend aus einem Östradiol responsiven (GRE) oder einem Tetrazyklin responsiven (TRE) Promotor, insbesondere dem Gal4ER-VP responsiven Promotor, oder einem alternativen, streng induzierbaren Promotor (wie z.B. Ecdyson responsive Promotoren), insbesondere für ein im Sequenzabschnitt [*Ins2*] enthaltenes, für I-*Sce*I Meganuklease (I-*Sce*I) kodierendes Gen, dem SV40-Promotor oder einem alternativen eükaryontischen Promotor (wie z.B. dem TK-Promotor) insbesondere für im Sequenzabschnitt [*Ins2*] enthaltene Resistenzgene, dem CMV-Promoter oder einem in verschiedenen Zell- und Gewebetypen ähnlich hoch wie der CMV-Promotor exprimierenden eukaryontischen Promotor (wie z.B. lentiviräle promotoren) für im Sequenzabschnitt [*Ins2*] enthaltene AFP-Gene.

Bei dem Vektor ist die Sequenz des Wildtyp-Gens (*AFP*), die für das Humanized Renilla Green Fluorescent Protein (hrGFP), die für das Enhanced Green Fluorescence Protein (EGFP), die für das Enhanced Blue Fluorescence Protein (EBFP), die für das Enhanced Cyan Fluorescence Protein (ECFP), die für das Enhanced Yellow Fluorescence Protein (EYFP) oder die für das Red Fluorescent Protein (RFP oder *Ds*Red) kodierende: Nukleinsäuresequenz oder eine für ein Enzym, dessen Aktivität wie die der alkalischen Phosphatase über eine Chemolumineszenz-Reaktion nachweisbar ist; kodierende Nukleinsäuresequenz, einschließlich der von den genannten Sequenzen abgeleiteten Varianten oder Mutanten.

Gemäß einer bevorzugten Ausfuhrungsform ist das autofluorezierende protein aus der Gruppe bestehend aus Humanized Renilla Green Fluorescent Protein (hrGFP), Enhanced Green Fluorescence Protein (EGFP), Enhanced Blue Fluorescence Protein (EBFP), Enhanced Cyan Fluorescence Protein (ECFP), Enhanced Yellow Fluorescence. Protein (EYFP) oder Red Fluorescent Protein (RFP oder DsRed) ausgewählt. Bei dem biolumineszierenden Protein handelt es sich beispielsweise um Luciferase, bei dem Chemolumineszenz-Substrate umsetzenden Enzym handelt es sich beispielsweise um alkalische Phosphatase (Roche Diagnostics, Applied Biosystems), Peroxidase (Roche Diagnostics, Applied Biospstems), β-Galaktosidase (Arakawa, H. et al. (1999) Journal of Bioluminescence and Chemiluminescence 13 : 349-354) oder Luciferase (Baldwin, T.O et al. (1999) in Bioluminescence and ) Chemiluminescence: Perspective for the 21st Century. (Roda, K.A., Pazzagli, M., Kricka, L.J., and Stanley, P.E., eds.) John Wiley & Sons, England pp. 376-379).

Vorzugsweise wird ein Vektor zur Verfügung gestellt, bei dem [*Marker1*] und *[Marker2]* jeweils mindestens eine Mutation - aufweisen, wobei die Positionen der Mutationen so gewählt sind, daß
a) der Sequenzbereich zwischen der Position im [*Marker2*], die einer Mutation im [*Marker1*] entspricht, und der ersten, in 3'-Richtung liegenden Mutation im [*Marker2*] oder
b) der Sequenzbereich zwischen der Position im [*Marker1*], die einer Mutation, im [*Marker2*] entspricht, und der ersten, in 3'-Richtung liegenden Mutation im *[Marker1]*
mindestens etwa 150 Basenpaare umfaßt.

In einer weiteren Ausführungsform wird ein Vektor verwendet, bei dem der Sequenzabschnitt [*Marker1*] die Erkennungssequenz für die Meganuklease, I-*Sce*I aus *Saccharomyces* cerevisiae enthält oder derselben benachbart ist. Dieser Vektor ist dadurch gekennzeichnet, daß innerhalb dieser Erkennungssequenz ein Doppelstrangbruch in Gegenwart von I-*Sce*I entsteht und dadurch Doppelstrangbruch-Reparatur auslöst, also nicht nur homologe Rekombination sondern auch nicht-homologes *End Joining* alleine oder in Verbindung mit homologer Rekombination. Auch durch *End Joining* kann das Wildtyp-Gen rekonstituiert werden.

Die Erfindung betrifft ferner Verfahren under Verwendung von von dem erfindungsgemäßen Vektor der allgemeinen Struktur
5' - [*Ins1*]*-* [*Prom1*] - [*Marker1*]*-* [*Prom2*]*-* [*Ins2*] - [*Marker2*]*-*3'
abgeleitete Vektoren, wie zum Beispiel einen Vektor; bei dem der Sequenzabschnitt [*Marker*2] deletiert ist,

Die Erfindung betrifft ferner Verfahren under Verwendung eines von den oben genannten ) . Vektoren abgeleiteten Vektors, bei dem der Sequenzabschnitt [*Marker2*] deletiert ist, wobei [*Marker1*] innerhalb des Sequenzabschnittes zusätzlich eine Erkennungssequenz für die Meganuklease I-SceI aus *Saccharomyces cerevisiae* enthält oder derselben benachbart ist. Dieser Vektor ist dadurch gekennzeichnet, daß innerhalb dieser Erkennungssequenz ein Doppelstrangbruch in Gegenwart von I-*Sce*I entsteht und Doppelstrangbruch-Reparatur auslöst, also homologe Rekombination und *End Joining*. Durch *End Joining* kann das Wildtyp-Gen auch in. Abwesenheit von [*Marker2*] rekonstituiert werden.

Der Vektor kann in eukaryontische Zellen, insbesondere Säugerzellen über Transfektion oder über retrovirale Transduktion eingeführt werden, wobei dei Zellen keine menöchlichen Keinzellen sind.

- Gemäß einer Ausführungsform handelt es sich bei den Zellen um K562-Leukämiezellen, in welchen der Transkriptionsfaktor Gal4ER-VP konstitutiv exprimiert wird, wobei Gal4ER-VP eine durch β-Östradiol streng induzierbare Expression von Genen via Gal4ER-VP responsive Promotoren erlaubt, so daß z.B. I-*Sce*I-Meganuklease-Aktivität gezielt angeschaltet werden kann D.h., daß als [*Prom2*] der Gal4ER-VP responsive Promotor und als [*Ins2*] eine für I-SceI. kodierende Nukleinsäuresequenz verwendet wird.

Im Rahmen der vorliegenden Erfindung kann erstmals ein transgener, nicht-menschlicher Säuger, vorzugsweise ein Nager, bereitgestellt werden, dessen Keim- und somatische Zellen eine Sequenz
5'-[*Ins1*]-[*Prom1*]-[*Marker1*]-[*Prom2*]-[*Ins2*]-[*Marker2*]-3'
enthalten, wobei die Sequenzabschnitte [*Ins1*], [*Prom1*], [*Marker1*], [*Prom2*], [*Ins2*] und [*Marker2*] die oben angegebene Bedeutung haben. Ferner sind Säuger beschrieben, die bezüglich des strukturellen Aufbaus der Sequenz 5'-[*Ins1*]-[*Prom1*]-[*Marker1*]-[*Prom2*]-[*Ins2*]-[*Marker2*]-3' die oben im Zusammenhang mit den erfindungsgemäßen Vektoren erläuterten Variationen und Abwandlungen bzw. Modifikationen aufweisen.

Mit der vorliegenden Erfindung wird somit ein Verfahren zur Bestimmung von Gentoxizitäten zur Verfügung gestellt, bei dem man die genannten (eukaryontischen)- Zellen oder die erwähnten transgenen nicht-menschlichen. Säuger mit einer Testsubstanz in Kontakt bringt, wobei man anschließend mittels FACS-Analyse, Fluoreszenz-Messung, Fluoreszenz-Mikroskopie, Laser Scanning Cytometrie oder mittels einer Lumineszenz-Nachweis-Readction ein Auftauchen, eine Zunahme oder Abnahme von fluoreszierenden oder lumineszierenden Zellen entsprechend der Aktivität des Wildtyp-Genproduktes von [*Marker1*], [*Marker2*] , [*Ins1*] oder [*Ins2*] in den genannten Zellen oder in den Zellen des genannten Säugers auf die gentoxische Wirkung der Testsubstanz hinweist.

Der Verlust einer Fluoreszenz- oder Lumineszenz-Aktivität in einzelnen Zellen der Population weist auf nicht-konservative DNA-Austausch-Ereignisse, Replikationssprünge oder inaktivierende Mutationen hin, wenn es sich bei *[Ins2]* des verwendeten Vektors um die kodierende Sequenz eines AFPs oder LEs handelt und, wenn es sich bei [*Marker1*] oder *[Marker2]* um homologe Sequenzen von mindestens etwa 200 Bp handelt. Soweit [*Ins1*] und *[Ins2]* durch Mutation inaktivierte AFP- oder LE-Gene darstellen, werden revertierende Mutationen in *[Ins2]* bzw. *[Ins1]* durch das Auftauchen eines Fluoreszenz- oder Chemilumineszenz-Signals des Produktes des jeweiligen Wildtyp-Gens nachweisbar. Im Falle von einem weiteren Fluoreszenzsignal durch die Genprodukte der Wildtyp-Gene von *[Ins1]* oder *[Ins2]* unterscheiden sich diese von denen eventueller Genprodukte der Wildtyp-Gene von [*Marker1*] oder *[Marker2],* da Homologien zu Marker-Genen und zwischen Ins-Genen und ähnliche Fluoreszenzspektren der AFP/LEs nicht erlaubt sind.

Schließlich bietet die vorliegende Erfindung noch weitergehende Anwendungsmöglichkeiten. So ist beispielsweise die Verwendung des Vektors zur Feststellung von Krebs-Suszeptibilitäten über die Analyse von Patientenblut, Haut- oder Biopsiematerial eingeschlossen. Hierbei sollen die vom Probanden gewonnenen Zellen nach Standardmethoden kurzzeitig in Kultur genommen werden, um den erfindungsgemäßen Vektor, insbesondere den Vektor, bei dem [*Ins2*] ein Sequenzabschnitt ist, der eine für ein autofluoreszierendes Protein oder eine für ein biolumineszierendes oder ein Chemolumineszenz-Substrate umsetzendes Enzym oder für eine Mutante derselben kodierende Nukleinsäuresequenz enthält, mittels physikalischer oder chemischer Transfektionsmethoden oder über retrovirale Infektion in diese einzubringen, und um anschließend mittels FACS-Analyse, Fluoreszenz-Mikroskopie, Laser Scanning Cytometrie, Fluoreszenz-Messung oder Lumineszenz-Nachweis-Reaktion die transfizierten Zellen zu analysieren, wobei Zellen mit Fluoreszenz oder Lumineszenz entsprechend der Aktivität des Wildtyp-Genproduktes von [*Marker1*] und *[Marker2]* DNA-Austausch-Ereignisse anzeigen. Der Verlust einer weiteren Fluoreszenz-oder Lumineszenz-Aktivität in einzelnen Zellen der Population weist darüberhinaus auf nicht-konservative DNA-Austausch-Ereignisse, Replikationssprünge oder Mutationen hin, wenn es sich bei *[Ins2]* des verwendeten Vektors um die kodierende Sequenz eines AFPs oder LEs handelt. Wegen der Erhöhung von bestimmten Karyotyp-Abnormalitäten in den Lymphozyten von Brustkrebspatienten und den prädisponierten Verwandten ersten Grades sind gesteigerte DNA-Austausch-Häufigkeiten zumindest in den Zellen dieser Individuen, möglicherweise aber auch bei Patienten mit anderen Krebsarten und deren Verwandten ersten Grades zu erwarten (Patel, R.K. et al. (1997) Int. J. Cancer 72: 1-5; Trivedi, A.H., et al. (1998) Breast Cancer Research and Treatment 48: 187-190). Insgesamt können demnach, je nach Vektoraufbau, erhöhte DNA-Austausch-Aktivitäten und damit indirekt Krebssuszeptibilitäten durch das vermehrte Auftreten von fluoreszierenden/lumineszierenden Zellen (bei konservativer homologer Rekombination zwischen [*Marker1*] und [*Marker2*]*)* und/oder durch das vermehrte Verschwinden von Zellen mit einer anderen Fluoreszenz/Lumineszenz (bei nicht-konservativer homologer Rekombination oder Replikationssprüngen zwischen [*Marker1*] und [*Marker2*] und damit Verlust von [*Ins2*] angezeigt werden.

Die Erfindung betrifft somit ein Verfahren zur Analyse von Gentoxizitäten auf der Basis des Nachweises von konservativen homologen Rekombinations-Prozessen, nicht konservativen homologen Rekombinations-Prozessen, von inaktivierenden und revertierenden Mutationen getrennt oder im gleichen Reaktionsansatz, bei dem man die genannten Zellen, die mit einem erfindungsgemäßen Vektor transfiziert sind, oder die genannten Säuger mit einer Testsubstanz in Verbindung bringt, mit einem I-SceI-Expressionsplasmid transfiziert, I-*Sce*I durch Induktion (z.B. mittels Östradiol- oder Tetrazyklin-Gabe) exprimiert oder unbehandelt läßt. Anschließend können mittels FACS-Analyse, Fluoreszenz-Messung, Fluoreszenz-Mikroskopie, Laser Scanning Cytometrie oder mittels einer Lumineszenz-Nachweis-Reaktion DNA-Austausch-Häufigkeiten festgestellt werden, wobei fluoreszierende oder lumineszierende Zellen entsprechend der Aktivität des Wildtyp-Genproduktes von [*Marker1*] und *[Marker2]* diese Ereignisse anzeigen. Der Verlust einer Fluoreszenz oder Lumineszenz in einzelnen Zellen der Population weist auf nicht-konservative homologe DNA-Austausch-Ereignisse, Replikationssprünge oder inaktivierende Mutationen hin, wenn es sich bei *[Ins2]* des verwendeten Vektors um die kodierende Sequenz des Wildtyp-AFPs oder LEs handelt, oder auf inaktivierende Mutationen, wenn es sich bei [*Ins1*] um ein AFP/LE-Gen handelt. Soweit *[Ins1]* und *[Ins2]* durch Mutation inaktivierte AFP- oder LE-Gene darstellen, werden revertierende Mutationen in *[Ins2]* bzw. in *[Ins1]* durch das Auftauchen eines Fluoreszenz- oder Chemilumineszenz-Signals des Produktes des jeweiligen Wildtyp-Gens nachweisbar.

Desweiteren lassen sich die oben genannten Vektoren auch zur Charakterisierung der Bedeutung eines Gens in Bezug auf seine möglichen Funktionen bei der Aufrechterhaltung oder der Reduktion der genetischen Stabilität generell, bei der DNA-Rekombination oder bei der Entstehung von Mutationen im besonderen, verwenden, wobei auf Zellen oder nicht menschlichen Säuger mit unterschiedlichem Status (Status bedeutet in diesem Zusammenhang Änderungen im Gen, welche Auswirkungen auf den Phänotyp haben, d.h. im einfachsten Fall eine Mutation, die das Genprodukt inaktiviert) in Bezug auf das interessante Gen (durch Mutation, Deletion, Überexpression etc.) das oben genannte Verfahren zur Bestimmung von Gentoxizitäten, sowohl unter Verwendung als auch in Abwesenheit einer Testsubstanz, angewandt wird. Genetische Rearrangements werden schließlich durch auftretende Fluoreszenz nachgewiesen. In Bezug auf die Rolle einzelner Gene führt beispielweise der Aktivitätsausfall des Rekombinations-Regulators p53 zur Senkung spontaner oder durch gentoxische Behandlung (beispielsweise die Erzeugung eines Doppelstrangbruches mittels *I-Sce*I*)* induzierter, homologer Rekombinations-Ereignisse (Xia, F. et al. (1994) Mol. Cell. Biol. 14: 5850-5857; Bertrand, P. et al. (1997) Oncogene 14: 1117-1122; Mekeel, K.L. et al. (1997) Oncogene 14: 1847-1857; Dudenhöffer, C. (1998) Mol. Cell. Biol. 19: 2155-2168; 18: 5773-5784; Dudenhöffer, C. et al. (1999) Oncogene 18: 5773-5784; Saintigny, Y.et al. (1999) Oncogene 18: 3553-3565; Willers, H. et al. (2000) Oncogene 19: 632-639). Beim Vergleich von isogenen Zellen mit oder ohne p53 mit dem in dieser Erfindung vorgestellten Verfahren basierend auf dem AFP-Funktions-Nachweis, beobachtet man entsprechend der Rekombinations-Unterdrückung durch p53 eine geringere Anzahl fluoreszierender Zellen unter der Gesamtzellpopulation mit p53. Allgemein kann durch die Ausnutzung existierender Zellen oder tierischer Organismen mit unterschiedlichem Status in Bezug auf ein zu untersuchendes Gen die Rolle des Genproduktes in Bezug auf genomische Stabilität, Gentoxizität oder Krebssuszeptibilität festgestellt werden, indem in diese Zellen oder in diese Tiere ein Vektor zur Bestimmung von Gentoxizitäten mittels physikalischer oder chemischer Transfektionsmethoden oder über retrovirale Infektion eingebracht wird, und indem die Zellen anschließend mittels FACS-Analyse; Fluoreszenz-Mikroskopie, Laser Scanning Cytometrie, Fluoreszenz-Messung oder Chemilumineszenz-Nachweis-Reaktion analysiert werden, wobei Zellen mit Fluoreszenz oder Chemilumineszenz entsprechend der Aktivität des Wildtyp-Genproduktes von [*Marker1*] und *[Marker2]* DNA-Austausch-Ereignisse anzeigen. Der Verlust einer Fluoreszenz- oder Chemilumineszenz-Aktivität in einzelnen Zellen der Population weist darüberhinaus auf DNA-Austausch-Ereignisse oder inaktivierende Mutationen hin, wenn es sich bei [*Ins1*] oder [*Ins2*] des verwendeten Vektors um die kodierende Sequenz eines alternativen AFPs oder LEs handelt. Soweit [*Ins1*] und [*Ins2*] durch Mutation inaktivierte AFP- oder LE-Gene darstellen, werden revertierende Mutationen in *[Ins2]* oder [*Ins1*] durch das Auftauchen eines Fluoreszenz- oder Lumineszenz-Signals des Produktes des jeweiligen Wildtyp-Gens nachweisbar. Der Status

Ferner wird ein Verfahren zur Bestimmung, der genetischen Stabilität bzw. Instabilität eines Zelltyps oder den Rekombinations- oder Mutations-Häufigkeiten, eines Zelltyps, Gewebetyps oder eines eukaryontischen Organismus bereitgestellt; bzw ein Verfahren zur Charakterisierung der genetischen Stabilität bzw. Instabilität von oder den Rekombinations- oder Mutations-Haufigkeiten in Zellen, Geweben oder Säugern in Abhängigkeit vom physiologischen Zustand derselben (z.B. Zellzyklusstadium). Bei diesem Verfahren wird das oben genannte Verfahren zur Bestimmung von Gentoxizitäten angewandt, bei dem man Zellen des fraglichen Zelltyps, Gewebetyps oder allgemein eines eukaryontischen Organismus einsetzt, die man mit den genannten Vektoren transfiziert oder retroviral transduziert. Anschließend können mittels FACS-Analyse, Fluoreszenz-Messung, Fluoreszenz-Mikroskopie, Laser Scanning Cytometrie oder mittels einer Lumineszenz-Nachweis-Reaktion DNA-Austausch-Häufigkeiten festgestellt werden, wobei fluoreszierende oder chemilumineszierende Zellen entsprechend der AFP/LE-Aktivität des Wildtyp-Genproduktes von [*Marker1*] und [*Marker2*] diese Ereignisse anzeigen. Der Verlust einer Fluoreszenz oder Lumineszenz in Zellen der Population weist auf DNA-Austausch-Ereignisse, Replikationssprünge oder inaktivierende Mutationen hin, wenn es sich bei *[Ins1]* oder *[Ins2]* des verwendeten Vektors um die kodierende Sequenz eines AFPs oder LEs handelt. Soweit [*Ins1*] und [*Ins2*] durch Mutation inaktivierte AFP- oder LE-Gene darstellen, sind revertierende Mutationen in [*Ins2*] bzw. in [*Ins1*] durch das Auftauchen eines Fluoreszenz- oder Lumineszenz-Signals des Produktes des jeweiligen Wildtyp-Gens nachweisbar. Zeigt ein Zelltyp, Gewebetyp oder der Organismus von Interesse gegenüber den Durchschnittswerten der Kontrollen (Vergleich mit Kontrollzellen, -geweben oder -organismen, die diese Vektoren nicht enthalten) signifikant erhöhte DNA-Austausch- oder Mutations-Raten, so ist dieser als genetisch instabil einzustufen. Für die Gewebs- und Organismus-Analyse besteht die Möglichkeit der Gewinnung von Zellen aus dem lebenden Tier, z.B. aus dem Blut, oder die Möglichkeit der Analyse von bestimmten Geweben am toten Tier, z. B. mittels Laser Scanning Cytometrie.

Schließlich betrifft die Erfindung auch ein Verfahren zur Feststellung der Prädisposition eines eukaryontischen Individuums für das Auftreten bzw. die Progression von Krebs, der wie Brustkrebs mit genetischen Instabilitäten assoziiert ist (Parshad, R. et al. (1996) Brit. J. Cancer 74: 1-5). Bei diesem Verfahren führt man das oben genannte Verfahren zur Bestimmung von Gentoxizitäten durch, bei dem man Zellen des Individuums (z.B. aus Blut, Biopsie) einsetzt, die man mit den genannten Vektoren transfiziert oder retroviral transduziert. Die vom Probanden gewonnenen Zellen werden nach der Transfektion mittels FACS-Analyse, Fluoreszenz-Mikroskopie, Laser Scanning Cytometrie, Fluoreszenz-Messung oder Lumineszenz-Nachweis-Reaktion analysiert, wobei Zellen mit Fluoreszenz oder Lumineszenz entsprechend der Aktivität des Wildtyp-Genproduktes von [*Marker1*] und [*Marker2*] DNA-Austausch-Ereignisse anzeigen. Der Verlust einer Fluoreszenz oder Lumineszenz in Zellen der Population weist auf nicht-konservative DNA-Austausch-Ereignisse, Replikationssprünge -oder inaktivierende Mutationen hin, wenn es sich bei [*Ins1*] oder [*Ins2*] des verwendeten Vektors um die kodierende Sequenz eines AFPs: oder LEs handelt. Soweit [*Ins1*] und [*Ins2*] durch Mutation inaktivierte AFP- oder LE-Gene darstellen, werden revertierende Mutationen in *[Ins2]* bzw. in [*Ins1*] durch das Auftauchen eines Fluoreszenz- oder Lumineszenz-Signals des Produktes des jeweiligen Wildtyp-Gens nachweisbar. Erhöhte DNA-Austausch- und Mutations-Raten gegenüber den Durchschnittswerten von den Zellen von. Kontroll-Personen sind indikativ für eine Prädisposition für das Auftreten von, den Ausbruch bzw. die Progression von Brustkrebs und möglicherweise auch von anderen Krebsformen.

Die vorliegende Erfindung betrifft ein neuartiges Testsystem zur Bestimmung von Gentoxizitäten auf der Basis von Rekombinations-*Markern* auf transfizierbaren Plasmiden oder mit Lokalisation auf den Chromosomen von Säugerzellen. In diesem Testsystem wird DNA-Schädigung durch das gehäufte Auftreten von genetischen Veränderungen direkt in den geschädigten Säugerzellen detektiert. Die besondere Auswahl und Anordnung ) der einzelnen Sequenz-Elemente ermöglicht hier die Erfassung aller derzeit bekannten DNA-Rekombinations-Typen und zusätzlich inaktivierender Punktmutationen. Damit wird erfindungsgemäß: ein Großteil aller Mutationen erfaßbar gemacht und damit wiederum die Sensitivität des Nachweises von Gentoxizitäten maximiert. Als Signal dient die Rekonstitution eines Genes kodierend für ein intensiv fluoreszierendes Protein (Clontech, Palo Alto, CA, USA), z.B. das grün leuchtende EGFP, sowie zusätzlich oder alternativ der Erhalt oder Verlust eines weiteren Genes kodierend für ein andersfarbig fluoreszierendes Protein, z.B. das rot leuchtende DsRed. Die quantitative Analyse kann über Messung der jeweiligen Fluoreszenzen direkt in den geschädigten Zellen am Fluoreszenz-Mikroskop, am FACS-Scan Durchflußzytometer oder am Laser scanning Cytometer erfolgen. Dieses auf der Rekonstitution bzw. dem Verlust der Autofluoreszenzen von Proteinen basierende Nachweisverfahren ist weiterhin durch die Auswahl und Anordnung der Sequenz-Elemente so aufgebaut, daß es in verschiedene Säugerzellen und auf lebende Tiere übertragen werden kann, um DNA-Rearrangements *in situ* verfolgen zu können. Die zu analysierenden DNA-Rearrangements betreffen transgene Sequenzen, welche mittels retroviraler Transduktion intakt in eines der zellulären Chromosomen integriert werden können. Durch dieses retrovirale Vektordesign können erfindungsgemäß die *Marker*-Gene linear und als Einzelkopien inseriert werden und der individuelle Chromatinkontext bei Messungen an Zellen oder am Tiermodell mitberücksicht werden. Auch in transienten *Assays*, d.h. nach vorübergehender Einführung des Rekombinations-Konstruktes durch Transfektion, können DNA-Rearrangements mit diesem Testsystem als Antwort auf gentoxische Behandlung abgeschätzt werden. Deswegen müssen nicht für alle Messungen stabile Einzelzell-Klone etabliert werden, d.h es können auch primäre Zellen z.B. aus Biopsiematerialien analysiert werden. Der Einbau einer. Erkennungssequenz für die Meganukiease I-*Sce*-I aus *Saccharomyces cerevisiae* in eines der Marker-Gene eröffnet darüberhinaus die Möglichkeit, durch Einführung der Meganuklease in geeignete Testzellen und der damit verbundenen Einführung eines Doppelstrangbruches (DSBes) gezielt in eines der *Marker*-Gene die Basalrate der DNA-Rearrangements zu erhöhen (Rouet, P.. et al. (1994) Proc. Natl. Acad. Sci: USA 91: 6064-6068) . Erfindungsgemäß können so mögliche synergistische Wirkungen zwischen der Erzeugung von DSBen bzw. deren Reparatur und den zu analysierenden Noxen auf die Häufigkeit von Mutationen erfaßt werden.

Das Testsystem ist erfindungsgemäß den oben aufgeführten Systemen in allen beschriebenen Problempunkten überlegen. Es wurde zur Detektion von Gentoxizitäten in Säugerzellen etabliert und ist auf lebende. Tiere übertragbar. D.h. die Metabolisierung von Prokarzinogenen im Testsystem entspricht, der des gewünschten Säugersystems. Darüberhinaus wird für den Nachweis kein zweiter Organismus benötigt. Für den Gentoxizitäts-Nachweis sind weder mehrtägige . Zellzucht unter Ausübung eines Selektionsdrucks, noch Zelllyse oder Zellextraktion., noch die Applikation einer weiteren. Substanz außer der zu testenden, potentiell gentoxischen nötig. Mutagenizität, hier angezeigt über das Fluoreszenzsignal, kann innerhalb von Stunden nach gentoxischer Behandlung gemessen werden. Im Gegensatz zu den zytogenetischen Verfahren (SCE-Nachweis, Mikronukleus *Assay),* dem *Comet Assa*y und dem Nachweis erhöhter DNA-Reparatur-Synthese-Aktivitäten ist der Nachweis von fluoreszierenden Zellen, in einer Population von nicht fluoreszierenden Zellen sehr einfach und geeignet für die Analyse im Hochdurchsatz. Das Design des Testsystems gibt keine zwingenden Einschränkungen des Gentoxizitäts-Nachweises auf bestimmte Entwicklungsstadien oder Gewebetypen beim Tiermodell vor.

In einer kürzlich von Jasin und Kollegen veröffentlichten Arbeit (Pierce, A.J. et al. (1999) Genes & Development 13: 2633-2638) wurde die Analyse von Genkonversions-Häufigkeiten in Abhängigkeit des Reparatur-Genes *Xrcc3* gemessen. Das in dieser Arbeit verwendete Genkonversions-Testsystem basierte auf der Rekonstitution eines Wildtyp-*EGFP*-Gens aus zwei mutierten Formen des EGFP-Gens. Dieses Prinzip ist auch ein Element der hier beschriebenen Erfindung. Im Gegensatz zum Rekombinations-Testsystem von Jasin und Kollegen können jedoch mit dem erfindungsgemäßen Testsystem außer der Genkonversion und dem *Double Crossover,* also konservativen, nicht deletierenden Rekombinations-Ereignissen (siehe unten) auch nicht konservatives *Single Crossover, Single Strand Annealing* (SSA) und Replikationssprünge sowie Punktmutationen erfaßt werden. In einer weiteren Ausführform des Testsystems kann darüberhinaus *End Joining* nachgewiesen werden, welches DNA-Enden über Homologien von nur wenigen Basenpaaren verknüpft. Dies war durch eine Kombination von Genen kodierend für zwei unterschiedlich autofluoreszierende Proteine (AFPs), durch das Design der Mutationen und durch die spezielle Anordnung der einzelnen DNA-Elemente im Gesamtkonstrukt möglich geworden. Ein wesentlicher Fortschritt wird im erfindungsgemäßen System insbesondere dadurch erzielt, daß darauf verzichtet wird, die AFPs als Fusionsproteine zu exprimieren. Dadurch konnten die damit verbundenen Verluste der Leuchtintensitäten der AFPs vermieden werden. Gleichzeitig konnten die mutierten Genabschnitte, welche nach erfolgreichem homologen DNA-Austausch für ein AFP, z.B. EGFP kodieren, in ihren Homologien maximiert werden, indem nur 4 Basenpaare kodierend für die Aminosäuren im Chromophorbereich bzw. nur das Startkodon in ein Stopkodon mutiert wurden. Da hier also keine trunkierten Gene als Mutanten-Gene verwendet wurden, mußten also auch die homologen Bereiche nicht über Fremdsequenzen, kodierend für eine Fusion mit EGFP, verlängert werden, um für den Nachweis ausreichende Austausch-Raten zu gewährleisten. In Kombination mit der Maximierung der Transkription der AFP-Gene durch den extrem starken CMV-Promotor ist die Sensitivität des erfindungsgemäßen Systems durch die aufgelisteten Maßnahmen so hoch, daß die Nachweisgrenze unter der Rate für spontane DNA-Austausch-Raten liegt. Dies bewirkt, daß nicht nur die 100-10000 fach häufigeren genetischen Austausch-Prozesse nach Initiation durch einen gezielten Schnitt in einem der beiden Gen-Varianten durch die Meganuklease *I-SceI* gemessen werden können (Liang, F. et a1. (1996) Proc. Natl. Acad. Sci. USA 93: 8929-8933). Im Gegensatz zum Genkonversions-Testsystem von Jasin und Kollegen können dank der vorliegenden Erfindung durch den Nachweis verschiedenfarbig leuchtender Zellen am FACS-Scan Durchflußzytometer oder am Fluoreszenzmikroskop die Ergebnisse verschiedener Rekombinations-Typen und potentiell auch Punktmutationen nachgewiesen werden. Eine weitere vor kurzem veröffentlichte Arbeit (Slebos, R.J.C. und Taylor J.A. (2001) Biochem. Biophys. Res. Commun. 281: 2121-2129) beschreibt ein Rekombinations-Testsystem, welches die Rekonstitution eines GFP- mit Hilfe eines EBFP-Gens ermöglicht. Der Vorteil des erfindungsgemäßen Gentoxizitäts-Bestimmungs-Systems gegenüber diesem Rekombinations-Testsystem ist auf technischer Ebene der vollständige Verzicht auf die leuchtschwächeren Fusionsproteine, die Ausnutzung der 35 fach stärkeren Leuchtintensität von EGFP im Vergleich zum GFP (Clontech,, Palo Alto, CA, USA) und die Maximierung der Homologien auf 533 Bp. Das erfindungsgemäße System bietet sogar eine beliebige Erweiterung der homologen Bereiche und damit eine Steigerung der DNA-Austausch-Ereignisse infolge gentoxischer Behandlung, da beliebig lange homologe Sequenzen innerhalb der Abschnitte *[Marker1]* und *[Marker2]* inseriert werden können, wenn diese eine kodierende Sequenz für ein autofluoreszierendes Protein oder ein biolumineszierendes oder Chemolumineszenz-Substrate umsetzendes Enzym in Abschnitt *[Ins2]* flankieren. Außerdem bezieht das erfindungsgemäße Testsystem jede Art von Gen-inaktivierenden DNA-Austausch- und Mutations-Ereignissen mit ein, indem eine kodierende Sequenz für ein autofluoreszierendes Protein oder ein biolumineszierendes oder Chemolumineszenz-Substrate umsetzendes Enzym in Abschnitt *[Ins2]* eingeführt wurde. Außerdem bietet das erfindungsgemäße Gentoxizitäts-Nachweis-System die Möglichkeit zur Einführung eines gezielten DSBes mittels I-*Sce*I-Meganuklease und damit auch zur Analyse von gentoxischer Wirkung in Verbindung mit oder im Vergleich zu DSB-Reparatur-Ereignissen. Weiterhin wurden DNA-Substrate des erfindungsgemäßen Systems stabil in die Chromosomen von Säugerzellen eingeführt, wodurch es zum sensitiven Nachweis auch von seltenen genetischen Veränderungen eingesetzt werden kann. Dies bedeutet, daß sich das erfindungsgemäße Verfahren für den empfindlichen und routinemäßigen Nachweis von Reparatur-Antworten auf unterschiedliche gentoxische Behandlungen eignet. Diese Eigenschaft wurde experimentell belegt, wohingegen Pierce und Kollegen wie auch Slebos und Kollegen ausschließlich Rekombinations-Messungen durchführten und diese wiederum ausschließlich für die um Größenordnungen frequenteren Ereignisse nach Einführung eines DSBes mittels I-*Sce*I-Meganuklease bzw. nach Einführung von 10⁵ bis 10⁶ DNA-Kopien durch transiente Transfektion analysierten. Eine deutliche Verbesserung im Hinblick auf die kommerzielle, und damit automatisierte Nutzung eines entsprechenden Gentoxizitäts-Bestimmungs-Systems stellt bei dem erfindungsgemäßen System auch die Auswahl von Säugerzellen dar, welche in Suspensionskultur wachsen und welche darüberhinaus resistent gegenüber dem aktiven Zelltod sind, aber trotzdem p53-negativ sind und damit eine um mindestens eine Größenordnung höhere Rekombinationsrate aufweisen (Mahdi, T., D. et al. (1998) Biol. Chem. 90: 15-27). Schließlich kann das Testsystem der Erfindung zur Bestimmung von Gentoxizitäten über Retroviren transduziert werden. Dies erlaubt nicht nur die effiziente Übertragung in neue Zielzellen, sondern auch die lineare und einmalige Insertion ins zelluläre Genom (Coffin, J.M. (1996) in Fundamental Virology, Fields, B.N., Knipe, D.M. und Howley, P.M. (eds.), Lippincott-Raven Publishers, Philadelphia, 763-844). Da Retroviren für den therapeutischen Gentransfer eingesetzt werden, ist darüberhinaus bedeutsam, daß mittels dieses Testsystems auch die Stabilitäten bzw. Instabilitäten an den Insertionsorten genomisch integrierter Proviren analysiert werden können (Positionseffekte).

### 3.1 Bedeutung des Nachweises der DNA-Rekombination an Zellulären Chromosomen

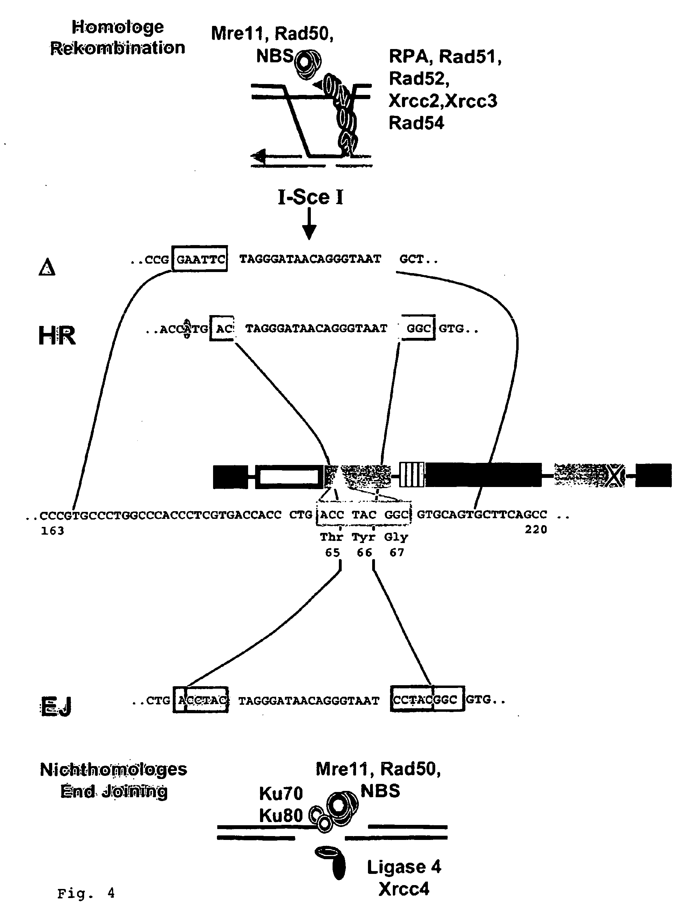

Rekombinations-Prozesse repräsentieren den letztmöglichen und unersetzlichen Reparatur-Mechanismus, wenn DNA-Quervernetzungen, DNA-Doppelstrangbrüche oder DNA-Lücken es nicht erlauben, die fehlende Information vom komplementären Strang zu übertragen. Darüberhinaus müssen Einzelstrangbrüche oder noch unreparierte Läsionen an der Replikationsgabel, also während der DNA-Synthese in proliferierenden Zellen, ebenfalls rekombinativ beseitigt werden (Haber, J. (1999) Trends Biochem. Sci. 24: 271-275; Cox, M.M. et al. (2000) Nature 404: 37-40). Aus diesem Grunde repräsentieren rekombinative Reparatur-Prozesse eine der wichtigsten und vor allem auf unterschiedlichste DNA-Schädigungen reagierende DNA-Reparatur-Form. Dies macht den Nachweis von Rekombinations-Prozessen als Indikator für DNA-Schädigungen nach Einwirkung unterschiedlicher gentoxischer Agentien oder Bestrahlung interessant. Die bei der Reparatur involvierten Rekombinations-Prozesse müssen nach ihrem Mechanismus in verschiedene Typen eingeteilt werden (Kanaar, R. et a1. (1998) Trends Cell Biol. 8: 483-489): Bei der homologen Rekombination werden identische oder sehr ähnliche Sequenzen kopiert, welche sich auf dem gleichen Chromatid (z.B. bei repetitiven Sequenzen), auf dem Schwesterchromatid (v.a. während der DNA-Synthese) oder auf dem homologen Chromosom (v.a. während der Meiose) befinden. Bei Eukaryonten sind ununterbrochene Homologien von 134 bis 232 Basenpaaren für den erfolgreichen Austausch nötig (Waldman, A.S. und Liskay, M. (1988) Mol. Cell. Biol. 8: 5350-5357). Konservative homologe Rekombinations-Prozesse bewirken eine vollständige Rekonstitution der geschädigten Sequenzen. Hierzu gehören das reziproke *Double Crossover* und die unilaterale Genkonversion. Bei nichtkonservativen homologen Rekombinations-Ereignissen (nkHR) gehen DNA-Abschnitte zwischen den homologen DNA-Austausch-Partnern verloren, d.h. sie sind die Ursache von Deletionen. Hierzu zählen das *Single Crossover,* das *Single* Strand Annealing und Replikationssprünge. Homologe Rekombination repräsentiert den etwa zur Hälfte genutzten DSB-Reparatur-Pfad in Säugern (Liang, F. et al. (1998) Proc. Natl. Acad. Sci. USA 95: 5172-5177). In proliferierenden Zellen dienen als Rekombinations-Substrate fast ausschließlich Schwesterchromatid-Sequenzen oder wiederholte Sequenzen auf dem ) gleichen Chromatid. Für die zu Grunde liegenden Strangaustausch-Prozesse werden vor allem die Proteine Rad51, Rad52, Rad54, Xrcc2 und Xrcc3 benötigt (siehe Abb. 4 und Referenz Lambert, S. et al. (1999) Mutat. Res. 433: 159-168). Doppelstrangbrüche (DSBe) werden darüberhinaus über *End Joining* (EJ), d.h. eine einfache Verknüpfung der DNA-Enden, also eine Ligation, oder eine Verknüpfung über sehr kurze Homologien von nur wenigen Basenpaaren wieder zusammengefügt. Hierbei spielen die Enzymkomplexe Mrell-Rad50-NBS, Ku70-Ku80 und Ligase4-Xrcc4 eine wichtige Rolle (Abb. 4). Die wichtigsten Beiträge zur DSB-Reparatur werden in Säugern von der Genkonversion, dem SSA und dem EJ geleistet (Critchlow, S.E. und Jackson, S.P. (1998) Trends Biochem. Sci. 23: 394-398; Lambert, S. et al. (1999) Mutat. Res. 433: 159-168; Johnson, R.D. und Jasin, M. (2000) EMBO J. 19: 3398-3407). Dazu kommt, daß EJ und Genkonversions-Ereignisse aneinander gekoppelt sein können, was möglicherweise mit der Beteiligung von Mrell-Rad50-NBS sowohl beim EJ als auch bei der Initiation der homologen Rekombination zu tun haben kann (siehe Abb. 4).

Aufgrund der besonderen Eignung von homologen rekombinativen Reparatur-Prozessen als Indikatoren für DNA-Schädigung durch Karzinogene (Schiestl, R.H. (1989) Nature 337: 285-288; Brennan, R.J. et al. (1994) Mutat. Res. 308: 159-167; Carls, N. und Schiestl, R.H. (1994) Mutat. Res. 320: 293-303; Aubrecht, J. et al. (1995) Carcinogenesis 16: 2841-2846; Schiestl, R.H. et al. (1997) Proc. Natl. Acad. Sci. USA 94: 4576-4581) repräsentierte die Entwicklung eines Rekombinations-Testsystems in Säugerzellen den Ausgangspunkt der hier beschriebenen Erfindung. Ziel war außerdem, durch entsprechendes Vektordesign zu ermöglichen, daß die Rekombinations*-Marker* des Testsystems linear und als einmalige Kopie ins zelluläre Genom mittels retroviraler Transduktion inseriert werden können, um eindeutige Aussagen über den Typ der Rekombination (z.B. EJ versus homologer Rekombination) treffen zu können (siehe Kapitel 3.5).

### 3.2 Gezielte Initiation von Rekombinations-Ereignissen

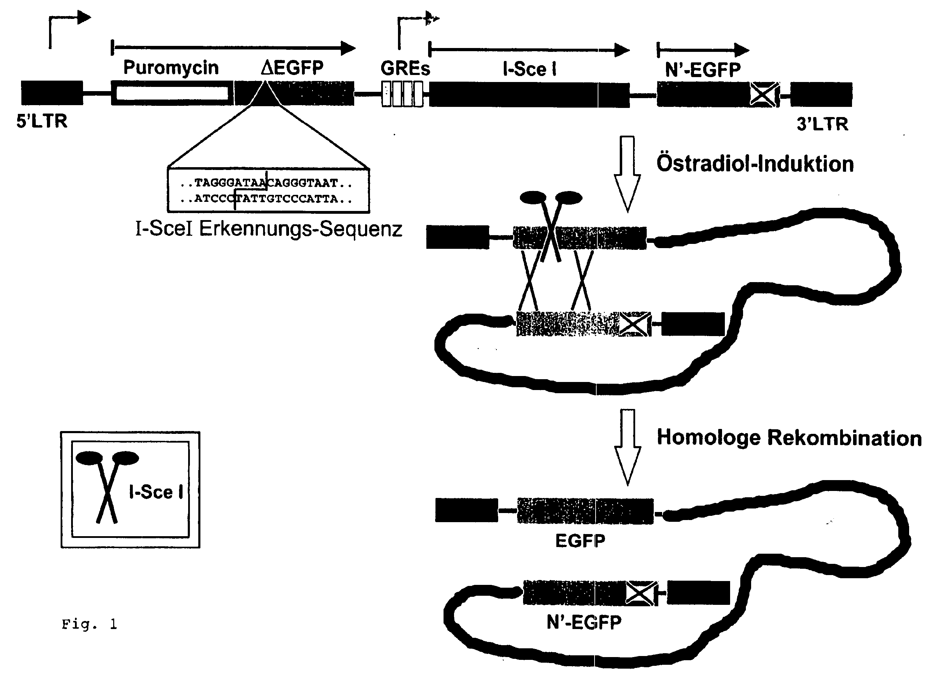

Ein weiterer wichtiger Aspekt des Assay-Designs war, daß Rekombinations-Ereignisse nicht nur durch Strahlung und gentoxische Substanzen sondern auch durch gezielte Einführung eines DSBes in eines der Marker-Gene initiierbar sein sollten. Dies ermöglicht einerseits die Etablierung des Tests, dient als interner Standard und erlaubt darüberhinaus mögliche Synergien zwischen dem gezielt eingeführten DSB und den Auswirkungen von gentoxischen Behandlungen auf DNA-Reparatur-Ereignisse festzustellen. Zur Einleitung von DNA-Austausch-Ereignisse durch gezielte DSBe wurde die I-SceI-Meganuklease gewählt (Liang, F. (1996) Proc. Natl. Acad. Sci. USA 93: 8929-8933). I-SceI ist eine Endonuklease aus *S. cerevisiae* mit einer 18 Basenpaar langen Erkennungssequenz. Gemäß statistischer Berechnung sollten keine natürlichen Erkennungssequenzen in Säugergenomen vorkommen. Die kodierende cDNA wurde von Dr. Maria Jasin, Sloan-Kettering Institute, New York, für die Expression in höheren Eukaryonten zugeschnitten und N-terminal mit dem Hämagglutinin-Peptid-Epitop (HA) für den monoklonalen Antikörper 12CA5 (Roche) verknüpft. Dr. M. Jasin demonstrierte, daß die Erzeugung von DSBen durch I-SceI in Hamsterzellen homologe Rekombinations-Ereignisse 10²-10⁴ fach stimuliert. Um lokal im gewünschten Rekombinations-Marker, im einfachsten Falle dem EGFP-Gen, DSBe erzeugen zu können, wurde erstens die I-SceI-Erkennungssequenz molekularbiologisch in eines der EGFP-Marker-Gene inseriert (siehe Kapitel 3.3) und zweitens die Meganuklease zur Expression gebracht (siehe Abb. 1).

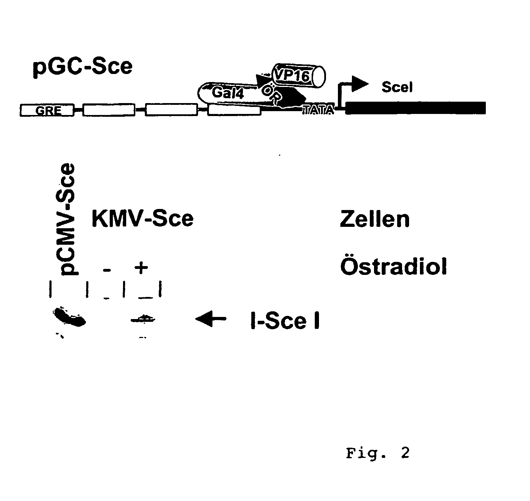

Um aufgrund der antiproliferativen Wirkung von DSBen Steuerbarkeit der Meganuklease-Expression zu erreichen, wurde gemäß einer bevorzugten Ausführungsform der Erfindung das äußerst zuverlässige, durch β-Östradiol induzierbare Expressions-System gewählt (Braselmann, S. et al. (1993) Proc. Natl. Acad. Sci. USA 90: 1657-1661; Dudenhöffer, C. (1999) Oncogene 18: 5773-5784). Das Expressions-System koppelt den sogenannten Gal4ER-Transkriptionsfaktor an eine durch einen Gal4ER-responsiven Promotor gesteuerte Expressions-Kassette (siehe Abb. 2). Gal4ER repräsentiert eine Fusion aus der DNA-Binde-Domäne des Gal4-Transkriptionsfaktors mit der starken, viralen VP16-Transaktivator-Domäne und einer ß-Östradiol-Binde-Domäne des humanen ß-Östradiol-Rezeptors, was Gal4ER als Transkriptionsfaktor funktionell durch ß-Östradiol induzierbar macht. Dieses Expressions-Induktions-Prinzip erlaubt induzierbare Steigerung der Protein-Expression von mindestens einer Größenordnung. Im Rahmen der vorliegenden Erfindung wurden für die Tests ausgehend von parentalen Methylcholanthren-induzierten Balb/c-Maus Fibrosarkom-Zellen (DeLeo, A.B. et al. (1977) J. Exp. Med. 146: 720-734), von humanen WTK1-Lymphoblasten (Xia, F. et al. (1994) Mol. Cell. Biol. 14: 5850-5857) und von humanen myelogenen K562-Leukämiezellen (Andersson, L.C. et al. (1979) Int. J. Cancer 23: 143-147) abgeleitete MMV-, WMV- und KMV-Linien etabliert, in welchen der Transkriptionsfaktor Gal4ER konstitutiv, also ständig zur Expression gebracht wurde. Dies erfolgte über Elektroporation der Zellen mit dem Expressions-Plasmid pMVGalERVP und anschließender Isolierung von Einzelzell-Klonen in Softagar mittels der von pMVGalERVP kodierten Neomycin-Antibiotika-Resistenz. Anschließend wurden von KMV abgeleitete Zellinien etabliert, welche I-SceI nach ß-Östradiol-Induktion exprimieren. Hierfür wurden KMV-Zellen mit dem Vektor pGC-Sce (Abb. 2) und einem Hygromycin-Resistenz vermittelnden Plasmid elektroporiert und Einzelzell-Klone unter Antibiotika-Selektionsdruck isoliert. Induzierbare *I-SceI-*Expression wurde in Western-Blot-Experimenten nachgewiesen. Bezüglich der Stabilität und Steuerbarkeit der Gal4ER-Expression konnten über einen Zeitraum von mehreren Monaten keine Veränderungen beobachtet werden. Wie in Abb. 2 demonstriert, konnte die Expression der I-SceI-Meganuklease auf diesem Wege, also durch einfache ß-Östradiol-Gabe (≥ 50 nM) ins Medium innerhalb von 4 h in Gal4ER exprimierenden Zellen an-und abgeschaltet werden. Deswegen wurden innerhalb der erfindungsgemäßen retroviralen Vektoren mit den AFP-Rekombinations-Markern (siehe Kapitel 3.3) die für die I-SceI-Meganuklease kodierenden Sequenzen den Erkennungssequenzen des Gal4-Transkriptionsfaktors (GREs) wie in pGC-Sce nachgeordnet. Von ß-Östradiol abhängige Expression konnte über ein leicht nachzuweisendes Marker-Protein, nämlich p53, auch im Kontext des Rekombinations-Vektors demonstriert werden. Insgesamt wurden also die Voraussetzungen geschaffen, um zu einem definierten Zeitpunkt, z.B. innerhalb des Zellzyklusses, Rekombination in Zellen durch die I-SceI-Meganuklease nach ß-Östradiol-Gabe zu initiieren. Alternativ dazu kann das I-SceI-Enzym durch Transfektion mit einem Expressions-Plasmid vorübergehend in Säugerzellen produziert werden (siehe Kapitel 5 Beispiel 4). Es konnte gezeigt werden, daß nach Transfektion 80 % der Moleküle mit einer I-SceI-Erkennungssequenz gespalten werden (Rouet, P. und Jasin, M. (1994) Mol. Cell. Biol. 14: 8096-8106) .

### 3.3 Messung der Rekombination durch Rekonstitution eines AFP-Gens

Grundsätzlich muß ein Testsystem für homologe Rekombination zwei homologe DNA-Sequenz-Abschnitte als Rekombinations-Marker besitzen. Die beiden Sequenzen repräsentieren in einer Ausführform mutierte Varianten eines Gens dergestalt, daß erst durch den homologen DNA-Austausch das Wildtyp-Gen, und damit das kodierte Wildtyp-Protein, rekonstituiert werden kann. Im sehr häufigen Fall der Genkonversion, also dem unilateralen Genaustausch, bezeichnet man den Sequenz-Abschnitt, welcher die Information zur Wiederherstellung liefert, als Donor und das zweite Gen als den Akzeptor. Im Falle der Einführung eines gezielten DSBes, z.B. über I-SceI, in eines der beiden Gene, wird dieses zum Akzeptor.

Auf der ersten Stufe wurde für den Nachweis von homologen Rekombinations-Ereignissen die stark grüne Autofluoreszenz des EGFP-Proteins verwendet (siehe Abb. 1). Das erfindungsgemäße Nachweissystem ist ebensogut für den Einsatz anderer ebenso intensiv leuchtender AFPs von Clontech, Palo Alto, CA, USA, wie EBFP (blau), ECFP (cyan), EYFP (gelb), DsRed (rot) oder den AFPs anderer Hersteller geeignet. EGFP absorbiert Licht maximal bei 488 nm und emittiert grünes Licht bei 507 nm. Die Fluoreszenz des Proteins ist äußerst stabil und eignet sich demnach vorzüglich als Reporter für den Nachweis einzelner fluoreszierender Zellen in einer Population nichtfluoreszierender Zellen über Fluoreszenz aktiviertes Zell-Sortieren (FACS) oder durch Fluoreszenz-Mikroskopie. Um Rekombinations-Frequenzen mittels Nachweis einzelner fluoreszierender Zellen am FACS-Scan Durchflußzytometer quantifizieren zu können, muß ausreichende Sensitivität sichergestellt sein. Hierzu wurden im Vorfeld Mischungs-Experimente mit nicht leuchtenden mKSA-Tumorzellen und mit murinen mKSA-Tumorzellen, welche nach stabiler Integration der *EGFP*-Expressions-Kassette des Clontech-Vektors pEGFP-N1 ins zelluläre Genom EGFP produzieren, durchgeführt. Die Auswertungen am FACS-Scan Durchflußzytometer Calibur von Becton Dickinson ergaben, daß für EGFP, exprimiert mittels CMV-Promotor, die Fluoreszenz-Intensität um mehr als zwei Größenordnungen über der schwach orangen endogenen Fluoreszenz der Zellen liegt. Weiterhin konnten mittels EGFP leuchtende Zell-Subpopulationen bis zu einer Verdünnung von mindestens 10⁻⁵ nachgewiesen werden.

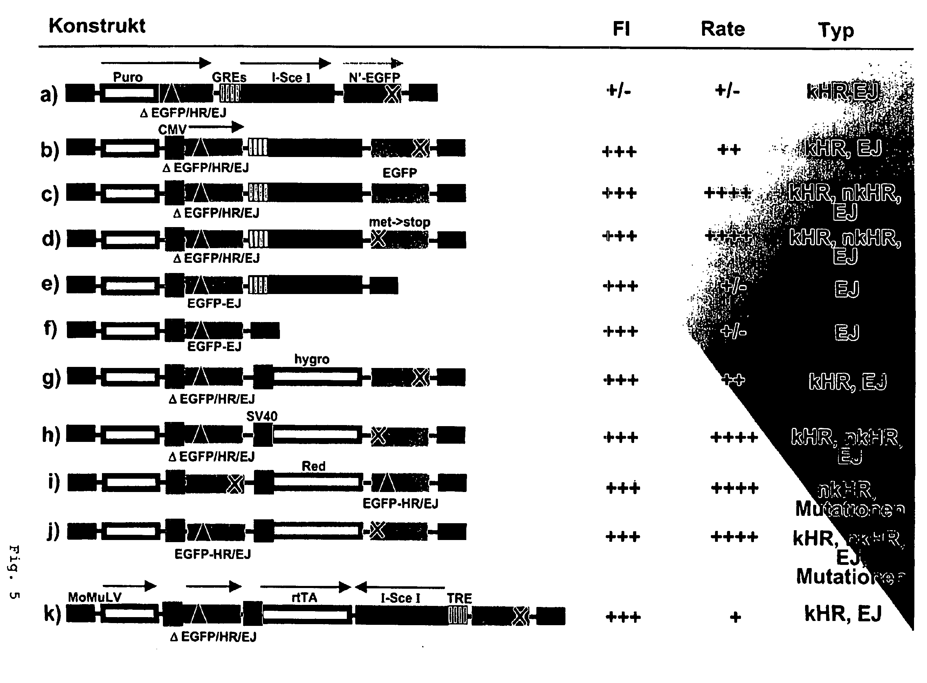

Für die Rekonstitution eines Wildtyp-*EGFP*-Gens wurden mutierte Varianten des EGFP-Gens als Rekombinations-Marker eingesetzt. Die erste Variante, *ΔEGFP* als Akzeptor-Substrat, wurde mittels PCR-Technologie aus einem 731 Basenpaar langen cDNA-Fragment mit der für EGFP kodierenden Region (beginnend 2 Basenpaare vor dem Startkodon und endend 9 Basenpaare hinter dem Stop-Kodon) erzeugt, indem die für die Chromophor-Aminosäuren 65-67 kodierenden Basenpaare, 29 weitere Basenpaare im 5'-Bereich und 7 Basenpaare im 3'-Bereich durch die Erkennungssequenz der Meganuklease I-*Sce*I und der Restriktions-Endonuklease *Eco*RI ersetzt wurden (siehe Abb. 4). Die zweite Variante, *N'-EGFP* als Donor-Substrat, repräsentiert ein am 3'-Ende innerhalb der für EGFP kodierenden Region um 278 Basenpaare verkürztes Fragment, so daß dem resultierenden Protein die Ummantelung des Chromophors zum Schutz vor Quenching-Effekten fehlt. *N'-EGFP* überdeckt somit den im *ΔEGFP* mutierten Bereich, kann also als Matrize für homologe DSB-Reparatur dienen. *ΔEGFP* wurde in den Leserahmen des Puromycin-Resistenz-Gens inseriert, so daß das ΔEGFP-Protein in Fusion mit der Puromycin-N-Acetyltransferase nach mRNA-Synthese ausgehend vom viralen 5'-LTR-Promotor (siehe Kapitel 3.5) translatiert wird. *N'-EGFP* wurde in Serie hinter *△EGFP* eingesetzt, so daß ausschließlich *Double Crossover* und Genkonversions-Ereignisse zu einem intakten Wildtyp-*EGFP*-Gen führen können. Zwischen den Rekombinations-Markern mußte ein Abstandhalter eingeführt werden, um die für den DNA-Austausch nötige Flexibilität der DNA zu gewährleisten. In der ersten Variante der Erfindung wurde hier die vollständige I-SceI-Expressions-Kassette inseriert (siehe Abb. 1 und Abb. 5a und Kapitel 3.2). Der Einbau des Puromycin-Resistenzgens PAC verhindert die Hemmung der Proteinbiosynthese in Gegenwart des Antibiotikums Puromycin. Dies erlaubt die Etablierung von Einzelzell-Klonen, welche das in Abb.1 und Abb. 5a dargestellte Konstrukt zum Nachweis von Rekombinations-Ereignissen stabil ins zelluläre Genom integriert tragen.

Einen weiteren entscheidenden Vorteil des auf EGFP basierenden erfindungsgemäßen Testsystems für DNA-Austausch-Prozesse stellt die kurze Reaktionszeit dar, da so gentoxische Wirkung unverfälscht durch lange Auswertungsphasen oder gar durch Selektions-Prozesse nachgewiesen werden kann. Eine Abschätzung des Zeitraums zwischen Abschluß des Rekombinations-Prozesses und der Detektion fluoreszierender Zellen wurde folgendermaßen vorgenommen: Gal4ER exprimierende KMV-Zellen wurden mit dem dafür hergestellten Vektor pGC-EGFP als *Reporter* elektroporiert, nach einer eintägigen Erholungsphase Östradiol zu den Zellen gegeben und anschließend zu verschiedenen Zeiten Autofluoreszenz-Entwicklung überprüft. Bereits nach 4 h waren fluoreszierende Zellen deutlich von nichtfluoreszierenden zu unterscheiden. Dies stellt einen deutlichen Vorsprung gegenüber den sonst üblichen Zell-Klonierungsstrategien dar, welche Reaktionszeiten von mehreren Tagen bis zu mehreren Wochen benötigen.

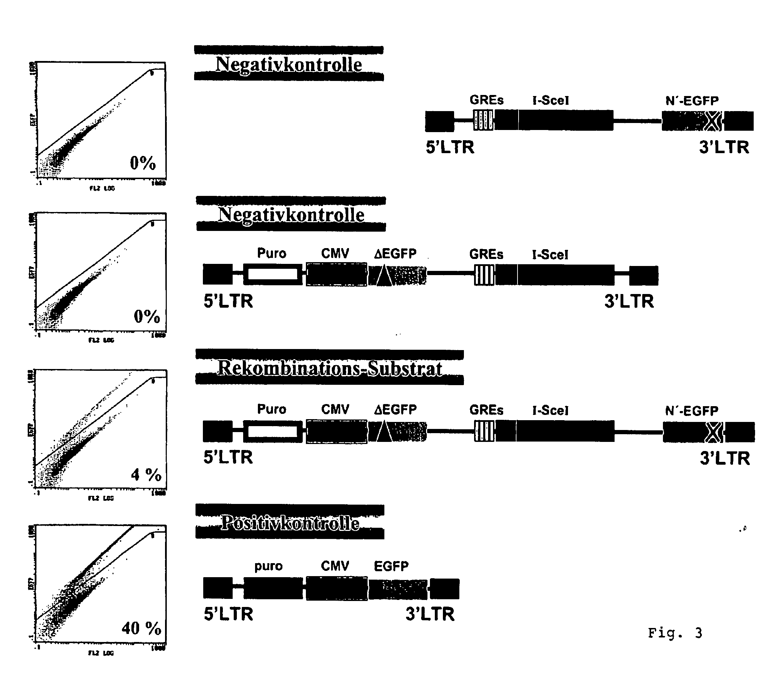

Erfindungsgemäß wurden Rekombinations-Experimente zunächst in transienten Elektroporations-Assays mit dem Rekombinations-Konstrukt aus Abb. 1 in KMV-Zielzellen durchgeführt. Nach Anschaltung der I-*Sce*I-Meganuklease wurde für einige % der Zellen am FACS-Scan Cytometer eine geringfügige Verschiebung der Fluoreszenz-Intensitäten beobachtet. Diese von I-*Sce*I und vom Rekombinations-Substrat abhängige Verschiebung im Histogramm deutete zwar eine durch Rekombination erzielte Rekonstitution des Wildtyp-*EGFP*-Gens an, war aber aufgrund der geringfügigen Fluoreszenzsteigerung schwer quantifizierbar. Um sichere Aussagen über erfolgreiche EGFP-Gen-Rekonstitution treffen zu können, wurde eine Fluoreszenz-Intensitäts-Steigerung von 1-2 Größenordnungen angestrebt. Eine klarere Trennung der endogenen orangen Autofluoreszenz gegenüber der grünen Fluoreszenz im *Dot Plot* versus Histogramm (FL2 versus EGFP in Abb. 3) und die Darstellung am Fluoreszenzmikroskop sollte ermöglicht werden. Aus diesem Grunde wurden Strategien zur Maximierung der Fluoereszenzintensitäten, sowie der Rekombinations-Frequenzen verfolgt.

### 3.4 Maximierung der Signale zum Nachweis der Homologen Rekombination

Da es sich derzeit beim EGFP um das leuchtstärkste autofluoreszierende Protein handelt, wurde nicht der AFP-Typ selbst sondern dessen Art der Expression verändert. Interessanterweise zeigte sich beim direkten Vergleich der Leuchtstärken des Puromycin-N-Acetyltransferase-EGFP-Fusionsproteins und dem EGFP, daß das Fusionsprotein eine 10 fach geringere Fluoreszenz aufwies. Diese Beobachtung trifft in unterschiedlichem Ausmaß auf alle EGFP-Fusionsproteine zu, wie in Fachkreisen zunehmend klarer wird. Dazu kam, daß in Titrationsexperimenten ein Vergleich der Promotorstärken des retroviralen MPSV-5'LTRs und des CMV-Promotors des Cytomegalievirus eine 3-4 fach höhere Expressions-Rate für den CMV-Promotor aufdeckte. Konsequenterweise wurden der Rekombinations-Akzeptor *ΔEGFP* und das Puromycin-Resistenzgen getrennt und das Rekombinations-Konstrukt um einen CMV-Promotor zwischen dem Puromycin-Resistenzgen und dem Δ*EGFP*-Gen ergänzt (siehe Abb. 3 und Abb. 5b). Wie in Abbildung 3 dargestellt, konnten für die so modifizierten Konstrukte eindeutig grün leuchtende Zellen im *Dot* Plot im oberen linken Dreieck nach FACS-Analyse identifiziert werden. Diese leuchtende Subpopulation enthielt Zellen nach erfolgreicher Rekombination, welche durch transiente Einführung der Plasmide in GalERVP exprimierende KMV-Zellen durch Elektroporation und durch Östradiol-Behandlung, d.h. I-*Sce*I-Anschaltung, provoziert wurde. Schließlich reichte die Leuchtintensität nun auch für den Nachweis grün leuchtender Zellen am Immunfluoreszenzmikroskop aus. Bei der Analyse von Negativkontroll-Konstrukten, welchen eines der beiden *EGFP*-Gen-Varianten fehlte, konnten keinerlei fluoreszierende Zellen festgestellt werden. Dies machte klar, daß intakte EGFP-Gen-Sequenzen durch Rekombination zwischen dem gezielt geschnittenen Akzeptor-Substrat und dem Donor-Substrat rekonstitutiert worden waren. Die Rekombinations-Frequenz lag bei 4 % bezogen auf die Gesamtpopulation der Zellen, bzw. bei 10 % bezogen auf transfizierte Zellen. Die Elektroporation mit einem Wildtyp-*EGFP*-Gen haltigen Konstrukt zeigte Elektroporations-Effizienzen von 40 % der Zellen an (Abb. 3).

Neben der Steigerung der EGFP-Fluoreszenz-Intensität wurden die Raten der homologen Rekombination durch Maximierung der homologen Bereiche zwischen dem Donor- und dem Akzeptor-Substrat erhöht (Waldman, A.S. und Liskay, M. (1988) Mol. Cell. Biol. 8: 5350-5357). Zu diesem Zweck wurden die zum Donor-Substrat homologen Bereiche im Akzeptor-Substrat erweitert, indem nicht wie im Akzeptor *△EGFP* insgesamt 45 Basenpaare, sondern nur 4 Basenpaare im für die Chromophor-Aminosäuren kodierenden Bereich durch die I-SceI-Erkennungssequenz ersetzt wurden (EGFP-HR in Abb. 4). Zweitens wurde als Donor-Substrat statt dem 443 Basenpaar langen *N'-EGFP* der gesamte 720 Basenpaar lange kodierende Wildtyp-*EGFP*-cDNA-Bereich verwendet, um die Homologie zu den Akzeptor-Substraten *△EGFP* und *EGFP-HR* im 3'-Bereich zu vergrößern (siehe Abb. 5c). Wildtyp-*EGFP* kann in dieser Anordnung, d.h. ohne eigenen Promotor, in Eukaryonten nicht polycistronisch exprimiert werden. Nach Einführung der entsprechenden Konstrukte in KMV-Zellen durch Elektroporation und nach Anschaltung der I-SceI-Meganuklease durch Östradiol wurden mit *EGFP-HR* ähnliche Rekombinations-Frequenzen wie für Δ*EGFP* gemessen, mit Wildtyp-*EGFP* jedoch eine deutliche Steigerung auf 30 % der transfizierten Zellen erreicht. Jedoch wurde auch in Experimenten mit der Akzeptor-Substrat freien Negativkontrolle eine geringe Anzahl (1-2 %) von grün leuchtenden Zellen beobachtet. Dies bedeutete, daß entweder durch kryptisches Spleissen oder durch interne Proteintranslations-Initiation Wildtyp-*EGFP* produziert werden konnte. Um dieses auszuschließen, wurde eine weitere Verbesserung ins System integriert, ohne dabei jedoch die hohen Rekombinations-Frequenzen zu ändern. Hierfür wurde das Startkodon des Wildtyp-EGFP-Gens im Akzeptor-Bereich durch PCR-Technologie in zwei aufeinanderfolgende Stopkodons umgewandelt (Abb. 5d). Dadurch blieben maximale Homologien aufrechterhalten, gleichzeitig aber EGFP-Expression außer nach erfolgreicher Rekombination vollständig unterdrückt. Das neue Donor-EGFP-Gen wurde als *met-*>stop bezeichnet. 18-38 % der elektrotransfizierten Zellen durchliefen DNA-Austausch-Prozesse mit dem entsprechenden Konstrukt. Zu beachten bleibt hier, daß gleichzeitig mit der Erweiterung der Homologien durch Wildtyp-*EGFP* oder durch das *met*->*stop*-Gen im Gegensatz zu *N'-EGFP* in dieser Anordnung auch nichtkonservative homologe Rekombination ermöglicht wurde (siehe Kapitel 3.5). Denn 189 Basenpaare 5' von der I*-Sce*I-Erkennungssequenz sind homolog zwischen dem *met->stop-* und dem *N'-EGFP-Gen* und stehen für nichtkonservative homologe Rekombinations-Prozesse zur Verfügung. Insgesamt war also die 7 fache Steigerung der Rekombinations-Frequenzen mit dem Wildtyp-*EGFP-* bzw. dem *met->stop-*Gen im vergleich zum *N'-EGFP-Gen* als Donor sowohl durch die Verlängerung der homologen Paarungsbereiche als auch durch die Miterfassung von nichtkonservativen Rekombinations-Prozessen erklärbar.

### 3.5 Nachweispotential für Konservative und Nichtkonservative Homologe Rekombinations-Ereignisse, End Joining sowie Punktmutationen

Spontane Genkonversions-Raten, gemessen in immortalisierten Säugerzellen, liegen bei 10⁻⁵ bis 10⁻⁶ pro Zelle. Ist die Anordnung von Rekombinations-Substraten dergestalt, daß neben konservativen Ereignissen, wie der Genkonversion, auch nichtkonservative Ereignisse, wie das SSA, ablaufen können, kommt es zu einer Steigerung der spontanen Raten, so daß diese nun im Bereich von 10⁻⁴ bis 10⁻⁵ liegen (Lambert, S. et al. (1999) Mutat. Res. 433: 159-168; Moynahan, M.E. et al. (1999) Mol. Cell 4: 511-518; Dronkert, M.L.G. et al. (2000) Mol. Cell. Biol. 20: 3147-3156). Der Verlust der regulatorischen Funktionen des Tumorsuppressors p53 bei der homologen Rekombination in Säugerzellen, wie den vorliegend verwendeten K562-, WTK1- oder MethA-Zellen führt zu einer Steigerung um mindestens eine Größenordnung (Akyüz et al. (2000) Gen. Ther. Mol. Biol. 5, 1-17). Dies bedeutet, daß bei Nachweis aller Typen der homologen Rekombination bereits spontane, mit Sicherheit aber durch Noxen gesteigerte Rekombinations-Frequenzen am FACS-Scan Durchflußzytometer mit dem erfindungsgemäßen Nachweissytem direkt darstellbar sind.

Um die Möglichkeit zu schaffen, völlig unabhängig von der I-SceI-Meganuklease, dafür aber z.B. in Abhängigkeit von Umwelt-, Lebensmittelgiften oder Chemotherapeutika DNA-Rearrangements nachzuweisen, wurde das Rekombinations-Konstrukt in weiteren varianten vollständig von der I-*Sce*I-Expressions-Kassette befreit und durch ein Hygromycin-Resistenz-Gen mit SV40- oder CMV-Promotor ersetzt (Abb. 5g und h). In den folgenden Experimenten konnten mit diesen neuen Varianten nun auch spontane DNA-Austausch-Raten gemessen werden. Repräsentativ für eine starke DNA-Schädigung durch exogene Faktoren wurden parallel dazu die I-SceI-Meganuklease durch Elektroporation mit einem entsprechenden Expressions-Konstrukt transient in den Zielzellen produziert.

Um insbesondere mit Blick auf den Nachweis der von I-SceI unabhängigen, also selteneren Austausch-Ereignisse optimale Voraussetzungen für nichtkonservative Rekombinations-Ereignisse zu schaffen, wurde ein neues Konstrukt erzeugt, welches speziell auf die Messung dieser am häufigsten vorkommenden homologen Rekombinations-Form zugeschnitten wurde (Lambert, S.et al. (1999) Mutat. Res. 433: 159-168). Gleichzeitig sollte mit diesem Konstrukt abgeschätzt werden, welchen Beitrag nichtkonservative Rekombinations-Ereignisse auf die Austausch-Raten im Konstrukt mit dem met->stop-Donor-Substrat leisten. Dazu wurde das *EGFP-HR*-Gen entgegen der bisherigen Anordnung 3' vom *N'-EGFP*-Gen positioniert und die beiden *EGFP*-Gen-Varianten durch eine Expressions-Kassette für das Wildtyp-*DsRed*-Gen getrennt (Abb. 5i, Kapitel 5 Beispiel 1 und Kapitel 6 Sequenzprotokoll). Auf diese Art und Weise steht zwischen der I-*SceI*-Erkennungssequenz und dem 3'-Ende im *EGFP-HR*-Gen eine Sequenz von 242 Basenpaaren mit Homologien zum *N'-EGFP-Gen* zur Verfügung. *Single Crossover,* SSA oder Replikationssprünge können über diese Sequenzen ein intaktes Wildtyp-*EGFP-*Gen rekonstituieren. Diese nichtkonservativen Rekombinations-Vorgänge wären gleichzeitig mit dem Verlust des DsRed-Gens verbunden, und können somit sowohl über grüne Fluoreszenz wie auch über den Verlust der roten Fluoreszenz nachgewiesen werde. Aber auch fehlerhafte nichtkonservative Rekombinations-Vorgänge, welche kein intaktes Wildtyp-*EGFP*-Gen rekonstituieren, sind so über den Verlust der roten Fluoreszenz nachweisbar. Auch Punktmutationen im *DsRed*-Gen, welche die Autofluoreszenz des Genproduktes stören, sollten so erfaßbar sein. AFPs benötigen für die Ausübung der vollen Autofluoreszenz die perfekte Faltung des Proteins, so daß eine Vielzahl von kleinen Änderungen innerhalb der kodierenden Sequenzen mit dem Verlust der Fluoreszenz einhergehen (Cubitt, A.B. et al. (1995) Trends Biochem. Sci. 20: 448-455). Eine Wechselwirkung zwischen den von EGFP und den von *DsRed* abgeleiteten Sequenzen ist nicht zu erwarten, da das *EGFP-*Gen aus der Qualle *Aequorea* victoria isoliert wurde, das *DsRed*-Gen hingegen von der Seeanemonen-Verwandten *Discosoma sp.* stammt, also keine signifikanten Homologien zwischen den beiden Genen existieren. Promotor-Interferenzen zwischen den beiden CMV-Promotoren vor dem N'*-EGFP-*Gen bzw. dem *DsRed*-Gen konnten aufgrund ähnlicher Experimente von Kollegen bereits im Vorfeld ausgeschlossen werden (Dr. Geoffrey Margison, Manchester, persönliche Mitteilung). Dieses Analyseprinzip kann bei einer Beschränkung auf die hochfrequenten inaktivierenden Ereignisse durch Verwendung beliebiger, also nicht notwendigerweise für AFPs kodierender, homologer Sequenzen flankierend zum Sequenzabschnitt [*Ins2*], in diesem Beispiel dem *DsRed-Gen,* erzielt werden.

Zur Bestätigung des Funktionsprinzips wurden nach transienter Elektroporation von K562-Zellen mit dem Konstrukt i von Abb. 5 mit *EGFP-HR-*Gen oder einem Kontrollkonstrukt ohne [*Marker1*] zur Bestimmung der Transfektionseffizienz über rote Fluoreszenz am FACS-Scan Cytometer nach Anregung bei 488 nm auf grüne EGFP-Fluoreszenzen bzw. auf rote DsRed-Fluoreszenzen hin untersucht. Unter den transfizierten Zellen zeigten 4 % der Zellen grüne Fluoreszenz. In den Negativkontrollen mit Kontrollkonstrukt wurden keinerlei grün fluoreszierende Zellen beobachtet. Das heißt, daß im transienten Elektroporations-Test die Raten der Wildtyp-*EGFP*-Gen-Rekonstitution ähnlich hoch für Konstrukt i wie für Konstrukt h in Abb. 5, welches im Gegensatz zu i außer nichtkonservativer homologer Rekombination auch Genkonversion nachweist, waren, nämlich bei 3-12 % wieder bezogen auf transfizierte Zellen. Nach Koelektroporation des Konstruktes i aus Abb. 5 oder einem abgeleiteten Konstrukt mit dem Wildtyp-*EGFP*-Gen im [*Marker1*] *-* Sequenzabschnitt und einem Expressionsplasmid für die Meganuklease I-SceI konnten durch die Einführung eines DSBes im *EGFP-HR*-Gen gesteigerte Raten für die Wildtyp-EGFP-Gen-Rekonstitution, nämlich von 13 % in transfizierten Zellen festgestellt werden. Im Vergleich dazu lag die durch I-Scel induzierte Rekombinations-Frequenz für das Konstrukt h in Abb. 5 bei 6-22 % bezogen auf transfizierte Zellen. Unter den transfizierten Zellen kam es zu einer Abnahme der deutlich rotfluoreszierenden Zellen um 56 % in Abwesenheit von I-*Sce*I und von 76 % in Gegenwart von I-SceI. Letztere lagen für das Konstrukt mit dem Wildtyp-*EGFP*-Gen im *[Marker1]-*Sequenzabschnitt bei 98 %. Dies bedeutet, daß inaktivierende Rekombinations- und Mutations-Ereignisse in Abwesenheit von I-SceI um bis zu 14 fach über den Werten für rekonstituierende Ereignisse lagen. Eine weitere Zunahme der inaktivierenden Ereignisse kann, wie hier mit Hilfe des Wildtyp-*EGFP*-Gens im Vergleich zum *N*'-*EGFP-*Gen gezeigt, durch Verlängerung der homologen Bereiche erzielt werden. Dies stellt eine weitere wichtige Sensitivitätssteigerung für die Gentoxizitäts-Bestimmung dar. Weitere Verbesserungsmöglichkeiten liegen in der Optimierung der Anregungswellenlängen (488 nm für EGFP versus 558 nm für Red). Außerdem sind inaktivierende Ereignisse nach stabiler Integration der Konstrukte ins zelluläre Genom durch den totalen Verlust der Red-Fluoreszenz und nicht nur durch die Absenkung des Fluoreszenzsignals unter einen Schwellenwert zu erkennen. Insbesondere für diese Anwendung erscheint das Laser Scanning Cytometer von Compucyte als ein geeignetes Werkzeug, also zum Aufspüren einzelner, nicht fluoreszierender Zellen in einer Population fluoreszierender Zellen, da dieses Gerät die Vorteile der Einzelzellanalyse am Fluoreszenzmikroskop mit den Vorteilen der Populationsanalyse am FACS-Scan Cytometer vereinigt. Eine weitere Verbesserung dieses Tests besteht in dem Einsatz von zwei Genen kodierend für AFPs mit noch deutlicher als EGFP und Red voneinander getrennten Anregungs- und Emissions-Spektren. Zukünftige nichthomologe Varianten gelb und cyan fluoreszierender Proteine wären hierfür geeignete Kandidaten. Darüberhinaus repräsentiert auch der Einsatz beliebiger, jedoch homologer Sequenzen in den Sequenzabschnitten [*Marker1*] und *[Marker2],* und damit auch von Sequenzen mit beliebig langen Sequenzhomologien, eine interessante Verbesserungsmöglichkeit zum Nachweis von inaktivierenden Ereignissen im [*Ins*2]-Sequenzabschnitt.

wie erwähnt repräsentiert auch EJ eine wichtige Form der DSB-Reparatur in Säugerzellen (Critchlow, S.E. und Jackson, S.P. (1998) Trends Biochem. Sci. 23: 394-398). Um in Verbindung mit der Entstehung von DSBen durch die Meganuklease I-SceI EJ und in Verbindung mit EJ Gentoxizität messen zu können, wurde ein speziell für den Nachweis dieses Rekombinations-Typs designiertes Substrat generiert. Da beim EJ im Gegensatz zum homologen DNA-Austausch keine zwei homologen Partner nötig sind, enthalten die in Abb. 5e und f dargestellten Rekombinations-Konstrukte nur ein Rekombinations-Marker-Gen, nämlich *EGFP-EJ.* Zur Einführung eines gezielten DSBes- enthält *EGFP-EJ* wiederum im EGFP-Sequenz-Bereich, welcher für Chromophor-Aminosäuren kodiert, eine I-SceI-Erkennungssequenz (siehe Abb. 4) und außerdem im Konstrukt e der Abb. 5 eine von Gal4ER-VP anschaltbare I-SceI-Expressions-Kassette. Darüberhinaus wurden 5 Basenpaare der *EGFP*-Sequenz verdoppelt, so daß 5' und 3' von der I-SceI-Erkennungssequenz 5 Basenpaar lange Homologien existieren. Diese kurzen Homologien dienen beim EJ nach Einführung eines DSBes durch I-SceI zur Rekonstitution der Wildtyp-*EGP*-Sequenz. Auch hier wurde das Funktionsprinzip nach transienter Elektroporation des Konstruktes in KMV-Zellen geprüft. Die Produktion der I-SceI-Meganuklease erfolgte über Östradiol-Gabe oder Koelektroporation mit einem I-*Sce*I-Expressionsplasmid. Die Raten für erfolgreiches EJ ergaben sich wieder aus dem Anteil grün leuchtender Zellen und wurden per FACS-Scan Cytometer bestimmt. Diese lagen mit 1-3 % in transfizierten Zellen unter den für homologe Rekombinations-Typen bestimmten Werten. Um generell neben der homologen Rekombination auch die Möglichkeit des EJ sowie des an EJ gekoppelten homologen DNA-Austausches (Johnson, R.D. und Jasin, M. (2000) EMBO J. 19: 3398-3407) zu integrieren, wurden *△EGFP* bzw. *EGFP-HR* durch *EGFP-EJ* in den existierenden Rekombinations-Konstrukten in zusätzlichen Varianten ersetzt (Abb. 5 a) b) c) d) g) h) i) k) ) .

Insgesamt wurden im Rahmen dieser Erfindung Konstrukte generiert, welche den Nachweis von konservativen homologen Rekombinations-Prozessen, EJ und nichtkonservativen homologen Rekombinations-Prozessen oder von nichtkonservativen homologen Rekombinations-Prozessen und Mutationen als Antwort auf gentoxische Behandlung erlauben. Die in Abb. 5j dargestellte Kombination aus den optimierten Varianten unter h und i sollte schließlich alle genannten Möglichkeiten zum Nachweis von genetischen Veränderungen als Indikatoren für Gentoxizitäten vereinen. Darüberhinaus wahrt diese optimierte Variante in Abb. 5j vor allem auch die in h dargestellte Anordnung zweier *EGFP-*Gen-Varianten und die damit erreichten maximalen spontanen Rekombinations-Raten .

Im Vergleich zu den Testsystemen für homologe Rekombination von anderen Wissenschaftlern (Pierce, A.J. et al. (1999) Genes & Development 13: 2633-2638; Slebos, R.J.C. und Taylor J.A. (2001) Biochem. Biophys. Res. Commun. 281: 2121-2129) ist die vorliegende Erfindung eines Gentoxizitäts-Bestimmungs-Verfahrens technisch dadurch überlegen, daß ein AFP-Fusionsprotein vermieden wird und ausschließlich intensiv leuchtende AFPs eingesetzt werden und darüberhinaus die homologen Sequenzbereiche bis hin zur beliebigen Länge maximiert werden können, und damit eine sehr hohe Sensitivität des erreicht wird. Ferner liegen die Vorteile darin, daß die Möglichkeit besteht verschiedenste Rekombinations-Typen, Mutationen und vor allem auch die besonders häufigen Gen-inaktivierenden Ereignisse nachzuweisen. Weiter besteht die Möglichkeit zur gezielten Einführung von DSBen durch I-SceI in das Genom lebender Zellen, ohne die Notwendigkeit der Transfektion mit einem Expressions-Plasmid. Schließlich liegt ein Vorteil auch im Transfer der Testkonstrukte mittels Retroviren (s. Kapitel 3.6). Der gewählte Zelltyp schafft darüberhinaus die Voraussetzungen für die Automatisierung durch Suspensionskultur und Apoptose-Unempfindlichkeit bei gleichzeitig hohen Raten an DNA-Rearrangements.

### 3.6 Anwendungspotential des Testsystems für den Nachweis von Gentoxizitäten

Das Funktionsprinzip des Rekombinations- und Gentoxizitäts-Nachweises wurde auch nach stabiler Integration der Konstrukte ins zelluläre Genom bestätigt. Auf diese Weise wurden murine MethA-, humane WTK1-, humane K562- und die Gal4ER-VP produzierenden KMV-Zellen analysiert. Im Vergleich zu den transienten *Assays* nach Elektroporation mit den Rekombinations-Konstrukten werden nach stabiler Integration ins zelluläre Genom geringere Rekombinations-Raten erwartet, da transfizierte DNA nackt, also ohne Chromatinverpackung für Reparatur-Enzyme frei zugänglich ist, vor allem aber auch da nach Transfektion höhere Kopienzahlen an Rekombinations-Substraten pro Zelle vorliegen. Die Rekombinations-Marker wurden stabil ins Genom inseriert, indem die Zellen mit der entsprechenden DNA elektroporiert wurden, gegenüber Puromycin resistente Einzelzell-Klone isoliert wurden und die Integrität des Rekombinations-Konstruktes über PCR-Analysen an genomischer DNA der Einzelzell-Klone bestätigt wurde. Für das in Abb. 5h beschriebene Konstrukt mit *EGFP-EJ-Akzeptor* wurden über EGFP-Fluoreszenz in 6 voneinander unabhängigen Klonen Rekombinations-Ereignisse in 0,2-0,6 % der mit einem I-SceI-Expressions-Plasmid elektrotransfizierten Zellen nachgewiesen. Spontane Rekombinations-Raten lagen bei 10⁻⁵. Die postulierten DNA-Rearrangements wurden für entsprechende Zellpopulationen nach erfolgreichem DNA-Austausch mittels genomischer PCR bewiesen, analog zum Nachweis der Integrität der Rekombinationskontrukte nach Integration ins zelluläre Genom.

Das Chemotherapeutikum und TopoisomeraseII-Inhibitor Etoposid kann DSBe auslösen, wird aber vom Ames *Assay* nicht erfaßt. Um die Anwendbarkeit des erfindungsgemäßen Testsystems als Gentoxizitäts-Nachweissystem zu belegen wurden ionisierende Strahlung und Etoposid in ihrer Wirkung untersucht. Hierfür wurden 7 unterschiedliche von K562-Zellen abgeleitete Klone mit stabil ins Genom integrierten Testkonstrukten (h in Abb. 5) mit 500 Rad bestrahlt und die Fluoreszenzen von je 30000 Zellen 24 h , 48 h, 72 h und 96 h nach Behandlung im Vergleich zu unbehandelten Zellen am Durchflußzytometer analysiert. Die Ergebnisse zeigten, daß das Auftauchen grünfluoreszierender Zellen nach Bestrahlung zu mehr als einem Zeitpunkt bei zwei Zellinien beobachtet wurde, wohingegen in unbehandelten Zellen keine Fluoreszenzsignale detektiert wurden. Ebenso wurde nach zweistündiger Etoposid-Behandlung (100 µM) das Auftauchen grünfluoreszierender Zellen registriert. Die gemessenen Raten lagen bei 0,3-1 x 10⁻⁴. Dies bedeutet, daß das erfindungsgemäße Testsystem die Voraussetzungen schafft, um die Auswirkungen von Strahlung, Chemotherapeutika oder anderen Noxen in menschlichen Zellen als Zunahme der DNA-Austauschraten zu bewerten.

Um die effiziente und kontrollierte Übertragung der Test-Konstrukte in jede beliebige Säugerzelle zu ermöglichen, wurden diese grundsätzlich innerhalb eines retroviralen Vektorgerüstes generiert. Als Grundlage der in Abb. 5a bis j dargestellten Vektoren diente der von Frau Dr. Carol Stocking, Heinrich-Pette-Institut Hamburg, entwickelte retrovirale Vektor p5NM, welcher sich vom Genom des myeloproliferativen Sarkom-Virus, MPSV, ableitet. Zwischen 5'LTR mit Verpackungssignal und dem 3'LTR wurden die Rekombinations-Marker jeweils einkloniert, so daß zwischen den LTRs positionierte Sequenzen in entsprechenden Helferzellinien in Retroviren verpackt und anschließend über retrovirale Transduktion in das Genom der gewünschten Zellen integriert werden können. Dies repräsentiert gegenüber chemischen und physikalischen Methoden des Gentransfers den wesentlichen Vorteil, daß die lineare Anordnung der Marker-Sequenzen bewahrt wird und multiple Tandem-Insertionen, wie man sie vor allem nach Calcium-Phosphat-Kopräzipitationen häufig findet, vermieden werden. Die Aufrechterhaltung der Anordnung der einzelnen Elemente innerhalb der Vektoren wiederum ist die Voraussetzung für die nachzuweisenden DNA-Austausch-Vorgänge. MPSV-Retroviren im Vergleich zu den MoMuLV-Viren haben darüberhinaus den Vorteil, daß LTR-gesteuerte Expression auch in embryonalen Zellen möglich ist (Akgün, E. et al. (1991) J. Virol. 65: 382-388).

Zur Produktion infektiöser Retroviren-Partikel wurden Zellen der Helferzellinie PA317 mit den Vektoren unter b, d, h, i und k in Abb. 5 einschließlich Rekombinations-Marker freier Negativ- und Wildtyp-*EGFP* haltiger Positivkontrollen elektroporiert. PA317 repräsentiert eine amphotrophe Retroviren-Helferzellinie, d.h. sie trägt alle Gene zur Verpackung von replikationsdefizienten Retroviren, welche Zellen von Maus, Mensch, Huhn, Hund und Katze infizieren können (Miller, A.D. und Buttimore, C. (1986) Mol. Cell. Biol. 6: 2895-2902). Da retroviralen Vektoren die gag-, pro-, pol-, und env-Gene fehlen, werden in infizierten Wirtszellen keine neuen Viren mehr gebildet. Mittels Puromycin-Selektion wurden PA317-Einzell-Klone isoliert und die lineare Insertion der Rekombinations-Marker ins Genom der einzelnen PA317-Linien über PCR-Analysen bewiesen. Infektiösität der Retroviren in den Überständen der jeweiligen Zellinie wurde für die Typen unter b, d und k in Abb. 5 geprüft und demonstriert. Nach der Infektion von murinen MethA- bzw. menschlichen K562-Zellen mit diesen neuen Retroviren belegten PCR-Analysen an den genomischen DNAs die Integrität der zwischen den LTRs ins Genom inserierten Sequenzen. Darüberhinaus wiesen MethA- und K562-Zellen, welche mit Viren der Wildtyp-*EGFP* haltigen Positivkontrolle zu den Vektortypen b bis j in Abb. 5 infiziert worden waren, deutlich sichtbare EGFP-Autofluoreszenz auf. Da Proviren als Einzelkopien ins zelluläre Genom inseriert werden, bedeutet dies für zukünftige Experimente zur Bestimmung von Gentoxizitäten, daß im hier vorgestellten Vektor-Kontext die Rekonstitution einer einzigen Wildtyp-*EGFP*-Gen-Kopie pro Zelle ausreicht, um die Auswirkungen einer gentoxischen Behandlung anzuzeigen.

Schließlich wurden im Rahmen der vorliegenden Erfindung auf der Basis des retroviralen Vektors pRetroOn von Clontech Konstrukte erzeugt, welche innerhalb eines einzigen Vektors Elemente für die Tetrazyklin-induzierbare Expression von I*-Sce*I-Meganuklease, für die konstitutive Expression des von Tetrazyklin abhängigen Transkriptionsfaktors rtTA und für die von EGFP abgeleiteten Rekombinations-Substrate enthalten (Abb. 5k). pRetroOn leitet sich vom murinen Moloney Leukämievirus (MoMuLV) ab. pRetroOn ermöglicht eine durch Tetrazyklin induzierbare I-SceI-Expression über einen Promotor (TRE), welcher durch den revers von Tetrazyklin kontrollierten Transaktivator (rtTA) in Gegenwart von Tetrazyklin oder Doxyzyklin aktiviert wird. Die für rtTA kodierenden Sequenzen sitzen bei diesen Konstrukten zwischen den Akzeptor-Substraten *ΔEGFP, EGFP-HR* bzw. *EGFP-EJ* und dem *I-SceI* -Gen. Der Aufbau entspricht ansonsten der unter b in Abb. 5 dargestellten Variante. Diese und davon abgeleitete Konstrukte haben den Vorteil, dai3 mit ihnen, falls gewünscht, auch im Tiermodell I-SceI-Expression steuerbar sein sollte, da es im Gegensatz zu Östradiol nicht natürlicherweise in Säugetieren vorkommt.

Das dargestellte Testssystem in seinen verschiedenen Ausführformen schafft gute Voraussetzungen auch für eine mögliche Automatisierung des *Assays* zur routinemäßigen Bestimmung von Gentoxizitäten in Säugerzellen. Hierzu tragen einerseits die maximierte Leuchtintensität des AFP-Reporters und andererseits die Erfassung möglichst vieler Reparatur-Antworten auf DNA-Schädigung bei. Darüberhinaus erleichtert im Falle aller bisher zur Analyse eingesetzten Zelltypen die Möglichkeit der Suspensionskultur die Zellzucht im Großmaßstab (Moore, G.J. und Matsoukas, J.M. (1985) Bioscience Reports 5: 407-416) sowie die Apoptose-Unempfindlichkeit bei gleichzeitig hohen Raten an DNA-Rearrangements (Mahdi, T., D. et al. (1998) Biol. Chem. 90: 15-27).

Der erfindungsgemäße *Assay* zur Bestimmung von Gentoxizitäten über den Nachweis von DNA-Rearrangements kann weiterhin auf transgene Mäuse übertragen werden. Im Tiermodell kann Rekombination nur nachgewiesen werden über Gene, deren Produkte eine Färbung oder Fluoreszenz auslösen, aber nicht mit einem Resistenz-Gen, wie in den meisten der existierenden Gentoxizitäts-Nachweissysteme eingesetzt. Da der extrem starke CMV-Promotor jeweils vor das vom EGFP-Gen abgeleitete Akzeptor-Substrat geschaltet wurde, muß aufgrund existierender Studien erwartet werden, daß mit den optimierten Konstrukten (Abb.en 5h bis j) Autofluoreszenzen und damit Rekombinations-Ereignisse in situ per Fluoreszenz-Mikroskopie qualitativ und per Laser Scanning Cytometrie (Compucyte) quantitativ nachgewiesen werden können (Walter, I. et al. (2000) Histochemical Journal 32: 99-103). Dies bedeutet, daß Gewebeschnitte direkt ohne Färbung auf Autofluoreszenzen und damit direkt auf DNA-Rearrangements analysiert werden können. Zur Erzeugung der entsprechenden transgenen Mäuse müssen die Test-Konstrukte in plasmidaler Form durch Mikroinjektion in den Pronukleus fertilisierter Oozyten injiziert werden. Nach Reimplantation in pseudoschwangere Ammenmäuse müssen transgene Nachkommen mit intakten Integraten durch PCR-Analysen und *Southern-Blots* von genomischer DNA aus Mäuseschwänzen identifiziert werden. Schließlich besteht aufgrund des durchgehend verwendeten retroviralen Vektordesigns auch die Möglichkeit jedes beliebige der beschriebenen Konstrukte über retrovirale Transduktion in die Zellen muriner Embryonen einzuführen. Mit dieser Methode generierten bereits 1984 Jänisch und Kollegen transgene Mäuse (Stuhlmann, H. et al. (1984) Proc. Natl. Acad. Sci. USA 81: 7151-7155), in welchen ein bakterielles Transgen in vielen somatischen Geweben aktiv war. Auch für diesen Zweck ist es von großem Vorteil, daß die Rekombinations-Marker CMV-Promotoren nachgeschaltet sind, also die Gefahr einer Abschaltung der Promotoren in den transgenen Tieren durch DNA-Methylierung, wie man sie für retrovirale LTRs in Zellen der Keimbahn beobachtet, verringert ist (Jähner, D. et al. (1985) Proc. Natl. Acad. Sci. USA 82: 6927-6931). Mit dieser Erfindung wurden also auch die Vorraussetzungen geschaffen, um Fragen zum Fremdstoffmetabolismus und zur Toxikologie in spezifischen Geweben oder für bestimmte Zelltypen zu beantworten. Die erfolgreiche Etablierung eines Mausmodells schafft darüberhinaus die Voraussetzungen, um hierbei den Einfluß bestimmter endogener Faktoren zu analysieren (nach Einkreuzen von Knockout- oder transgenen Mäusen) und um mit der DSB-Reparatur verbundene Änderungen der Gen-Expressions-Profile zu erfassen (*Mikroarray*-Technik). Dadurch können genetisch bedingte Sensitivitäten bzw. Resistenzen gegenüber bestimmten Noxen aufgezeigt werden. Diese Informationen können der Entwicklung individueller Chemotherapie-Protokolle für genotypisierte Patienten dienen.

Die Erfindung wird nachfolgend anhand von Abbildungen, Beispielen sowie einem Sequenzprotokoll erläutert.

### Beschreibung der Abbildungen (Figuren):

### Abbildung 1: Prinzip des Rekombinations-Testsystems basierend auf der Rekonstitution eines AFP-Gens.

Dargestellt ist das einfachste Vektor-Design für die Analyse von konservativen homologen Rekombinations-Prozessen. Das Vektorrückgrat wird vom retroviralen Vektor p5NM von Frau Dr. Carol Stocking, Heinrich-Pette-Institut Hamburg, gebildet, welcher sich vom MPSV-Retrovirus ableitet (*5'LTR, 3'LTR* und durchgezogene schwarze Linie) (Laker, C. et al. (1998) J. Virol. 72: 339-348). Das Puromycin-Resistenzgen (*Puro*) wird vom 5' LTR-Promotor aus transkribiert und die kodierte Puromycin-N-Acetyltransferase in Fusion mit einem in der Chromophor-Region mutierten EGFP-Protein *(ΔEGFP)* exprimiert. Die 45 in *ΔEGFP* deletierten Basenpaare wurden durch eine *EcoRI* und eine *I-SceI-Erkennungs-Sequenz* ersetzt. Die kodierenden Sequenzen für die I-SceI-Meganuklease *(I-SceI)* werden ausgehend von den Gal4-Erkennungssequenzen *(GREs)* mit Hilfe des durch Östradiol aktivierbaren Transkriptionsfaktors Gal4ER-VP abgelesen. Das im 3'-Bereich des *EGFP-Gens* verkürzte *N'-EGFP-*Gen ist keinem Promotor nachgeschaltet. Durch Östradiol-Gabe wird der Gal4ERVP-Transkriptionsfaktor aktiviert und dadurch wiederum die I-*Sce*I-Meganuklease exprimiert. Die I-*Sce*I-Meganuklease schneidet die DNA in einem versetzten Schnitt innerhalb ihrer Erkennungs-Sequenz im Δ*EGFP*-Gen. Dies initiiert DSB-Reparatur-Prozesse, welche durch unilateralen homologen DNA-Austausch zwischen dem N'-*EGFP-* und dem Δ*EGFP*-Gen zur Rekonstitution eines Wildtyp-*EGFP*-Gens führen können. Graue, gewinkelte Pfeile zeigen die Orientierung der Promotoren, schwarze Pfeile die Lesrichtung der translatierten Sequenzen an. Die I-SceI-Meganuklease wurde durch ein Scherensymbol dargestellt.

### Abbildung 2: Durch Östradiol-Gabe induzierbare Expression von I-SceI-Meganuklease.

KMV-Zellen exprimieren den durch Östradiol aktivierbaren Transkriptionsfaktor GalERVP, welcher aus der DNA-Bindedomäne des Gal4-Transkriptionsfaktors *(Ga14),* der Östradiol-Bindedomäne des humanen Östradiol-Rezeptors *(ÖR)* und der Transaktivierungs-Domäne des Herpesvirusproteins VP16 *(VP16)* besteht. KMV-Zellen wurden mit dem Vektor *pGC-Sce* elektroporiert, welcher 4 synthetische Gal4-Erkennungssequenzen als Promotor vor der für I-*Sce*I kodierenden Sequenz enthält. Nach Selektionierung einzelner Klone mit stabil ins Genom integrierter I-SceI-Expressions-Kassette wurden diese in Gegenwart von 2 µM β-Östradiol 24 h kultiviert. Die von Östradiol abhängige Expression der I-SceI-Meganuklease in Fusion mit dem Peptid-Epitop des anti-Haemagglutinin (HA)-Antikörpers 12CA5 ist für einen repräsentativen Klon im *western-Blot* gezeigt. KMV-Zellen, welche transient mit *pCMV-Sce,* einem konstitutiv *I-SceI* exprimierenden Plasmid elektroporiert wurden, dienten als Positivkontrolle.

### Abbildung 3: Messung der Rekombinations-Häufigkeiten nach gezielter Einführung eines Doppelstrangbruches.

Mittels Elektroporation wurde ein *Rekombinations-Substrat* in KMV-Zellen eingeführt, welches aus einem p5NM-Vektorrückgrat mit 5'LTR und *3'LTR,* einem Puromycin-Resistenzgen (*Puro*), einem *CMV*-Promotor mit nachgeschaltetem Δ*EGFP*-Gen als Rekombinations-Akzeptor, einem N'-*EGFP*-Gen als Donor und einer durch Östradiol anschaltbaren I-*Sce*I-Expressions-Kassette besteht. Die Zellen wurden im gezeigten Experiment 48 h in Gegenwart von Östradiol kultiviert und grün fluoreszierende Zellen am FACS-Scan Cytometer ausgewertet. Intensiv grün fluoreszierende Zellen erscheinen im *Dot Plot* grüner versus oranger Fluoreszenzen (*FL2* versus *EGFP)* als Signale links oberhalb der Diagonale, während die Signale für endogene orangefarbene Fluoreszenz der Zellen unterhalb der Diagonale angesiedelt sind. 4 % der Zellen leuchteten nach Anregung bei 488 nm grün, wohingegen keinerlei leuchtende Zellen in den *Negativkontrollen* für Konstrukte ohne Rekombinations-Akzeptor bzw. -Donor zu beobachten waren. In der *Positivkontrolle* mit Wildtyp*-EGFP-*Expression wurden 40 *%* leuchtende Zellen festgestellt. Dies entspricht der Transfektionseffizienz im Experiment.

### Abbildung 4: Mutationen in den Rekombinations-Akzeptor-Substraten ΔEGFP, EGFP-HR und EGFP-EJ für den Nachweis von homologen Rekombinations-Ereignissen und von End Joining.

Die in den *ΔEGFP-* und *EGFP-HR-*Genen im Vergleich zum Wildtyp-*EGFP*-Gen zwischen Basenpaar 163 und 220 der kodierenden Region veränderten Sequenzen sind oberhalb des Rekombinations-Konstruktes aus Abb. 1 eingezeichnet. Die im *EGFP-EJ-Gen* im Vergleich zum Wildtyp-*EGFP*-Gen veränderten Sequenzen sind unterhalb des Rekombinations-Konstruktes eingezeichnet. Nach Einführung eines gezielten DSBes in die jeweils synthetisch eingeführte I-SceI-Erkennungssequenz unterstützt *ΔEGFP* bzw. *EGFP-HR* homologen DNA-Austausch mit dem Donor-Substrat, *EGFP-EJ* unterstützt darüberhinaus auch End *Joining* mit Hilfe der Mikrohomologien in der duplizierten *CCTAC*-Sequenz. *ΔEGFP*enthält 5' der neuen I-SceI-Erkennungssequenz eine Erkennungs-sequenz für das Restriktionsenzym *E*coRI *(GAATTC).* Der graugefüllte Kreis zeigt eine weitere Basenänderung in *EGFP-HR*im Vergleich zum Wildtyp-*EGFP*-*Gen* an. Das Prinzip der *homologen* Rekombination bzw. des *nichthomologen End Joinings* sowie die daran beteiligten Faktoren sind schematisch dargestellt.

### Abbildung 5: Optimierung der Konstrukte zur Identifizierung von genetischen Veränderungen als Gentoxizitäts-Indikatoren.

Ausgehend vom Rekombinations-Testsystem in Abb. 1 werden schematisch alle Änderungen des Vektordesigns zur Erhöhung der Fluoreszenzintensität (*FI*)*,* der Rekombinations-Austausch-Raten (*Rate*) und zur Erweiterung des Spektrums an nachweisbarer genetischen Veränderungen *(Typ)* und damit zur Sensitivitätssteigerung der Gentoxizitäts-Messung, wie in Kapitel 3 beschrieben, zusammengefaßt. Die Beschriftung der einzelnen Sequenz-Elemente sind jeweils nur für das erste Konstrukt angegeben und erklären sich wie folgt: Puromycin-Resistenzgen (Puro), Gal4ER-VP responsiver Promotor *(GREs),* kodierende Region für die I-*Sce*I-Meganuklease (*I-SceI*), im 3' - Bereich verkürztes *EGFP*-Gen (*N'-EGFP*), CMV-Promotor (*CMV*), Wildtyp-*EGFP*-Gen (EGFP), Hygromycin-Resistenzgen (*hygro*), SV40-Promotor *(SV40),* EGFP-Gen mit Unsinnmutation im Startkodon (met->stop), *DsRed-*Gen (Red), Vektorrückgrat des murinen Moloney Leukämievirus (*MoMuLV*)*,* revers von Tetrazyklin kontrollierter Transaktivator (rtTA), rtTA responsiver Promotor (*TRE*)*.* Die Bezeichnungen Δ*EGFP*/*HR*/*EJ* deuten an, daß das dargestellte Konstrukt jeweils in den drei Varianten mit entweder *ΔEGFP, EGFP-HR* oder *EGFP-EJ* an der angezeigten Position kreiiert wurde. Einige Typen von genetischen Veränderungen wurden abgekürzt mit *kHR* für konservative homologe Rekombination, *EJ* für *End Joining, nkHR* für nichtkonservative homologe Rekombination. Pfeile geben die Lesrichtung der kodierenden Sequenzen an.

### 4. Vorteile der Erfindung:

Dieses neuartige Testsystem für die Bestimmung von Gentoxizitäten überwindet alle Nachteile bisheriger Nachweissysteme: DNA-Schädigung und Nachweis erfolgen direkt in Säugerzellen, nicht in Mikroorganismen. Das Nachweissystem ist auf lebende Tiere übertragbar. Das Spektrum möglicher DNA-Schädigungen durch Mutagene ist durch die Erfassung vielfältigster rekombinativer Reparatur-Prozesse sowie von Punktmutationen und insbesondere auch jeder Art von anderweitigen Gen-inaktivierenden Ereignissen maximal breit. Die Sensitivität des Systems wurde durch den optimierten Einsatz von stark autofluoreszierenden Proteinen als Signale und die Erzeugung langer Sequenzhomologien maximiert. Die einfache und schnelle Durchführung der Tests schafft die Voraussetzung für routinemäßige Analysen im Großmaßstab.

### 5. Beispiele:

### Beispiel 1: Klonierung von p5-Puro-CMV-(N'-EGFP)-CMV-Red-(EGFP-EJ).

Repräsentativ für die Vektoren in Abb. 5 wird hier die Klonierung für das Konstrukt p5-Puro-CMV-(N'-EGFP)-CMV-Red-(EGFP-EJ) (siehe Abb. 5i und Kapitel 6 Sequenzprotokoll) beschrieben. Als Grundlage diente der retrovirale Vektor p5NM von Frau Dr. Carol Stocking, Heinrich-Pette-Institut Hamburg, welcher zur Familie der MPSV-Vektoren gehört (Laker, C. et al. (1987) Proc. Natl. Acad. Sci. USA 84: 8458-8462). Restriktionsenzyme, Rapid DNA Ligation Kit**^{®},** Klenow-Enzym, T4-Polymerase und Shrimp Alkaline Phosphatase (SAP) wurden von den Firmen New England Biolabs, Schwalbach/Taunus, Roche, Mannheim, und MBI Fermentas, St. Leon-Rot, bezogen. Bakterien-Transformationen zur Klonierung wurden mit den Bakterienstämmen XL10Gold^{®}, SURE^{®} oder SCS110 von Stratagene, Heidelberg, nach Standardmethoden durchgeführt (Ausubel, F.M. et al. (2000) Current Protocols in Molecular Biology, John Wiley and Sons). Glättung von DNA-Enden mittels Klenow-Enzym oder T4-Polymerase und DNA-Gelelektrophorese wurden ebenfalls nach diesen Standardmethoden durchgeführt. Plasmid-Präparationen aus Bakterien-Extrakten wurden gemäß der Anleitung des Qiaprep Miniprep Kits von Qiagen, Hilden, oder mit dem Anionenaustauscher-Säulen Nucleobond^{®} AX Kit von Macherey-Nagel, Düren, nach Angaben des Herstellers durchgeführt. Dephosphorylierung von DNA-Enden mittels SAP wurde nach der Vorschrift von Roche, Mannheim, praktiziert. DNA-Fragmente wurden nach Agarose-Gelelektrophorese mit Hilfe des QIAEX II Gel Extraction Kits von Qiagen, Hilden, aufgereinigt.

Vorbereitend für die finalen Klonierungen innerhalb des retroviralen Vektors p5NM wurde zunächst ein Fragment aus der Expressions-Kassette für das *DsRed*-Gen hergestellt. Hierzu wurde der Vektor pDsRed1-N1 (Clontech, Palo Alto, CA, USA) mit den Restriktionsenzymen HindIII und AgeI geschnitten, die DNA-Enden mittels Klenow-Enzym geglättet und anschließend mittels Rapid DNA Ligation Kit^{®} religiert, um die multiple Klonierungsstelle zwischen dem CMV-Promotor und dem *DsRed-Gen* zu entfernen. Um die Methylierung der plasmidalen DNA in Bakterien zu unterdrücken, und um anschließend auch an der in pDsRedl-N1 einmaligen XbaI-Erkennungssequenz schneiden zu können, wurde die Plasmid-DNA im methylierungsdefizienten Bakterienstamm SCS110 (Stratagene) amplifiziert. Das unmethylierte Plasmid wurde mit dem Restriktionsenzym AseI verdaut, die Enden durch eine Auffüllreaktion mit Klenow-Enzym geglättet und das Fragment bestehend aus dem CMV-Promotor und dem nachgeschaltetem *DsRed*-Gen durch *Xba*I ausgeschnitten. Das Fragment wurde mit Hilfe des QIAEX II Gel Extraction Kits von Qiagen, Hilden, von restlichen Vektorsequenzen getrennt. Nach der Isolierung wurde das Fragment in den Vektor pUC18 (MBI Fermentas, St. Leon-Rot) subkloniert, nachdem dieser mit den Enzymen SmaI und *Xba*I verdaut und beide Enden mittels SAP dephosphoryliert worden waren. Der resultierende Vektor pUC-CMV-Red wurde mit dem Restriktionsenzym NotI verdaut, mit Klenow-Enzym behandelt, mit X*ba*I nachverdaut und dephosphoryliert. Aus pCMV- (EGFP-EJ) (siehe Beispiel 2) wurde das Fragment CMV-(EGFP-EJ) ausgeschnitten, indem es mit XhoI geschnitten und mit Klenow-Enzym behandelt, mit *Xba*I nachverdaut und mit dem *Not*I/*Xba*I-gespaltenen pUC-CMV-Red ligiert wurde. Bei pCMV-(EGFP-EJ) handelt es sich um pEGFP-N1 von Clontech, in welchem der Sequenz-Bereich kodierend für Chromophor-Aminosäuren des EGFP-Gens dergestalt mutiert worden waren, daß eine I-*Sce*I-Erkennungsstelle und kurze Sequenz-Wiederholungen 5' und 3' von dieser Erkennungsstelle kreiiert wurden, so daß nicht nur homologe Rekombination mit einer zweiten *EGFP*-Gen-Variante sondern auch *End Joining* über diese Wiederholungen nach I-*Sce*I-Verdau ablaufen kann. Ein aus der Ligation resultierendes Plasmid pUC-CMV-Red-(EGFP-EJ), in welchem DsRed und *EGFP-EJ* die gleiche Lesrichtung aufwiesen, wurde mit *Eco*RI und *Sal*I geschnitten. Das CMV-Red-(EGFP-EJ)-Fragment wurde mit gleichermaßen verdautem und dephosphoryliertem pSNM-Vektor ligiert und ergab p5-CMV-Red-(EGFP-EJ).

Parallel dazu wurde das Puromycin-Resistenzgen aus dem Clontech-Vektor pRetroOn über die Enzyme NaeI und *Eco*RI in pUC18 nach *Sma*I*-* und EcoRI-Restriktionsverdau kloniert (pUC-Puro). Anschließend wurde das DEGFP-PCR-Fragment (siehe Beispiel 2) in den Leserahmen des Puromycin-Resistenzgens inseriert, indem es über die Restriktionsschnittstellen *Sex*AI und *Eco*RI in den pUC-Puro-Vektor kloniert wurde (pUC-Puro-ΔEGFP). Im nächsten Schritt wurden die über PCR künstlich in *ΔEGFP* eingeführten EcoRI-Schnittstellen am 3'-Ende und im zentral mutierten Bereich genutzt, um die 3'Hälfte des Gens auszuschneiden. Dieses Fragment wurde über die dem Puromycin-Resistenzgen nachgeschaltete *Eco*RI-Schnittstelle in einen pUC-Puro-Vektor dergestalt kloniert, daß die Lesrichtung des Puromycin-Resistenzgens und des halben Δ*EGFP*-Gens die gleiche Orientierung aufwiesen. Der resultierende pUC-Puro-AEGFP1/2-Vektor enthielt nun eine künstliche *Not*I-Schnittstelle am 3'Ende der Δ*EGFP*-Hälfte. pUC-Puro-ΔEGFP1/2 wurde mit *Nsi*I geschnitten und die DNA-Enden mittels T4-Polymerase-Behandlung geglättet. Anschließend wurde pUC-Puro-ΔEGFP1/2 von der 3'Hälfte des *ΔEGFP* -Gens durch einen zweiten Restriktionsverdau in der synthetischen NotI-Restriktionserkennungsequenz befreit und durch ein CMV-(EGFP-EJ)-Fragment ersetzt. Hierfür wurde der Vektor pCMV-(EGFP-EJ) (siehe Beispiel 2) mit AseI geschnitten, mit Klenow-Enzym die DNA-Enden aufgefüllt, mit *Not*I nachgeschnitten und mit dem pUC-Puro-Vektor-Fragment ligiert. So entstand der Vektor pUC-Puro-CMV-(EGFP-EJ).

Das am 3'-Ende verkürzte *EGFP*-Gen-Fragment *N'-EGFP* wurde aus pEGFP-N1 über Restriktionsverdaue mit GsuI und AgeI ausgeschnitten. *N'-EGFP* fehlen 277 Basenpaare am 3' Ende des kodierenden Bereiches vom EGFP-Gen. Nach Glättung der DNA-Enden über T4-Polymerase wurde das N'-EGFP-Fragment in die multiple Klonierungsschnittstelle des Vektors p5NM durch Ligation eingesetzt, indem dieser mit HindIII geschnitten, mit Klenow-Enzym an den Enden geglättet und dephosphoryliert wurde (pSNM-(N' -EGFP)).

In dem Plasmid pUC-Puro-CMV-(EGFP-EJ) wurde die für EGFP-EJ kodierende Region über *Not*I- und *Xho*I-Restriktionsverdaue entfernt, die verbleibenden Enden mittels Klenow-Enzym geglättet und dephosphoryliert. In gleicher Orientierung wie das ursprüngliche *EGFP-EJ*-Gen wurde nun das *N'EGFP-*Gen inseriert, indem es per *Eco*RI- und SalI-Verdau aus p5NM-(N' - EGFP) ausgeschnitten wurde, an den Enden per Klenow-Enzym geglättet und mit dem pUC-Puro-CMV-Vektor-Fragment ligiert wurde. Aus dem resultierenden Vektor pUC-Puro-CMV-N'EGFP schließlich konnte das Fragment Puro-CMV-N'EGFP als *Bam*HI-Fragment in den anfänglich bereitgestellten Vektor p5-CMV-Red-(EGFP-EJ) nach dessen *Bam*HI-Verdau und Dephosphorylierung eingebaut werden (beide EGFP-Gen-Varianten in gleicher Orientierung). So wurde das Endkonstrukt p5-Puro-CMV-(N'-EGFP)-CMV-Red-(EGFP-EJ) erzeugt. Im Anschluß an die Klonierung wurde das Rekombinations-Konstrukt in den nicht über Antibiotika-Resistenz oder rote Autofluoreszenz überprüfbaren Abschnitten sequenziert: *N*'-*EGFP* mit *Primern* EGFPwt-1 und EGFPSeq1, *EGFP-EJ* mit *Primer* EGFPSeq3 und der *N'EGFP* vorgeschaltete CMV-Promotor. Für die Sequenz-Analyse wurde der Reaktionskit ABI PRISM^{™} Big Dye Ready Reaction Cycle Sequencing Kit von der Firma PE Applied Biosystems, Weiterstadt, nach den Angaben des Herstellers verwendet. Die elektrophoretische Auftrennung und automatische Analyse der Absorptionsmaxima wurde an einem ABi 377-96 Sequenzierautomat mit zugehöriger Basecaller Software durchgeführt.

Sequenzier-Primer:
EGFPwt-1:GTGACCACCCTGACCTAC
EGFPSeqI:GCAGCACGACTTCTTCAAGT
EGFPSeq3:GTTGTGGCTGTTGTAGTTGTA

Das Plasmid "p5-Puro-CMV-(N'-EGFP)-CMV-Red-(EGFP-EJ)" wurde am 14. November 2000 bei der Deutschen Sammlung von Mikroorganismen und Zellkulturen (DSMZ), Mascheroder Weg 1b, 38124 Braunschweig, Deutschland, unter der Eingangsnummer DSM 13848 nach dem Budapester Vertrag hinterlegt.

### Beispiel 2: Generierung der ΔEGFP- sowie EGFP-EJ-Gene durch PCR-Mutagenese.

Repräsentativ für die verschiedenen Mutationen im für die Chromophor-Aminosäuren kodierenden Sequenz-Bereich innerhalb des EGFP-Gens werden hier die Herstellung des Δ*EGFP*-Gens und des *EGFP-EJ-*Gens beschrieben.

*ΔEGFP* wurde über stufenweise PCR-Reaktionen erzeugt. Die terminalen PCR-*Primer* (EGFP-13 und -15) für das komplette *ΔEGFP*-PCR-Fragment führten synthetische Restriktionsschnittstellen in das Δ*EGFP*-Gen ein, nämlich für *Sex*AI am 5'Ende und *Bam*HI und *Eco*RI am 3'Ende, so daß diese Schnittstellen für die anschließende Klonierung in den Leserahmen des Puromycin-Resistenzgens in pUC-Puro zur Verfügung standen (siehe Beispiel 1). Die im Bereich der Chromophor-Aminosäuren mutierte Region wurde erzeugt, indem zunächst die Sequenz-Bereiche 5' und 3' der Mutation im *ΔEGFP-*Gen über 2 voneinander unabhängige PCR-Reaktionen erzeugt wurden. Die gewünschten PCR-Produkte wurden nach Agarose-Gelelektrophorese mit Hilfe des QIAEX-Kits gereinigt. Anschließend wurden die beiden PCR-Fragmente über die synthetischen Enden mit der Mutation über eine verknüpfende PCR verbunden. In *△EGFP* werden so die für die Chromophor-Aminosäuren 65-67 kodierenden Basenpaare, und 29 weitere Basenpaare im 5'-Bereich und 7 Basenpaare im 3'-Bereich des *EGFP*-Gens durch die Erkennungssequenz der Meganuklease I-*Sce*I und der Restriktions-Endonuklease *Eco*RI ersetzt (siehe Abb. 4).

| Reaktionsgemisch 1: |
|---|
| 100 ng pEGFP-N1 |
| 50 pmol *Primer* EGFP-14 |
| 50 pmol *Primer* EGFP-15 |
| 200 µM dNTP |
| 10 µl 10x Taq-Polymerase-Puffer (Qiagen) |
| 10 µl Q-Solution (Qiagen) |
| 1 µl Taq DNA Polymerase (Qiagen) |
| ad 100 µl H₂O |

| Reaktionsgemisch 2: |
|---|
| wie Reaktionsgemisch 1, aber als *Primer:* |
| 50 pmol *Primer* EGFP-11 |
| 50 pmol *Primer* EGFP-13 |

| Reaktionsgemisch 3 (verknüpfend): |
|---|
| Gereinigte PCR-Produkte der Reaktionen 1 und 2 |
| 50 pmol *Primer* EGFP-13 |
| 50 pmol *Primer* EGFP-15 |
| 200 µM dNTP |
| 10 µl 10x Taq-Polymerase-Puffer (Qiagen) |
| 20 µl Q-Solution (Qiagen) |
| 1 µl Taq-Polymerase (Qiagen) |
| ad 100 µl H₂O |

Das Reaktionsgemisch wurde jeweils zunächst bei 92 °C für 4 min inkubiert. Anschließend wurden 35 Zyklen unter folgenden Bedingungen durchgeführt:
92 °C 60 s
60 °C 60 s
72 °C 120 s
Anschließend wurde das Reaktionsgemisch bei 72 °C für 4 min inkubiert.

Das entstehende ΔEGFP-PCR-Fragment wurde nach Restriktionsverdau mit *Sex*AI und *Eco*RI in das ebenso verdaute und dephosphorylierte Plasmid pUC-Puro inseriert (siehe Beispiel 1).

*EGFP-EJ* wurde wie *△EGFP* über stufenweise PCR-Reaktionen erzeugt. *EGFP-EJ* enthält im EGFP-Sequenz-Bereich, welcher für Chromophor-Aminosäuren kodiert, eine I-SceI-Erkennungssequenz als Insertion (siehe Abb. 4). Darüberhinaus wurden 5 Basenpaare der *EGFP-*Sequenz verdoppelt, so daß 5' und 3' von der I-SceI-Erkennungssequenz 5 Basenpaare lange Homologien existieren. Diese kurzen Homologien dienen beim EJ nach Einführung eines DSBes durch I-SceI zur Rekonstitution der Wildtyp-*EGP*-Sequenz.

Im Unterschied zur Erzeugung von *ΔEGFP* wurden in den Reaktionsgemischen 1 und 2 die Primer-Paare EGFP-EJ-1 und EGFP-13 bzw. EGFP-EJ-2B und EGFP-15 verwendet. Das entstehende EGFP-EJ-PCR-Fragment wurde nach QIAEX-Reinigung und Restriktionsverdau mit *Sex*AI und *Eco*RI in das ebenso verdaute und dephosphorylierte Plasmid pUC-Puro inseriert (siehe Beispiel 1). Zur Erzeugung von pCMV-(EGFP-EJ) (siehe Beispiel 1) wurde der resultierende Vektor pUC-Puro-(EGFP-EJ) mit *Sex*AI verdaut, die DNA-Enden mit Klenow-Enzym geglättet, und das EGFP-EJ-Fragment über NotI-Nachverdau gewonnen. Das gereinigte Fragment wurde in das Plasmid pEGFP-N1 anstelle des EGFP-Gens in der gleichen Orientierung inseriert, nachdem dieses mit HindIII geschnitten, mit Klenow-Enzym behandelt und mit NotI nachgeschnitten worden war. Das aus der Ligation resultierende Plasmid pCMV-(EGFP-EJ) wurde für weitere Klonierungen eingesetzt und, falls nötig, im methylierungsdefizienten Bakterienstamm SCS110 amplifiziert (siehe Beispiel 1).

Alle über PCR generierten DNA-Fragmente wurden nach Klonierung sequenziert (siehe Beispiel 1).

### Primer:

| | |
|---|---|
| EGFP-11: | GTTCATCTGCACCACCGGCAAGCTGCCCGGAATTCTAGGGATAACA GGGTAATGCTTCAGCCGCTACCCCGAC |
| EGFP-13: | AGGAGGGAATTCGGATCCGCGGCCGCTTTACTTGTACAGCT |
| EGFP-14: | TCCCTAGAATTCCGGGCAGCTTGCCGGTGGTGC |
| EGFP-15: | CGCGCGACCTGGTGCATGACCCGCAAGCCCGGTGCCATGGTGAGC AAGGGCGAGGAGC |
| EGFP-EJ-1: | TGACCTACTAGGGATAACAGGGTAATCCTACGGCGTG |
| EGFP-EJ-2B: | CACGCCGTAGGATTACCCTGTTATCCCTAGTAGGTCAGGGTGGTCACGA |

### Beispiel 3: Analyse von KMV-Einzelzell-Klonen mit genomischem pGC-Sce-Integrat auf I-SceI-Expression nach Östradiol-Gabe.

KMV-Zellen repräsentieren K562-Leukämie-Zellen (Andersson, L.C. et al. (1979) Int. J. Cancer 23: 143-147), welche den durch Östradiol-Gabe aktivierbaren Transkriptionsfaktor GalERVP exprimieren (Braselmann, S. et al. (1993) Proc. Natl. Acad. Sci. USA 90: 1657-1661). Es wurden nach Elektroporation mit dem Plasmid pGC-Sce KMV-Klone mit dem ins Genom integrierten Konstrukt isoliert (siehe Beispiel 6). Diese wurden in Zellkulturflaschen für Gewebe von Nunc^{™}, Wiesbaden, kultiviert. Als Kulturmedium wurde RPMI ohne Phenolrot (Gibco/BRL, Eggenstein) mit 10 % FCS ohne Steroidhormone (Promocell) verwendet. Die Inkubation der Zellen erfolgte bei 37 °C und 5 % CO₂ im Brutschrank.

Zur Aktivierung von GalERVP und damit indirekt dem Promotor in pGC-Sce über die Gal4-Erkennungssequenzen vor der für I*-Sce*I kodierenden Sequenz wurden anfänglich 2 µM und nach der Bestimmung der kleinsten Konzentration (50 nM) für vollständige Promotor-Induktion jedoch nur 200 nM β-Östradiol (ICN Biomedicals GmbH, Meckenheim) ins Medium gegeben und 4-24 h kultiviert. Anschließend wurden die Zellen zentrifugal sedimentiert (1000 x g, 5 min, Raumtemperatur), 1 x mit PBS (140 mM NaCl; 3 mM KCl ; 8 mM Na₂HPO₄ ; 1, 5 mM KH₂PO₄; pH 7,4) gewaschen und je 5 x 10⁶ Zellen in 50 µl Probenpuffer (65 mM Tris/HCl, pH 6,8; 10 % Glyzerin; 2,3 % SDS; 5 % ß-Mercaptoethanol (frisch zugesetzt); 0,05 % Bromphenolblau) suspendiert und 5 min aufgekocht. KMV-Zellen, welche transient mit *pCMV-Sce,* einem konstitutiv I-SceI exprimierenden Plasmid, elektroporiert worden waren (siehe Beispiel 4), dienten als Positivkontrolle.

Anschließend wurden die Proteine aus 1-2 x 10⁶ Zellen durch diskontinuierliche Gelektrophorese in einem 15 %igen SDS-Polyacrylamidgel aufgetrennt (Lämmli, U.K. (1970) Nature 227: 680-685). Als Molekulargewichtsstandard wurde ein Gemisch von Proteinen definierter Molekülmasse verwendet (Sigma, München). Die Auftrennung erfolgte bei einer Stromstärke von 25 mA/Gel. Anschließend wurden die Proteine aus dem Gel auf eine Polyvinylidenfluorid-Membran (Immobilon-P, Millipore) elektrotransferiert (Towbin, H. (1979) Proc. Natl. Acad. Sci. USA 76: 4350-4354). Der Transfer erfolgte in einer Hoefer-Naßblotapparatur gefüllt mit Tris-Glycin-Puffer (192 mM Glyzin; 50 mM Tris/HCl, pH 8,3) unter Eiskühlung bei 100 V für 60 min. Anschließend wurde die Membran 60 min in TBS-T-Puffer (20 mM Tris/HCl, pH 7,6; 137 mM NaCl; 0,2 % Tween 20) mit 5 % Magermilchpulver geschwenkt, um unspezifische Antikörperbindungsstellen abzusättigen. Zum Nachweis der von Östradiol abhängigen Expression der I-Scel-Meganuklease wurde die Tatsache ausgenutzt, daß dieses vom Konstrukt pGC-Sce in Fusion mit dem Peptid-Epitop des anti-Haemagglutinin (HA)-Antikörpers 12CA5 exprimiert wird. Deswegen wurde die Membran mit einer geeigneten Verdünnung des Antikörpers 12CA5 (1/20-1/50 fach verdünnter Hybridomazell-Kulturüberstand) in TBS-T-Puffer mit 5 % Magermilchpulver über Nacht bei 4 °C geschwenkt. Die Membran wurde dann 4 x je 15 min mit TBS-T-Puffer gewaschen und mit dem sekundären Antikörper (10⁻⁵ mg/ml) in TBS-T-Puffer mit 5 % Magermilchpulver 60 min inkubiert. Die Membran wurde dann 3 x für je 15 min mit TBS-T-Puffer gewaschen. Der sekundäre Antikörper, der an den FC-Teil des primären Antikörpers bindet, ist mit einer Peroxidase konjugiert. Diese katalysiert die chemilumineszenzreaktion zum Antigen-Nachweis, nämlich die Oxidation von Luminol durch Wasserstoffperoxid. Das angeregte Luminolmolekül sendet Licht der Wellenlänge λ = 428 nm aus, welches auf einem Röntgenfilm eine Schwärzung hervorruft. Diese Chemilumineszenzreaktion wurde mit einer Super-Signal^{®} ULTRA Chemilumineszenz-Lösung von Pierce (Illinois, USA) nach Anleitung des Herstellers ausgelöst, auf die abgetropfte Membran ein Biomax-MR Röntgenfilm aufgelegt und der Film nach Exposition entwickelt.

### Beispiel 4: Messung von spontanen, durch I-SceI-Expression verstärkten oder durch Noxen induzierten Rekombinations-Häufigkeiten durch EGFP-Fluoreszenz-Nachweis.

Beim transienten Rekombinations-Assay wurden die DNA-Substrate durch das physikalische Transfektions-Verfahren der Elektroporation in die Zellen eingeführt. Bei diesem Verfahren werden die Poren der Zellmembran durch den kurzzeitigen Aufbau eines elektrischen Feldes depolarisiert und dadurch für DNA durchgängig gemacht. Da es sich bei den Substraten nicht um episomal in Säugern replizierende Vektoren handelt, dünnen sich diese im Laufe der Tage nach Transfektion aus, d.h. der *Assay* ist transient.

Die Elektroporation von PA317-, MethA-, HL60-, WTK1-, K562- und abgeleiteten Zellen wurde wie von Baum und Kollegen beschrieben durchgeführt (Baum, C. et al. (1994) Biotechniques 17: 1058-1062). 2-5 x 10⁶ PA317- oder MethA- bzw. 10⁷ Zellen der restlichen Zelltypen wurden durch Trypsinierung (Zellen aus Adhäsions-Kultur) bzw. durch Zentrifugation (Zellen aus Suspensions-Kultur) geerntet. Das Zellpellet wurde in 400 µl DMEM- bzw. RPMI-Medium mit 10 % FCS resuspendiert und in einer Elektroporationsküvette (Biorad, München) mit 20 µg DNA vermischt. Die Elektroporation erfolgte bei 240 V und 1050 mF. Sofort danach wurden die elektroporierten Zellen in eine Zellkulturschale oder Zellkulturflasche überführt und im Brutschrank inkubiert (siehe Beispiel 3).

Wenn die verwendeten Zellen oder das eingesetzte Rekombinations-Konstrukt eine I-SceI-Expression in Abhängigkeit von Östradiol ermöglichten, wurden die elektroporierten Zellen aufgeteilt und zur Analyse von DNA-Rearrangements nach Einführung eines DSBes 200 nM ß-Östradiol (ICN Biomedicals GmbH, Meckenheim) unmittelbar nach der Elektroporation ins Kulturmedium einer Hälfte der Ansätze gegeben. Für Zellen oder Rekombinations-Konstrukte ohne diese Möglichkeit wurden spontane Rekombinations-Prozesse von denen nach I-*Sce*I-Verdau unterschieden, indem die Zellen mit einer Mischung aus 10 µg DNA-Rekombinations-Substrat und 10 µg pBlueScript M13-DNA von Stratagene, San Diego, USA (spontan) bzw. 10 µg DNA-Rekombinations-Substrat und 10 µg pCMV-Sce-DNA (I-*Sce*Iinduziert) elektroporiert wurde. Das Plasmid pCMV-Sce erlaubt die konstitutive Expression der I-*Sce*I-Meganuklease mit Hilfe des extrem starken CMV-Promotor in Säugerzellen.

Zum quantitativen Nachweis grün oder rot fluoreszierender Zellen wurden 2 x 10⁶ Zellen zu verschiedenen Zeiten (4-96 h nach Elektroporation) durch Trypsinierung bzw. durch Zentrifugation geerntet, 1 x mit PBS mit 0,2 % EDTA gewaschen und in 2 ml PBS mit 0,2 % EDTA resuspendiert. Anschließend wurden je 30000 Zellen am Coulter Counter EPICS XL-MCL™ FACS-Scan Cytometer oder am Calibur von Becton Dickinson analysiert. Die quantitative Auswertung der leuchtenden im Vergleich zu nicht leuchtenden Zellen wurde mittels der SystemII™ Software durchgeführt. Neben der Analyse über FACS-Scan Cytometrie waren grün oder rot leuchtende Zellen auch am Immunfluoreszenzmikroskop (Zeiss Axiovert 35) mit den entsprechenden Filtersätzen darstellbar. Das Laser Scanning Cytometer von Compucyte repräsentiert das optimale Werkzeug zur Quantifizierung von fluoreszierenden Zellen in einer Population nicht fluoreszierender Zellen, da dieses Gerät die Vorteile der Einzelzellanalyse am Fluoreszenzmikroskop und der Sortierung am Cytometer vereinigt.

Bei der Messung von spontanen Rekombinations-Häufigkeiten in Zellen mit genomisch integriertem Rekombinations-Konstrukt (siehe Beispiel 6) durch EGFP-Fluoreszenz-Nachweis blieben die Zellen entweder unbehandelt oder wurden mit 10 µg pBluescript M13-DNA elektroporiert. Zur Bestimmung der Antworten auf bestimmte Noxen wurden die Zellen mit einer Dosis von 500 Rad ionisierender Strahlung (¹³⁷Cs-Strahlungsquelle), oder mit 100 µM Etoposid (Sigma) im Medium für 2 h behandelt. Bei der Messung von DNA-Rearrangements nach I-SceI-Expression wurde die Meganuklease durch Elektroporation mit 10 µg des Plasmides pCMV-Sce in die Zellen eingeführt. Fluoreszenzanalysen wurden wie für den transienten *Assay* durchgeführt.

### Beispiel 5: Generierung einer Retroviren produzierenden Helferzellinie.

Retrovirale Verpackungszellinien bieten die Möglichkeit, aus replikationsdefekten Virengenomen intakte Retroviren-Partikel herzustellen, da die für die Replikation und die Verpackung benötigten Proteine *(gag-, pro-, pol-,* env-Genprodukte) von der Verpackungszellinie produziert werden (Coffin, J.M. (1996) in Fundamental Virology, Fields, B.N., Knipe, D.M. und Howley, P.M. (eds.), Lippincott-Raven Publishers, Philadelphia, 763-844). Zur Verpackung von Retrovirengenomen in diesen Verpackungszellinien benötigen retrovirale Vektoren einen 5'LTR mit *Primer*-Bindungsstelle, einen 3'LTR und ein Verpackungssignal. Darüberhinaus können Fremdsequenzen zwischen den LTRs über die resultierenden transgenen Retroviren bis zu einer Gesamtgenom-Größe von insgesamt 10 kiloBasenpaaren Länge transduziert werden. Replikationsdefiziente Retroviren aus Verpackungszellinien können Zellen infizieren, jedoch keine neuen Virionen bilden. Für die Erzeugung von Retroviren zur Transduktion der Rekombinations-Konstrukte wurde die murine Verpackungszellinie PA317 verwendet, welche amphotrophe Retroviren produzieren kann, d.h. sie trägt alle Gene zur Verpackung von replikationsdefizienten Retroviren, welche Zellen von Maus, Mensch, Huhn, Hund und Katze infizieren können (Miller, A.D. und Buttimore, C. (1986) Mol. Cell. Biol. 6: 2895-2902).

PA317-Zellen wurden in Adhäsions-Kultur in Zellkulturschalen für Gewebe von Nunc^{™}, Wiesbaden, kultiviert. Als Kulturmedium wurde DMEM (Gibco/BRL, Eggenstein) mit 10 % FCS (Boehringer, Mannheim) verwendet. Die Inkubation der Zellen erfolgte bei 37 °C und 5 % CO₂ im Brutschrank. Zur Passagierung wurden die Zellen nach Absaugen des Kulturmediums mit Trypsin-Lösung (0,05 % Trypsin; 1 x PBS; 0,5 mM EDTA, pH 8,0 von Gibco/BRL, Eggenstein) gewaschen, ca. 5 min inkubiert und die abgelösten Zellen in Kulturmedium überführt. Die Rekombinations-Konstrukte, welche alle auf der Basis von retroviralen Vektoren generiert worden waren, wurden durch Elektroporation in PA317-Zellen eingeführt (Elektroporations-Methode wie in Beispiel 4). Die als bevorzugte Ausführform in dieser Erfindung beschriebenen Konstrukte tragen jeweils eine Expressions-Kassette für Puromycin-Resistenz, welche zur Selektion von transgenen Klonen verwendet werden konnte. Nach der Elektroporation mit dem jeweiligen Konstrukt wurden Zellen für 2 d zur Erholung in Normalmedium gezüchtet. Danach wurde das Kulturmedium für 14 d auf eine Puromycin-Konzentration von 4,5 µg/ml eingestellt,
wobei das Medium alle 2-3 d gewechselt wurde und der Großteil der Zellen abstarben. Nach 14 d konnten Einzelzell-Klone von mindestens 100 Zellen am Boden der Zellkulturschale beobachtet werden. Zu diesem Zeitpunkt wurde das Medium abgesaugt und mit Trypsin-Lösung gewaschen. Anschließend wurde jeweils um den gewünschten Klon ein steriler Klonierungszylinder aus Borosilikatglas mittels sterilem Silikonfett befestigt. In diesen Ring wurden 100 µl Trypsin-Lösung gegeben und nach wenigen Minuten die abgelösten Zellen über eine Pipette aufgesogen und in je einem Loch einer 96- oder 24-Loch-Platte angezüchtet. Nach Vermehrung der Zellen wurde die Integrität des Konstruktes nach Zufalls-Integration ins zelluläre Genom über PCR-Analyse geprüft (siehe Beispiel 7). Für transgene PA317-Klone mit korrektem Integrat wurde die Freisetzung infektiöser Virenpartikel in den Kulturüberstand getestet, indem murine MethA- oder menschliche K562-Zellen infiziert und auf das Entstehen gegenüber Puromycin resistenter Zell-Klone getestet wurden (siehe Beispiel 6). Nach Erreichen einer Zellzahl von ca. 6 x 10⁶ Zellen wurden diese sofort in flüssigem Stickstoff zur Langzeitlagerung weggefroren, da der Virustiter mit der Dauer der Kulturhaltung abnimmt.

### Beispiel 6: Infektion von K562-Zellen mit Retroviren und Isolierung infizierter Klone.

Da es sich bei K562-Zellen um Zellen handelt, welche in Suspensionskultur wachsen, müssen die Zellen im Gegensatz zu adhäsiv wachsenden Zellen vor der Infektion mit Retroviren konzentriert werden. Aus diesem Grunde wird die Methode der retroviralen Infektion repräsentativ für K562-Zellen beschrieben.

Für die Infektion wurden K562-Zellen in 20 ml RPMI-Medium in Kultur gehalten (siehe Beispiel 3) bis eine Gesamtzahl von 1-2 x 10⁷ exponentiell wachsende Zellen in einer Gewebekulturflasche mit 80 cm² Grundfläche (Nunc^{™}, Wiesbaden) erreicht war. Parallel dazu wurden Zellen des PA317-Klones mit dem ins Genom integrierten Konstrukt von Interesse unmittelbar nach Klonierung oder nach Auftau angezüchtet (siehe Beispiel 5). Das Medium wurde gewechselt und 24 h später als Virus-Überstand abgenommen und sterilfiltriert (0,45 µm). Die angewachsenen K562-Zellen wurden durch Zentrifugation (1000 x g, 5 min, Raumtemperatur) geerntet und das Zellpellet in 10 ml Virus-Überstand zur Infektion oder in DMEM-Medium mit 10 % FCS als Negativkontrolle resuspendiert. Das Gemisch wurde konzentriert in einer Gewebekultürflasche mit 25 cm² Grundfläche für 1 h inkubiert und anschließend 1 h bei 1000 x g und Raumtemperatur zentrifugiert, um die Infektionsrate zu erhöhen. Anschließend wurden die Zellen für 1 d inkubiert und in eine Gewebekulturflasche mit 80 cm² Grundfläche umgesetzt.

Erfolgreich infizierte Zellen konnten aufgrund des über den Retrovirus transduzierten Puromycin-Resistenz-Gens als Einzelzell-Klone in Softagar mit Puromycin isoliert werden. Die Klonierung von Einzelzell-Klonen in Softagar wurde in gleicher Weise für K562- oder WTK1-Zellen nach Elektroporation mit DNA durchgeführt. Hierfür wurden die infizierten K562-Zellen nach 2 d Erholung aus der Gewebekulturflasche (100 ml) in Softagarkulturen (je 3 ml) aus RPMI-Medium; 200 mM Glutamin; 1 % Natriumpyruvat; 12 % FCS; 0,33 % Bactoagar (Difco, Detroit, USA) mit Puromycin (0,25 µg/ml für K562 und 0,1 µg/ml für WTK1) plattiert. Die Zellzahl lag bei 1,5 x 10⁵ pro Loch innerhalb einer 6-Lochplatte von Nunc^{™}, Wiesbaden. Nach 10 Tagen Inkubation im Brutschrank konnten Einzelzell-Klone mit einer Pasteurpipette isoliert werden und in 96- oder 24-Loch-Platten in Flüssigmedium angezüchtet werden. Nach Vermehrung der Zellen wurde die Integrität des Provirus über genomische PCR-Analyse geprüft (siehe Beispiel 7).

### Beispiel 7: Prüfung der Integrität eines Rekombinations-Konstruktes nach stabiler Integration ins Genom.

Nach der genomischen Integration wird die Retroviren-DNA, der sogenannte Provirus, von den LTRs umrahmt. Die Integration ins Genom ist zufällig, verläuft aber im Normalfall unter Beibehaltung der Genanordnung. Um jedoch Rearrangements im Verlauf des retroviralen Infektionszyklus ausschließen zu können, wurden die Proviren in infizierten Zellen, welche gegenüber Puromycin Resistenz aufweisen, bzgl. der Integrität der Rekombinations-Marker analysiert. Diese Kontrolle war insbesondere zur Auswahl potentiell Retroviren produzierender PA317-Klone nötig, um sicherzustellen, daß nach Elektroporation der PA317-Zellen und anschließender Zufallsintegration des jeweiligen Konstruktes ins Genom die über Retroviren zu transduzierenden Sequenzen unverändert geblieben waren.

Genomische DNA wurde für den jeweiligen Zelltyp aus ca. 5 x 10⁶ Zellen nach Anleitung des Herstellers des QIAamp DNA Mini kits, Qiagen, Hilden, gewonnen. Mit einem konstanten volumenäquivalent von 2 µl des genomischen DNA-Isolats wurden jeweils PCR-Reaktionen zum Nachweis der verschiedenen 400-1000 Basenpaare langen Sequenz-Abschnitte des Gesamt-Konstruktes durchgeführt. Parallel dazu wurde als Positivkontrolle jeweils eine Reaktion mit 10-100 ng des Konstruktes in plasmidaler Form angesetzt. Als Negativkontrollen wurden jeweils eine Reaktion ohne *Primer* und eine Reaktion ohne Matrizen-DNA angesetzt.

Reaktionsgemisch 1 (zum Nachweis der Sequenzen zwischen Puromycin-Resistenz-Gen und *EGFP-*Gen):

| DNA-Matrize (siehe Text oben) |
|---|
| 50 pmol *Primer* Purol |
| 50 pmol *Primer* EGFP20-2 |
| 200 µM dNTP |
| 10 µl 10x Taq-Polymerase-Puffer (Qiagen) |
| 20 µl Q-Solution (Qiagen) |
| 1 U Taq DNA Polymerase (Qiagen) |
| ad 100 µl H₂O |

Reaktionsgemisch 2 (zum Nachweis der Sequenzen zwischen I-*Sce*I-Gen und *EGFP-*Gen):

| wie Reaktionsgemisch 1, aber als *Primer:* |
|---|
| 50 pmol *Primer* Sce1-1 oder Scel-2b |
| 50 pmol *Primer* EGFP20-2 |

Reaktionsgemisch 3 (zum Nachweis der Sequenzen im EGFP-Gen):

| wie Reaktionsgemisch 1, aber als *Primer:* |
|---|
| 50 pmol *Primer* EGFP-PCR1 |
| 50 pmol *Primer* EGFP-PCR2 oder EGFP-PCR3 |

Reaktionsgemisch 4 (zum Nachweis der Sequenzen im Hygromycin-Resistenz -Gen) :

| wie Reaktionsgemisch 1, aber als *Primer:* |
|---|
| 50 pmol *Primer* Hygl-1 |
| 50 pmol *Primer* Hyg2-2 |

Reaktionsgemisch 5 (zum Nachweis der Sequenzen zwischen Hygromycin-Resistenz-Gen und EGFP-Gen):

| wie Reaktionsgemisch 1, aber als *Primer:* |
|---|
| 50 pmol *Primer* Hyg4-1 |
| 50 pmol *Primer* EGFP-PCR2 |

Das Reaktionsgemisch wurde jeweils zunächst bei 96 °C für 5 min inkubiert. Anschließend wurden 35 Zyklen unter folgenden Bedingungen durchgeführt:
94 °C 60 s
60 °C 60 s
72 °C 120 s
Anschließend wurde das Reaktionsgemisch bei 72 °C für 7 min inkubiert.

Die entstehenden PCR-Fragmente wurden per Agarose-Gelelektrophorese neben der Lambda DNA/HindII Leiter von MBI-Fermentas, St. Leon-Rot, zur Größen- und Mengenbestimmung analysiert.

### Primer:

| | |
|---|---|
| Purol: | CCCGCAACCTCCCCTTCTAC |
| EGFP20-2: | GTGAACAGCTCCTCGCCCTTG |
| Sce1-1: | GGTCCGAACTCTAAACTGCTGA |
| Scel-2b: | TCGGGGTCAGGTAGTTTTCA |
| EGFP-PCR1: | GTGAGCAAGGGCGAGGAGCT |
| EGFP-PCR2: | CTTTACTTGTACAGCTCGTCCAT |
| EGFP-PCR3: | GACGTTGTGGCTGTTGTAGTTGTA |
| Hyg1-1: | GGAAAGCCTGAACTCACCGCGA |
| Hyg2-2: | GCTTCTGCGGGCGATTTGTGTA |
| Hyg4-1: | TACACAAATCGCCCGCAGAAGC |

## Patentansprüche

1. In vitro-Verfahren zur Bestimmung von Gentoxizitäten, bei dem man
eukaryontische Zellen, die mit einem Vektor transfiziert sind, der den allgemeinen Aufbau
5'-[*Ins1*]-[*Prom1*]-[*Marker1*]-[*Prom2*]-[*Ins2*]-[*Marker2*]-3'
aufweist, wobei
[*Ins1*] ein Sequenzabschnitt ist, der für einen Selektionsmarker, für ein autofluoreszierendes Protein (AFP), für ein biolumineszierendes oder ein Chemilumineszenz-Substrate umsetzendes Enzym (LE), oder für eine Mutante derselben kodiert
[*Prom1*] ein Promotor ist,
[*Marker1*] ein Sequenzabschnitt ist, der für ein Derivat oder eine Mutante eines autofluoreszierenden Proteins (AFP) oder eines biolumineszierenden oder Chemilumineszenz-Substrate umsetzenden Enzyms (LE) kodiert,
[*Prom2*] ein Promotor ist,
[*Ins2*] ein Sequenzabschnitt ist, der für ein AFP, LE oder eine Mutante derselben oder für I-Sce I kodiert oder ein Abstandhalter ist,
[*Marker2*] ein Sequenzabschnitt ist, der für ein Derivat oder eine Mutante eines autofluoreszierenden Proteins oder eines biolumineszierenden oder Chemilumineszenz-Substrate umsetzendes Enzym (AFP oder LE) kodiert,
wobei *[Marker1]* und *[Marker2]* zwei über mindestens 200 Basenpaare homologe DNA-Sequenz-Abschnitte sind, die mutierte Varianten eines Gens sind, aus denen sich durch homologen DNA-Austausch das Wildtyp-Gen (AFP oder LE) rekonstituiert, wobei es bei nicht-konservativer homologer Rekombination oder Replikationssprüngen zwischen [*Marker1*] und *[Marker2]* zum Verlust von *[Ins2]* kommt, wobei es bei inaktivierenden Mutationen in [*Ins1*] oder [*Ins2*] zur Inaktivierung des jeweiligen Genproduktes kommt, wobei es bei revertierenden Mutationen in *[Ins1]* oder *[Ins2]* zur Aktivierung des jeweiligen Genproduktes kommt;
oder eukaryontische Zellen, die mit einem Retrovirus infiziert sind, der einen solchen Vektor enthält,
oder einen transgenen nicht-menschlichen Säuger, dessen Keimzellen oder somatische Zellen die Sequenz eines solchen Vektors umfassen,
mit einer Testsubstanz in Kontakt bringt, und man mittels FACS-Analyse, Fluoreszenz-Messung, Fluoreszenz-Mikroskopie, Laser Scanning Cytometrie oder mittels einer Lumineszenz-Nachweis-Reaktion ein Auftauchen, eine Zunahme oder Abnahme von fluoreszierenden oder lumineszierenden Zellen entsprechend der Aktivität des Wildtyp-Genproduktes von *[Marker1], [Marker2], [Ins1]* oder *[Ins2]* in den genannten Zellen oder in Zellen des genannten Säugers nachweist,
wobei die Zellen keine menschlichen Keimzellen sind.

2. Verfahren nach Anspruch 1, **dadurch gekennzeichnet, daß** der Selektionsmarker ein Puromycin- oder Hygromycin-Resistenzgen ist.

3. Verfahren nach Anspruch 1 oder 2, **dadurch gekennzeichnet, daß** die eukaryontischen Zellen Säugerzellen sind.

4. Verfahren nach Anspruch 1 oder 2, **dadurch gekennzeichnet, daß** der Säuger ein Nager ist.

5. Verfahren zur Feststellung der Suszeptibilität eines eukaryontischen Individuums für das Auftreten oder die Progression von Krebs, bei dem man
eine Analyse von Patientenblut, Haut- oder Biopsiematerial durchführt, bei der man in die Zellen einen Vektor einbringt, der den allgemeinen Aufbau
5'- [*Ins1*] - [*Prom1*] - [*Marker1*] - [*Prom2*] - [*Ins2*] - [*Marker2*] -3'
aufweist, wobei
*[Ins1]* ein Sequenzabschnitt ist, der für einen Selektionsmarker, für ein autofluoreszierendes Protein (AFP), für ein biolumineszierendes oder ein Chemilumineszenz-Substrate umsetzendes Enzym (LE), oder für eine Mutante derselben kodiert,
[*Prom1*] ein Promotor ist,
[*Marker1*] ein Sequenzabschnitt ist, der für ein Derivat oder eine Mutante eines autofluoreszierenden Proteins (AFP) oder eines biolumineszierenden oder Chemilumineszenz-Substrate umsetzenden Enzyms (LE) kodiert,
[*Prom2*] ein Promotor ist,
*[Ins2]* ein Sequenzabschnitt ist, der für ein AFP, LE oder eine Mutante derselben oder für I*-Sce* I kodiert oder ein Abstandhalter ist,
[*Marker2*] ein Sequenzabschnitt ist, der für ein Derivat oder eine Mutante eines autofluoreszierenden Proteins oder eines biolumineszierenden oder Chemilumineszenz-Substrate umsetzendes Enzym (AFP oder LE) kodiert,
wobei [*Marker1*] und [*Marker2*] zwei über mindestens 200 Basenpaare homologe DNA-Sequenz-Abschnitte sind, die mutierte Varianten eines Gens sind, aus denen sich durch homologen DNA-Austausch das Wildtyp-Gen (AFP oder LE) rekonstituiert, wobei es bei nicht-konservativer homologer Rekombination oder Replikationssprüngen zwischen [*Marker1*] und *[Marker2]* zum Verlust von [*Ins2*] kommt, wobei es bei inaktivierenden Mutationen in [*Ins1*] oder [*Ins2*] zur Inaktivierung des jeweiligen Genproduktes kommt, wobei es bei revertierenden Mutationen in [*Ins1*] oder [*Ins2*] zur Aktivierung des jeweiligen Genproduktes kommt;
und man die DNA-Austausch-Häufigkeiten mittels FACS-Analyse, Fluoreszenz-Mikroskopie, Laser Scanning Cytometrie, Fluoreszenz-Messung oder Lumineszenz-Nachweis-Reaktion an transfizierten oder retroviral infizierten Zellen analysiert.

6. Verfahren nach Anspruch 5, **dadurch gekennzeichnet, daß** der Selektionsmarker ein Puromycin- oder Hygromycin-Resistenzgen ist.

7. Verfahren nach Anspruch 1 bis 6, **dadurch gekennzeichnet, daß** das Wildtyp-Gen *(AFP oder LE)* die für das Enhanced Green Fluorescent Protein (EGFP), die für das Humanized Renilla Green Fluorescent Protein (hrGFP), die für das Enhanced Blue Fluorescent Protein (EBFP), die für das Enhanced Cyan Fluorescent Protein (ECFP), die für das Enhanced Yellow Fluorescent Protein (EYFP) oder die für das Red Fluorescent Protein (RFP oder DsRed) kodierende Nukleinsäuresequenz ist oder eine Nukleinsäuresequenz ist, die für ein Enzym kodiert, dessen Aktivität über eine Chemolumineszenz-Reaktion nachweisbar ist.

8. Verfahren nach einem der Ansprüche 1 bis 7, **dadurch gekennzeichnet, daß** [*Ins1*] ein Sequenzabschnitt ist, der für ein Puromycin- oder Hygromycin-Resistenzgen kodiert.

9. Verfahren nach einem der Ansprüche 1 bis 7, **dadurch gekennzeichnet, daß** [*Ins1*] ein Sequenzabschnitt ist, der eine für ein autofluoreszierendes Protein, für ein biolumineszierendes oder ein Chemilumineszenz-Substrate umsetzendes Enzym oder für eine Mutante derselben kodierende Nukleinsäuresequenz enthält.

10. Verfahren nach einem der Ansprüche 1 bis 9, **dadurch gekennzeichnet, daß** *[Ins2]* ein Sequenzabschnitt ist, der eine für ein autofluoreszierendes Protein oder eine für ein biolumineszierendes oder Chemilumineszenz-Substrate umsetzendes Enzym oder für eine Mutante derselben kodierende Nukleinsäuresequenz enthält.

11. Verfahren nach einem der Ansprüche 1 bis 10, **dadurch gekennzeichnet, daß** eine Erkennungssequenz für die Meganuklease I-*Sce*I aus *Saccharomyces cerevisiae* auf dem Vektor befindlich ist.

12. Verfahren nach einem der Ansprüche 1 bis 11, **dadurch gekennzeichnet, daß** [*Prom1*] ein CMV-Promotor oder ein alternativer eukaryontischer Promotor ist, durch den in den verschiedensten Zell- und Gewebetypen mindestens eine ebenso hohe AFP-Expression erzielt wird, wie bei Verwendung des CMV-Promotors.

13. Verfahren nach einem der Ansprüche 1 bis 12, **dadurch gekennzeichnet, daß** *[Prom2]* für in Sequenzabschnitt *[Ins2]* enthaltene AFP-Gene ein CMV-Promotor oder ein alternativer eukaryontischer Promotor ist, durch den in den verschiedensten Zelltypen und Geweben mindestens eine ebenso hohe AFP-Expression erzielt wird, wie bei Verwendung des CMV-Promotors.

14. Verfahren nach einem der Ansprüche 1 bis 13, **dadurch gekennzeichnet, daß** das autofluoreszierende Protein AFP aus der Gruppe bestehend aus Humanized Renilla Green Fluorescent Protein (hrGFP), Enhanced Green Fluorescent Protein (EGFP), Enhanced Blue Fluorescent Protein (EBFP), Enhanced Cyan Fluorescent Protein (ECFP), Enhanced Yellow Fluorescent Protein (EYFP), Red Fluorescent Protein (RFP oder DsRed) oder Varianten oder Mutanten dieser Proteine ausgewählt ist.

15. Verfahren nach einem der Ansprüche 1 bis 14, **dadurch gekennzeichnet, daß** das biolumineszierende oder Chemilumineszenz-Substrate umsetzende Enzym alkalische Phosphatase, β-Galaktosidase, Peroxidase, Luciferase oder eine Variante oder Mutante derselben ist.

16. Verfahren nach einem der Ansprüche 1 bis 15, **dadurch gekennzeichnet, daß** [*Marker1*] und [*Marker2*] jeweils mindestens eine Mutation aufweisen, wobei die Positionen der Mutationen so gewählt sind, daß
a) der Sequenzbereich zwischen der Position im [*Marker2*], die einer Mutation im [*Marker1*] entspricht, und der ersten, in 3'-Richtung liegenden Mutation im [*Marker2*] oder
b) der Sequenzbereich zwischen der Position im [*Marker1*], die einer Mutation im [*Marker2*] entspricht, und der ersten, in 3'-Richtung liegenden Mutation im [*Marker1*]
mindestens etwa 150 Basenpaare umfaßt.

17. Verfahren nach einem der Ansprüche 1 bis 16, **dadurch gekennzeichnet, daß** der Vektor von einem in den Ansprüchen 1 bis 14 genannten Vektoren abgeleitet ist, indem die Sequenz-abschnitte *[[Prom1]-[Marker1]]* und *[Marker2]* an ihren Positionen ausgetauscht sind, wobei gleichzeitig die Orientierung beider Sequenzabschnitte umgedreht ist.

18. Verfahren nach einem der Ansprüche 1 bis 16, **dadurch gekennzeichnet, daß** der Vektor von einem in den Ansprüchen 1 bis 14 genannten Vektoren abgeleitet ist, indem die Orientierung der Sequenzabschnitte [[*Prom1*]*-*[*Marker1*]] und/oder [*Marker2*] umgedreht ist oder die Sequenzabschnitte ohne Orientierungsänderung ausgetauscht sind und der Vektor die Sequenz
*5'-*[*Ins1*]*-*(*3'-*[*Marker1*]*-*[*Prom1*]*-5'*)*-*[*Prom2*]*-*[*Ins2*]*-*[*Marker2*]*-*3'*,*
5'*-*[*Ins1*]*-*[*Prom1*]*-*[*Marker1*]*-*[*Prom2*]*-*[*Ins2*]*-*(3'*-*[*Marker2*]-5')-3',
5'*-*[*Ins1*]*-*(3'*-*[*Marker1*]*-*[*Prom1*]*-*5')*-*[*Prom2*]*-*[*Ins2*]*-*(3'-[*Marker2*]*-*5')-3*'*
oder 5'-[*Ins1*]-[*Marker2*]-[*Prom2*]-[*Ins2*]-[*Prom1*]-[*Marker1*]-3'
aufweist.

19. Verfahren nach einem der Ansprüche 1 bis 16, **dadurch gekennzeichnet, daß** der Vektor von einem in den Ansprüchen 1 bis 14 genannten Vektoren abgeleitet ist, indem die Orientierung des Sequenzabschnitts [[*Prom2*]*-*[*Ins2*]] umgedreht ist.

20. Verfahren nach einem der Ansprüche 1 bis 16, **dadurch gekennzeichnet, daß** der Vektor von einem in den Ansprüchen 1 bis 14 genannten Vektoren abgeleitet ist, indem der Sequenz-abschnitt [*Marker2*] deletiert ist.

21. Verfahren nach einem der Ansprüche 1 bis 20, **dadurch gekennzeichnet, daß** [*Marker1*] innerhalb des Sequenzabschnittes zusätzlich eine Erkennungssequenz für die Meganuklease I-SceI aus *Saccharomyces cerevisiae* enthält oder derselben benachbart ist.

22. Verfahren nach einem der Ansprüche 1 bis 21, **dadurch gekennzeichnet, daß** der Vektor ein retroviraler Vektor ist.

23. Verfahren nach einem der Ansprüche 1 bis 22, **dadurch gekennzeichnet, daß** der Vektor ausgewählt ist aus der Gruppe bestehend aus:
5'-[Puro]-[CMV]-[ΔEGFP]-[GRE]-[I-SceI]-[N'EGFP]-3'
5'-[Puro]-[CMV]-[EGFP-HR]-[GRE]-[I-SceI]-[N' EGFP]-3'
5'-[Puro]-[CMV]-[EGFP-EJ]-[GRE]-[I-SceI]-[N'EGFP]-3'
5'-[Puro]-[CMV]-[ΔEGFP]-[GRE]-[I-SceI]-[EGFP]-3'
5'-[Puro]-[CMV]-[EGFP-HR]-[GRE]-[I-SceI]-[EGFP]-3'
5'-[Puro]-[CMV]-[EGFP-EJ]-[GRE]-[I-SceI]-[EGFP]-3'
5'-[Puro]-[CMV]-[ΔEGFP]-[GRE]-[I-SceI]-[EGFP(Met->Stop)]-3'
5'-[Puro]-[CMV]-[EGFP-HR]-[GRE]-[I-SceI]-[EGFP(Met->Stop)]-3'
5'-[Puro]-[CMV]-[EGFP-EJ]-[GRE]-[I-SceI]-[EGFP(Met->Stop)]-3'
5'-[Puro]-[CMV]-[N'EGFP]-[CMV]-[Red]-[EGFP-HR]-3'
5'-[Puro]-[CMV]-[N'EGFP]-[CMV]-[Red]-[EGFP-EJ]-3'
5'-[Puro]-[CMV]-[EGFP-HR]-[CMV]-[Red]-[EGFP(Met->Stop)]-3'
5'-[Puro]-[CMV]-[EGFP-EJ]-[CMV]-[Red]-[EGFP(Met->Stop)]-3'.

## Claims

1. In vitro process for determining genotoxicities
in which eucaryotic cells are transfected with a vector exhibiting the general structure
5'-[*Ins1*]-[*Prom1*]-[*Marker1*]-[*Prom2*]-[*Ins2*]-[*Marker2*]-3'
where
*[Ins1]* is a sequence segment which codes for a selection marker, for an autofluorescent protein (AFP), for a bioluminescent enzyme or an enzyme that converts chemiluminescent substrates (LE) or a mutant thereof,
*[Prom1]* is a promoter,
[*Marker1*] is a sequence segment which codes for a derivative or a mutant of an autofluorescent protein (AFP) or a bioluminescent enzyme or an enzyme that converts chemoluminescent substrates (LE),
*[Prom2]* is a promoter,
*[Ins2]* is a sequence segment which codes for an AFP, LE or a mutant thereof, for I-Sce I, or is a spacer,
*[Marker2]* is a sequence segment which codes for a derivative or a mutant of an autofluorescent protein or a bioluminescent enzyme or an enzyme that converts chemoluminescent substrates (AFP or LE),
where [*Marker1*] and [*Marker2*] are two DNA sequence segments which are homologous over at least 200 base pairs, which are mutated variants of a gene, from which the wild type gene (AFP or LE) is reconstituted by homologous recombination, wherein non-conservative homologous recombination or replication jumps between [*Marker1*] and *[Marker2]* result in loss of *[Ins2],* wherein inactivating mutations in [*Ins1*] or *[Ins2]* result in inactivation of the respective gene product,
wherein reverting mutations in [*Ins1*] or [*Ins2*] result in activation of the respective gene product;
or eukaryotic cells, which are infected with a retrovirus containing such vector,
or a transgenic non-human mammal, the germ cells or somatic cells of which comprise the sequence of such vector,
are contacted with a test substance, and wherein an occurance, an increase or decrease in fluorescent or luminescent cells corresponding to the activity of the wild-type gene product of [*Marker1*]*,* [*Marker2*]*,* [*Ins1*] or [*Ins2*] in said cells or in the cells of said mammal is indicated by means of FACS analysis, fluorescence measurement, fluorescence microscopy, laser scanning cytometry or by a luminescence detection reaction,,
wherein the cells are no human germ cells.

2. Process according to claim 1, **characterised in that** the selection marker is a puromycin or hygromycin resistance gene.

3. Process according to claim 1 or 2, **characterised in that** the eukaryotic cells are mammalian cells.

4. Process according to claim 1 or 2, **characterized in that** the mammal is a rodent.

5. Process for determining the susceptibility of a eukaryotic individual for the appearance or progression of cancer in which
an analysis of the patient's blood, skin, or biopsy material is carried out during which a vector is introduced into the cells, the vector exhibiting the general structure
*5'-*[*Ins1*]*-*[*Prom1*]*-*[*Marker1*]*-*[*Prom2*]*-*[*Ins2*]*-*[*Marker2*]*-*3'
wherein
[*Ins1*] is a sequence segment which codes for a selection marker, for an autofluorescent protein (AFP), for a bioluminescent enzyme or an enzyme that converts chemiluminescent substrates (LE) or a mutant thereof,
*[Prom1]* is a promoter,
*[Marker1]* is a sequence segment which codes for a derivative or a mutant of an autofluorescent protein (AFP) or a bioluminescent enzyme or an enzyme that converts chemoluminescent substrates (LE),
*[Prom2]* is a promoter,
*[Ins2]* is a sequence segment which codes for an AFP, LE or a mutant thereof, for I-Sce I, or is a spacer,
*[Marker2]* is a sequence segment which codes for a derivative or a mutant of an autofluorescent protein or a bioluminescent enzyme or an enzyme that converts chemoluminescent substrates (AFP or LE),
where [*Marker*1] and [*Marker*2] are two DNA sequence segments which are homologous over at least 200 base pairs, which are mutated variants of a gene, from which the wild type gene (AFP or LE) is reconstituted by homologous eecombination, wherein non-conservative homologous recombination or replication jumps between [*Marker1*] and *[Marker2]* result in loss of *[Ins2],* wherein inactivating mutations in *[Ins1]* or *[Ins2]* result in inactivation of the respective gene product, wherein reverting mutations in *[Ins1]* or *[Ins2]* result in activation of the respective gene product;
the DNA exchange frequency being analysed by FACS analysis, fluorescence microscopy, laser scanning cytometry, fluorescence measurement, or a luminescence detection reaction on transfected or retrovirally infected cells.

6. Process according to claim 5, **characterized in that** the selection marker is a puromycin or hygromycin resistance gene.

7. Process according to claim 1 to 6, **characterised in that** the wild-type gene *(AFP* or *LE)* is the nucleic acid sequence encoding for the enhanced green fluorescence protein (EGFP), the humanized renilla green fluorescent protein (hrGFP), the enhanced blue fluorescence protein (EBFP), the enhanced cyan fluorescence protein (ECFP), the enhanced yellow fluorescence protein (EYFP) or the red fluorescent protein (RFP or *DsRed)* or a nucleic acid sequence encoding an enzyme whose activity is detectable via a chemoluminescence reaction.

8. Process according to any one of claims 1 to 7, **characterised in that** *[Ins1]* is a sequence segment encoding a puromycin or hygromycin resistance gene.

9. Process according to one of claims 1 to 7, **characterised in that** [*Ins1*] is a sequence segment which contains a nucleic acid sequence encoding an autofluorescent protein, a bioluminescent enzyme or an enzyme that converts chemiluminescent substrates or for a mutant thereof.

10. Process according to one of claims 1 to 9, **characterised in that** *[Ins2]* is a sequence segment which contains a nucleic acid sequence encoding an autofluorescent protein or a bioluminescent enzyme or an enzyme that converts chemiluminescent substrates or a mutant thereof.

11. Process according to of claims 1 to 10, **characterised in that** a recognition sequence for meganuclease I-SceI from *Saccharomyces cerevisiae* is present on the vector.

12. Process according to one of claims 1 to 11, **characterised in that** [*Prom1*] is a CMV promoter or an alternative eukaryotic promoter by means of which at least as high an AFP expression is achieved as when the CMV promoter is used.

13. Vector according to one of claims 1 to 12, **characterised in that** *[Prom2]* is a CMV promoter or alternative eukaryotic promoter for AFP genes which are contained in sequence segment [*Ins*2]*,* by means of which at least as high an AFP expression is achieved in the various cell types and tissues as when the CMV promoter is used.

14. Process according to one of claims 1 to 13, **characterised in that** the autofluorescent protein is selected from the group consisting of humanized renilla green fluorescent protein (hrGFP), enhanced green fluorescent protein (EGFP), enhanced blue fluorescent protein (EBFP), enhanced cyan fluorescent protein (ECFP), enhanced yellow fluorescent protein (EYFP), red fluorescent protein (RFP or DsRed) or variants or mutants of these proteins.

15. Process according to one of claims 1 to 14, **characterised in that** the bioluminescent enzyme or the enzyme that converts chemoluminescent substrates is alkaline phosphatase, ß-galactosidase, peroxidase, luciferase or a variant or mutant thereof.

16. Process according to one of claims 1 to 15, **characterised in that** [*Marker1*] and [*Marker2*] each exhibit at least one mutation, the positions of the mutations being selected such that
a) the section of the sequence between the position in [*Marker2*] which corresponds to a mutation in [*Marker1*] and the first mutation in [*Marker2*] which is situated in the 3' direction, or
b) the section of the sequence between the position in [*Marker1*] which corresponds to a mutation in *[Marker2]* and the first mutation in [*Marker1*] which is situated in the 3' direction
comprises at least approximately 150 base pairs.

17. Process according to one of claims 1 to 16, **characterised in that** the vector is derived from a vector mentioned in claims 1 to 14 **in that** the sequence segments *[[Prom1]-[Marker1]]* and [*Marker2*] are exchanged in their positions, wherein the orientation of the two sequence segments is simultaneously reversed.

18. Process according to one of claims 1 to 16, **characterised in that** the vector is derived from a vector mentioned in claims 1 to 14 **in that** the orientation of the sequence segments [[*Prom1*]-[*Marker1*]] and/or [*Marker2*] is reversed or the sequence segments are exchanged without a change in orientation, and wherein the vector exhibits the sequence
*5'-*[*Ins1*]*-*(3'-[*Marker1*]-[*Prom1*]-5')-[*Prom2*]-[*Ins2*]-[*Marker2*]-3',
*5'-*[*Ins1*]*-*[*Prom1*]*-*[*Marker*1]*-*[*Prom2*]*-*[*Ins2*]*-*(3'-[*Marker2*]-5')-3'
*5'-*[*Ins1*]*-*(3'-[*Marker1*]-[*Prom1*]*-*5')*-*[*Prom2*]*-*[*Ins2*]*-*(3'-[*Marker2*]*-*5')*-*3*'*
or 5*'-*[*Ins1*]*-*[*Marker2*]*-*[*Prom2*]-[*Ins2*]-[*Prom1*]-[*Marker1*]-3' .

19. Process according to one of claims 1 to 16, **characterised in that** the vector is derived from a vector mentioned in claims 1 to 14 **in that** the orientation of the sequence segment [[Prom2]-[*Ins2*]] is reversed.

20. Process according to one of claims 1 to 16, **characterised in that** the vector is derived from a vector mentioned in claims 1 to 14 **in that** the sequence segment [*Marker2*] is deleted.

21. Process according to one of claims 1 to 20, **characterised in that** [*Marker1*] within the sequence segment additionally contains a recognition sequence for meganuclease I-SceI from *Saccharomyces cerevisiae* or is adjacent to the same.

22. Process according to one of claims 1 to 21, **characterised in that** the vector is a retroviral vector.

23. Process according to one of claims 1 to 21, **characterised in that** the vector is selected from the group consisting of:
5`- [Puro] - [CMV] - [ΔEGFP] - [GRE] - [I-SceI] - [N'EGFP] -3'
5'-[Puro]-[CMV]-[EGFP-HR]-[GRE]-[I-SceI]-[N'EGFP]-3'
5`-[Puro] - [CMV] - [EGFP-EJ] - [GRE] - [I-SceI] - [N*'* EGFP] -3'
5'- [Puro] - [CMV]- [ΔEGFP] - [GRE] - [I-SceI] - [EGFP] -3'
5'- [Puro] - [CMV] - [EGFP-HR] - [GRE] - [I-SceI] - [EGFP] -3'
5' - [Puro] - [CMV] - [EGFP-EJ] - [GRE] - [I-SceI] - [EGFP] - 3'
5`- [Puro] - [CMV] - [ΔEGFP] - [GRE] - [I-SceI] - [EGFP (Met->Stop)] -3'
5'-[Puro]-[CMV]-[EGFP-HR]-[GRE]-[I-SceI]-[EGFP(Met->Stop)]-3'.
5'-[Puro]-[CMV]-[EGFP-EJ]-[GRE]-[I-SceI]-[EGFP(Met->Stop)]-3'
5' - [Puro] - [CMV] - [N'EGFP] - [CMV] - [Red] - [EGFP-HR] - 3'
5'-[Puro]-[CMV]-[N'EGFP]-[CMV]-[Red]-[EGFP-EJ]-3'
5'-[Puro]-[CMV]-[EGFP-HR]-[CMV]-[Red]-[EGFP(Met->Stop)]-3'
5`-[Puro]-[CMV]-[EGFP-EJ]-[CMV]-[Red]-[EGFP(Met->Stop)]-3`.

## Revendications

1. Procédé *in vitro* pour déterminer des toxicités génétiques, dans lequel on met en contact des cellules eucaryotes transfectées avec un vecteur qui présente la structure générale suivante
5'-[Ins1]-[Prom1]-[Marqueur1]-[Prom2]-[Ins2]-[Marqueur2]-3'
où
[Ins 1] est une partie de séquence, qui code pour un marqueur de sélection, une protéine autofluorescente (AFP), une enzyme bioluminescente ou bien une enzyme transformant un substrat de chimioluminescence (LE) ou un mutant de ceux-ci,
[Prom1] est un promoteur
[Marqueur1] est une partie de séquence, qui code pour un dérivé ou un mutant d'une protéine autofluorescente (AFP), une enzyme bioluminescente ou bien une enzyme transformant un substrat de chimioluminescence (LE),
[Prom2] est un promoteur
[Ins2] est une partie de séquence, qui code pour une AFP, une LE ou un mutant de ceux-ci ou I-Sce I ou est un espaceur,
[Marqueur2] est un segment de séquence, qui code un dérivé ou un mutant d'une protéine autofluorescente ou d'une enzyme bioluminescente ou bien une enzyme transformant un substrat de chimioluminescence (AFP ou LE),
où [Marqueur1] et [Marqueur2] sont deux parties de séquence d'ADN homologues sur au moins 200 paires de bases, qui sont les variants mutés d'un gène, à partir duquel on peut reconstituer le gène de type sauvage (AFP ou LE) par échange homologue d'ADN, où l'on parvient lors d'une recombinaison homologue non conservative ou de sauts de réplication entre [Marqueur1] et [Marqueur2], à une perte de [Ins2], où l'on parvient, lors de mutations inactivantes dans [Ins1] ou [Ins2], à une inactivation de chaque produit génique, où l'on parvient lors de mutations réverses dans [Ins1] ou [Ins2], à une activation de chaque produit génique ;
ou bien des cellules eucaryotes, qui sont infectées avec un rétrovirus, qui contient un tel vecteur,
ou bien un mammifère transgénique non humain, dont des cellules souches ou des cellules somatiques comprennent la séquence d'un tel vecteur,
avec une substance à tester, et dans lequel on détecte par analyse FACS, mesure de fluorescence, microscopie par fluorescence, cytométrie à balayage scanner ou par réaction de détection par luminescence, une émergence, une augmentation ou une diminution des cellules fluorescentes ou luminescentes correspondant à l'activité du produit génique de type sauvage de [Marqueur1], [Marqueur2], [Ins1] ou [Ins2], dans les cellules citées ou dans les cellules du mammifère cité,
où les cellules ne sont pas des cellules souches humaines.

2. Procédé selon la revendication 1, **caractérisé en ce que** le marqueur de sélection est un gène de résistance à la puromycine ou l'hygromycine.

3. Procédé selon la revendication 1 ou 2, **caractérisé en ce que** les cellules eucaryotes sont des cellules de mammifère.

4. Procédé selon la revendication 1 ou 2, **caractérisé en ce que** le mammifère est un rongeur.

5. Procédé d'établissement de la susceptibilité d'un individu eucaryote pour l'apparition ou la progression d'un cancer, dans lequel on réalise une analyse du sang du patient, d'un matériel de peau ou de biopsie, dans laquelle on introduit un vecteur dans les cellules, lequel vecteur présente la structure générale
5'-[Ins1]-[Prom 1]-[Marqueur 1]-[Prom2]-[Ins2]-[Marqueur2]-3'
où
[Ins1] est une partie de séquence, qui code pour un marqueur de sélection, une protéine autofluorescente (AFP), une enzyme bioluminescente ou bien une enzyme transformant un substrat de chimioluminescence (LE) ou un mutant de ceux-ci,
[Prom1] est un promoteur
[Marqueur1] est une partie de séquence, qui code pour un dérivé ou un mutant d'une protéine autofluorescente (AFP) ou d'une enzyme bioluminescente ou bien une enzyme transformant un substrat de chimioluminescence (LE),
[Prom2] est un promoteur
[Ins2] est une partie de séquence, qui code pour
une AFP, une LE ou un mutant de ceux-ci ou I-Sce I ou bien est un espaceur,
[Marqueur2] est une partie de séquence, qui code un dérivé ou un mutant d'une protéine autofluorescente ou d'une enzyme bioluminescente ou transformant un substrat de chimioluminescence (AFP ou LE),
où [Marqueur1] et [Marqueur2] sont deux parties de séquence d'ADN homologues sur au moins 200 paires de bases, qui sont les variants mutés d'un gène, à partir duquel on peut reconstituer le gène de type sauvage (AFP ou LE) par échange homologue d'ADN, où l'on parvient lors d'une recombinaison homologue non conservative ou de sauts de réplication entre [Marqueur1] et [Marqueur2], à une perte de [Ins2], où l'on parvient, lors de mutations inactivantes dans [Ins1] ou [Ins2], à une inactivation de chaque produit génique, où l'on parvient lors de mutations réverses dans [Ins1] ou [Ins2], à une activation de chaque produit génique ;
et en ce que l'on analyse les fréquences d'échange d'ADN par analyse FACS, microscopie par fluorescence, cytométrie à balayage scanner, mesure de fluorescence ou par réaction de détection par luminescence, les cellules transfectées et infectées, de manière rétrovirale.

6. Procédé selon la revendication 5, **caractérisé en ce que** le marqueur de sélection est un gène de résistance à la puromycine ou l'hygromycine.

7. Procédé selon les revendications 1 à 6, **caractérisé en ce que** le gène de type sauvage (AFP ou LE) est la séquence d'acide nucléique codant la protéine fluorescente verte amplifiée (EGFP), la protéine fluorescente verte de *Renilla* humanisée (hrGFP), la protéine fluorescente bleue amplifiée (EBFP), la protéine fluorescente cyan amplifiée (ECFP), la protéine fluorescente jaune amplifiée (EYFP) ou la protéine fluorescente rouge (RFP ou DsRed) ou est une séquence d'acide nucléique, qui code une enzyme dont l'activité est détectable par une réaction de chimioluminescence.

8. Procédé selon l'une des revendications 1 à 7, **caractérisé en ce que** [Ins1] est une partie de séquence, qui code un gène de résistance à la puromycine ou l'hygromycine.

9. Procédé selon l'une des revendications 1 à 7, **caractérisé en ce que** [Ins1] est une partie de séquence, qui contient une séquence d'acide nucléique codant une protéine autofluorescente, une enzyme bioluminescente ou bien une enzyme transformant un substrat chimioluminescent ou un mutant de celles-ci.

10. Procédé selon l'une des revendications 1 à 9, **caractérisé en ce que** [Ins2] est une partie de séquence, qui contient une séquence d'acide nucléique codant une protéine autofluorescente, une enzyme bioluminescente ou transformant un substrat chimioluminescent ou un mutant de celles-ci.

11. Procédé selon l'une des revendications 1 à 10, **caractérisé en ce qu'**une séquence de reconnaissance de la méganucléase 1 SceI de *Saccharomyces cereviasiae* se trouve dans le vecteur.

12. Procédé selon l'une des revendications 1 à 11, **caractérisé en ce que** [Prom1] est un promoteur de CMV ou un promoteur eucaryote alternatif, par lequel on obtient dans des types différents de cellule ou de tissu, au moins une expression aussi élevée de l'AFP, comme lors de l'utilisation du promoteur de CMV.

13. Procédé selon l'une des revendications 1 à 12, **caractérisé en ce que** [Prom2] pour le gène AFP présent dans la partie de séquence [Ins2], est un promoteur de CMV ou un promoteur eucaryote alternatif, par lequel on obtient dans des types différents de cellule ou de tissu, au moins une expression aussi élevée de l'AFP, comme lors de l'utilisation du promoteur de CMV.

14. Procédé selon l'une des revendications 1 à 13, **caractérisé en ce que** la protéine autofluorescente AFP est choisie parmi le groupe consistant en la protéine fluorescente verte de *Renilla* humanisée (hrGFP), la protéine fluorescente verte amplifiée (EGFP), la protéine fluorescente bleue amplifiée (EBFP), la protéine fluorescente cyan amplifiée (ECFP), la protéine fluorescente jaune amplifiée (EYFP) ou la protéine fluorescente rouge (RFP ou DsRed) ou des variants ou mutants de ces protéines.

15. Procédé selon l'une des revendications 1 à 14, **caractérisé en ce que** l'enzyme bioluminescente ou transformant un substrat chimioluminescent est la phosphatase alcaline, la (β-galactosidase, une peroxydase, la luciférase ou un variant ou mutant de celles-ci.

16. Procédé selon l'une des revendications 1 à 15, **caractérisé en ce que** [Marqueur1] et [Marqueur2] présentent chacun, au moins une mutation, où les positions des mutations sont choisies de sorte que
a) le domaine de séquence entre la position dans [Marqueur2], qui correspond à une mutation dans [Marqueur1], et la première mutation se trouvant en direction 3' dans [Marqueur2],
b) le domaine de séquence entre la position dans [Marqueur1], qui correspond à une mutation dans [Marqueur2], et la première mutation se trouvant en direction 3' dans [Marqueur1],
comprend au moins de l'ordre de 150 paires de bases.

17. Procédé selon l'une des revendications 1 à 16, **caractérisé en ce que** le vecteur dérive d'un des vecteurs indiqués dans les revendications 1 à 14, **en ce que** les parties de séquence [[Prom1]-[Marqueur1]] et [Marqueur2] sont échangées au niveau de leur position, où, simultanément, on intervertit l'orientation des deux parties de séquence.

18. Procédé selon l'une des revendications 1 à 16, **caractérisé en ce que** le vecteur dérive d'un des vecteurs indiqués dans les revendications 1 à 14, **en ce que** l'orientation des parties de séquence [[Prom1]-[Marqueur1]] et/ou [Marqueur2] est inversée ou les parties de séquence sont échangées sans modification d'orientation et le vecteur présente l'une des séquences suivantes :
5'-[Ins1]-(3'-(Marqueur 1]-[Prom 1]-5')-[Prom2]-[Ins2]-[Marqueur2]-3'
5'-[Ins 1]-[Prom 1]-[Marqueur1]-[Prom2]-[Ins2]-(3' -[Marqueur2]-5')-3'
5'-[Ins 1]-(3'-[Marqueur1]-[Prom 1]-5')-[Prom2]-[Ins2]-(3'-[Marqueur2]-5')-3'
ou
5'-[Ins1]-[Marqueur2]-[Prom2]-[Ins2]-[Prom1]-[Marqueur1]-3'.

19. Procédé selon l'une des revendications 1 à 16, **caractérisé en ce que** le vecteur dérive d'un des vecteurs indiqués dans les revendications 1 à 14, **en ce que** l'orientation des parties de séquence [[Prom2]-[Ins2]] est inversée.

20. Procédé selon l'une des revendications 1 à 16, **caractérisé en ce que** le vecteur dérive d'un des vecteurs indiqués dans les revendications 1 à 14, **en ce que** la partie de séquence [Marqueur2] fait l'objet d'une délétion.

21. Procédé selon l'une des revendications 1 à 20, **caractérisé en ce que** [Marqueur1] contient dans le segment de séquence, en outre, une séquence de reconnaissance de la méganucléase I SceI de *Saccharomyces cerevisiae* ou est voisin de celle-ci.

22. Procédé selon l'une des revendications 1 à 21, **caractérisé en ce que** le vecteur est un vecteur rétroviral.

23. Procédé selon l'une des revendications 1 à 22, **caractérisé en ce que** le vecteur est choisi parmi le groupe consistant en
5'-[Puro]-[CMV]-[ΔEGFP]-[GRE]-[I-SceI]-[N'EGFP]-3'
5'-[Puro]-[CMV]-[EGFP-HR]-[GRE]-[I-SceI]-[N'EGFP]-3'
5'-[Puro]-[CMV]-[EGFP-EJ]-[GRE]-[I-SceI]-[N'EGFP]-3'
5'-[Puro]-[CMV]-[ΔEGFP]-[GRE]-[I-SceI]-[EGFP]-3'
5'-[Puro]-[CMV]-[EGFP-HR]-[GRE]-[I-SceI]-[EGFP]-3'
5'-[Puro]-[CMV]-[EGFP-EJ]-[GRE]-[I-SceI]-[EGFP]-3'
5'-[Puro]-[CMV]-[ΔEGFP]-[GRE]-[I-SceI]-[EGFP(Met->stop)]-3'
5'-[Puro]-[CMV]-[EGFP-HR]-[GRE]-[I-SceI]-[EGFP(Met->stop)]-3'
5'-[Puro]-[CMV]-[EGFP-EJ]-[GRE]-[I-SceI]-[EGFP(Met->stop)]-3'
5'-[Puro]-[CMV]-[N'EGFP]-[CMV]-[Red]-[EGFP-HR]-3'
5'-[Puro]-[CMV]-[N'EGFP]-[CMV]-[Red]-[EGFP-EJ]-3'
5'-[Puro]-[CMV]-[EGFP-HR]-[CMV]-[Red]-[EGFP(Met->stop)]-3'
5'-[Puro]-[CMV]-[EGFP-EJ]-[CMV]-[Red]-[EGFP (Met->stop)]-3'.
